# EUROPEAN PATENT SPECIFICATION

(11) **EP 2 285 360 B1**
(45) Date of publication and mention of the grant of the patent: **13.04.2016**
(21) Application number: 09747335.9
(22) Date of filing: 12.05.2009
(51) Int. Cl.: A61K 9/22, A61M 5/20, A61M 5/32, A61M 5/31, G09B 19/00, A61M 5/315

(54) **MEDICAMENT DELIVERY DEVICE HAVING AN ELECTRONIC CIRCUIT SYSTEM**
MEDIKAMENTENFREISETZUNGSVORRICHTUNG MIT ELEKTRONISCHER SCHALTUNG
DISPOSITIF DE DISTRIBUTION DE MÉDICAMENT COMPRENANT UN SYSTÈME DE CIRCUIT ÉLECTRONIQUE

(30) Priority: 12.05.2008 US 119016; 28.07.2008 US 180708
(43) Date of publication of application: 23.02.2011
(73) Proprietor: Kaleo, Inc., Richmond VA 23219 (US)
(72) Inventor: EDWARDS, Eric, S., Midlothian, VA 23112 (US); EDWARDS, Evan, T., Gordonsville, VA 22942 (US); LICATA, Mark, J., Doswell, VA 23047 (US); MEYERS, Paul, F., Fishers, IN 46037 (US); WEINZIERL, David, A., Andover, MN 55304 (US)
(74) Representative: Gill Jennings & Every LLP
(86) International application number: PCT/US2009/043578
(87) International publication number: WO 2009/140251

(56) References cited:
- WO-A1-96/25965
- CA-A1- 2 644 547
- US-A- 4 613 328
- US-A- 5 814 020
- US-A1- 2003 181 852
- US-A1- 2007 239 114
- US-A1- 2008 033 393
- US-A1- 2008 059 133
- US-B1- 6 482 185

## Description

### Background

The invention relates generally to a medical device, and more particularly to a medicament delivery device, and/or a simulated medicament delivery device having an electronic circuit system.

Exposure to certain substances, such as, for example, peanuts, shellfish, bee venom, certain drugs, toxins, and the like, can cause allergic reactions in some individuals. Such allergic reactions can, at times, lead to anaphylactic shock, which can cause a sharp drop in blood pressure, hives, and/or severe airway constriction. Accordingly, responding rapidly to mitigate the effects from such exposures can prevent injury and/or death. For example, in certain situations, an injection of epinephrine (i.e., adrenaline) can provide substantial and/or complete relief from the allergic reaction. In other situations, for example, an injection of an antidote to a toxin can greatly reduce and/or eliminate the harm potentially caused by the exposure. Because emergency medical facilities may not be available when an individual is suffering from an allergic reaction, some individuals carry a medicament delivery device, such as, for example, an auto-injector, to rapidly self-administer a medicament in response to an allergic reaction.

To actuate such a medicament delivery device, however, the user may be required to execute a series of operations. For example, to actuate some known auto-injectors, the user must remove a protective cap, remove a locking device, place the auto-injector in a proper position against the body and then press a button to actuate the auto-injector. Failure to complete these operations properly can result in an incomplete injection and/or injection into an undesired location of the body. In certain instances, for example, users who have become confused in the operation of some known auto-injectors have inadvertently injected the medicament into their thumb by improperly positioning the auto-injector.

The likelihood of improper use of known medicament delivery devices can be compounded by the nature of the user and/or the circumstances under which such devices are used. For example, many users are not trained medical professionals and may have never been trained in the operation of such devices. Moreover, in certain situations, the user may not be the patient, and may therefore have no experience with the medicament delivery device. Similarly, because some known medicament delivery devices are configured to be used relatively infrequently in response to an allergic reaction or the like, even those users familiar with the device and/or who have been trained may not be well practiced at operating the device. Finally, such devices are often used during an emergency situation, during which even experienced and/or trained users may be subject to confusion, panic, and/or the physiological effects of the condition requiring treatment.

Some known medicament delivery devices include printed instructions to inform the user of the steps required to properly deliver the medicament. Such printed instructions, however, can be inadequate for the class of users and/or the situations described above. Moreover, because some known medicament delivery devices, such as, for example, auto-injectors, pen injectors, inhalers or the like, can be compact, such printed instructions may be too small to read and comprehend during an emergency situation.

Some known medicament delivery devices are associated with simulated medicament delivery devices (e.g., "trainers") to provide a method for users to practice using the medicament delivery device without being exposed to the medicament and/or needles typically contained therein. Such simulated medicament delivery devices, however, can also include inadequate use instructions as described above.

Some known medicament delivery devices can produce sounds, such as a beep or a click, that can be used as prompts to users of medicament delivery devices. The sounds of such know devices and the manner in which the sounds are produced, however, provide limited information to the user. For example, some known medicament delivery devices produce a single tone to indicate that a proper dosage has been set but cannot provide a user with instructions associated with the use of the device. Moreover, the sound level and/or the quality of the sound produced by such known medicament delivery devices is limited by the size, performance, and/or cost associated with the speaker and/or electronic components necessary to produce the sounds.

Monitoring the patient's compliance with known medicament delivery devices can also be problematic. For example, some known medicament delivery systems include a medicament delivery device and an electronic system to assist the user in setting the proper dosage and/or maintaining a compliance log. Such known medicament delivery systems and the accompanying electronic systems can be large and therefore not conveniently carried by the user. Such known medicament delivery systems and the accompanying electronic systems can also be complicated to use and/or expensive to manufacture. Moreover, some known medicament delivery systems include sensors disposed within the medicament delivery path, which can interfere with the delivery, result in contamination, or the like.

. Thus, a need exists for medicament delivery systems and/or devices that provide instructions that can be easily understood and/or heard by a user in any type of situation. Additionally, a need exists for simulated medicament delivery systems and/or devices that can provide instructions and that can be reused multiple times. Moreover, a need exists for medicament delivery systems and/or devices that can provide compliance information associated with the use of the device and/or that can communicate electronically with other communications devices.

WO96/25965 discloses an apparatus with a medicament delivery device and container with associated electronic circuit system.

According to the present invention there is provided an apparatus, comprising: a medicament delivery device including a housing, a medicament container, an actuator, and a delivery member, the housing defining a first region, a second region, and an exterior region, at least one sidewall of the housing separating the exterior region, the first region, and the second region from each other, the first region including the medicament container and the delivery member, the delivery member configured to be in fluid communication with the exterior region via a first opening of the housing; and an electronic circuit system configured to be disposed within the second region, the electronic circuit system configured to output an electronic output when the electronic circuit system is actuated, a cover configured to be removeably coupled to the housing, a first portion of the cover configured to cover the first opening when the cover is coupled to the housing, a second portion of the cover extending into the second region of the housing via a second opening when the cover is coupled to the housing, the electronic circuit system configured to be actuated when the cover is removed from the housing..

### Brief Description of the Drawings

FIG. 1 is a schematic illustration of a medicament delivery device according to an embodiment of the invention.
FIG. 2 is a schematic illustration of a medicament delivery device according to an embodiment of the invention.
FIGS. 3 and 4 are perspective views of a medical injector according to an embodiment of the invention, in a first configuration.
FIG. 5 is a front view of the medical injector illustrated in FIG. 3 with the cover removed.
FIG. 6 is a back view of the medical injector illustrated in FIG. 3 with the cover removed.
FIG. 7 is a front view of a portion of the medical injector illustrated in FIG. 3.
FIG. 8 is a perspective view of a portion of the medical injector illustrated in FIG. 3.
FIG. 9 is a bottom perspective view of a housing of the medical injector illustrated in FIG. 3.
FIG. 10 is a top perspective view of a housing of the medical injector illustrated in FIG. 3.
FIG. 11 is a perspective view of a proximal cap of the medical injector illustrated in FIG. 3.
FIG. 12 is a front view of a medicament delivery mechanism of the medical injector illustrated in FIG. 3.
FIG. 13 is a back view of an electronic circuit system of the medical injector illustrated in FIG. 3.
FIG. 14 is a front view of a portion of the electronic circuit system of the medical injector illustrated in FIG. 13.
FIG. 15 is a side view of the electronic circuit system of the medical injector illustrated in FIG. 13.
FIG. 16 is a front view of an electronic circuit system housing of the medical injector illustrated in FIG. 13.
FIG. 17 is a perspective view of the electronic circuit system housing of the medical injector illustrated in FIG. 16.
FIG. 18 is a perspective view of a battery clip of the medical injector illustrated in FIG. 13.
FIG. 19 is a perspective view of a portion of an electronic circuit system of the medical injector illustrated in FIG. 3, in a first configuration.
FIG. 20 is a front view of the medical injector illustrated in FIG. 3 in a first configuration showing the electronic circuit system.
FIGS. 21, 22, and 23 are front views of a portion of the electronic circuit system of the medical injector labeled as Region Z in FIG. 20 in a first configuration, a second configuration, and a third configuration, respectively.
FIGS. 24 and 25 are perspective views of a cover of the medical injector illustrated in FIG. 3.
FIG. 26 is a perspective view of a safety lock of the medical injector illustrated in FIG. 3.
FIG. 27 is a front view of the safety lock of the medical injector illustrated in FIG. 26.
FIG. 28 is a bottom view of the safety lock of the medical injector illustrated in FIG. 26.
FIG. 29 is a perspective view of a needle sheath of the safety lock of the medical injector illustrated in FIG. 26.
FIG. 30 is a perspective view of a base of the medical injector illustrated in FIG. 3.
FIG. 31 is a front view of the base of the medical injector illustrated in FIG. 3.
FIG. 32 is a back view of the medical injector illustrated in FIG. 3 in a second configuration.
FIG. 33 is a back view of the medical injector illustrated in FIG. 3 in a third configuration.
FIG. 34 is a back view of the medical injector illustrated in FIG. 3 in a fourth configuration.
FIG. 35 is a perspective view of a housing of a medical injector according to an embodiment of the invention.
FIG. 36 is a perspective view of an electronic circuit system of a medical injector according to an embodiment of the invention.
FIG. 37 is a back view of a printed circuit board of the electronic circuit system shown in FIG. 36.
FIG. 38 is a schematic illustration of the electronic circuit system shown in FIG. 36.
FIG. 39 is a perspective cross-sectional view of the housing and the electronic circuit system illustrated in FIG. 35 and FIG. 36 respectively.
FIG. 40 is a cross-sectional perspective view of a portion of the electronic circuit system illustrated in FIG. 36, taken along line X-X in FIG. 39.
FIG. 41 is a flow chart illustrating a method of assembling a medical injector according to an embodiment of the invention.
FIG. 42 is a flow chart illustrating a method of assembling a simulated medical injector according to an embodiment of the invention.
FIG. 43 is a flow chart illustrating a method of testing a medical injector according to an embodiment of the invention.
FIG. 44 is a schematic illustration of a medicament delivery device having an acoustic enclosure, according to an embodiment of the invention.
FIG. 45 is a schematic illustration of a medicament delivery device having a ported acoustic enclosure, according to an embodiment of the invention.
FIG. 46 is a schematic illustration of a medicament delivery device according to an embodiment of the invention.
FIG. 47 is a schematic illustration of a medicament delivery device and an electronic circuit system assembly according to an embodiment of the invention.
FIG. 48 is a schematic illustration of the electronic circuit system assembly shown in FIG. 47 coupled to the medicament delivery device shown in FIG. 47.
FIG. 49 is a cross-sectional view of the electronic circuit system assembly and the medicament delivery device shown in FIG. 48, taken along a plane including line X-X.

### Detailed Description

In some embodiments, an apparatus includes a medical injector and an electronic circuit system. The medical injector includes a housing, a medicament container, and a medicament delivery member. The housing defines a first region and a second region. The first region includes the medicament container and is physically isolated from the second region. The electronic circuit system is configured to be disposed within the second region defined by the housing. The electronic circuit system is configured to output an electronic output associated with a use of the medical injector.

In some embodiments, an apparatus includes a medical injector and an electronic circuit system. The medical injector includes a housing, a medicament container, and a medicament delivery member. The medicament delivery member can be, for example, a needle or an injection nozzle. The housing defines a first region and a second region. The first region includes the medicament container and is physically isolated from the second region. The electronic circuit system is configured to be disposed within the second region defined by the housing. The electronic circuit system includes a printed circuit board having a substrate and an electrical conductor disposed on the substrate. The substrate of the printed circuit board is configured to receive an actuator configured to disrupt the electrical conductor. The actuator can be, for example, an actuator configured to initiate delivery of a medicament from the medical injector. The electronic circuit system is configured to output an electronic output associated with a use of the medical injector when the electrical conductor is disrupted. In some embodiments, the electronic output can be, for example, associated with recorded speech.

In some embodiments, an apparatus includes a medicament delivery device and an electronic circuit. The medicament delivery device, which can be, for example, a pen injector, an auto-injector, an inhaler or a transdermal delivery device, includes a housing, a medicament container, and a medicament delivery member. The medicament container and at least a portion of the medicament delivery member are disposed within the housing. The medicament container and the medicament delivery member define a medicament delivery path. The electronic circuit system is coupled to the housing and is physically isolated from the medicament delivery path. The electronic circuit system is configured to output an electronic output in response to a delivery of a medicament via the medicament delivery path. In some embodiments, the electronic output can be, for example, a visual output, an audible output, and/or a haptic output.

In some embodiments, an apparatus includes a medical injector having a housing, a medicament container and a medicament delivery member. The housing defines a first region including at least the medicament container and a second region configured to receive an electronic circuit system. The first region of the housing is physically isolated from the second region of the housing. The electronic circuit system is configured to output an electronic output associated with a use of the medical injector. In some embodiments, the medical injector is configured to deliver only a single dose of medicament into a body. In other embodiments, the medical injector is configured to be reusable.

In some embodiments, an apparatus includes an electronic circuit system configured to be coupled to a medical injector such that, the electronic circuit system is physically isolated from a medicament delivery path. The electronic circuit system is configured to output an electronic output in response to the delivery of a medicament via the medicament delivery path. The electronic output can be, for example, a visual output, an audible output, and/or a haptic output.

In some embodiments, an apparatus includes a medicament delivery device and an electronic circuit system. The medicament delivery device includes a housing, a medicament container, and a medicament delivery member. The medicament container and at least a portion of the medicament delivery member are disposed within the housing. The electronic circuit system is coupled to the housing and includes an audible output device and a cover. The housing of the medicament delivery device and the cover of the electronic circuit system collectively define an acoustic enclosure. The audible output device, which can be, for example, a speaker, is configured to be disposed within the acoustic enclosure.

In some embodiments, an apparatus includes a medicament delivery device and an electronic circuit system. The medicament delivery device includes a housing and a medicament container. The medicament container is disposed within the housing. The electronic circuit system is coupled to the housing and includes a speaker and a cover. The speaker includes a front portion and a back portion. The front portion of the speaker is configured to output a first audible output including a first set of sound waves. The back portion of the speaker is configured to output a second audible output including a second set of sound waves. The housing of the medicament delivery device defines a first opening through which the first set of sound waves is configured to travel. The cover of the electronic circuit system defines a second opening through which the second set of sound waves is configured to travel.

In some embodiments, an apparatus includes a medicament delivery device and an electronic circuit system, the medicament delivery device including a housing, a medicament container, and a medicament delivery member. The medicament container and at least a portion of the medicament delivery member are disposed within the housing. The electronic circuit system is coupled to the housing and includes an audio processor and an audible output device. The audio processor is configured to output an electronic signal associated with recorded speech to the audible output device via an electronic path devoid of an amplifier. The audible output device can be configured to output an audible output in response to the electronic signal.

In some embodiments, a method includes assembling a medical device configured to deliver a medicament into a body of a patient. The medical device includes a housing, a medicament container, an actuator and a safety lock. The medicament container is disposed within the housing. The actuator is configured to initiate delivery of the medicament from the medicament container when the actuator is actuated. The safety lock is configured to prevent actuation of the actuator. An electronic circuit system is coupled to the housing of the assembled medical device such that an opening defined by a substrate of the electronic circuit system is disposed about a portion of the safety lock. The electronic circuit system is configured to output an electronic output in response to a movement of the safety lock within the opening.

In some embodiments, a method includes coupling an electronic circuit system to a simulated medicament delivery device such that a portion of the housing actuates a switch of the electronic circuit system. The simulated medicament delivery device is configured to simulate an actual medicament delivery device. The electronic system is configured to output an electronic output associated with a use of the simulated medicament delivery device and a state of the switch. The electronic output can be, for example, a visual output, an audible output, and/or a haptic output.

In some embodiments, an apparatus includes a simulated medicament delivery device and an electronic circuit system. The simulated medicament delivery device, which can be, for example, a pen injector, an auto-injector, an inhaler or a transdermal delivery device, is configured to simulate an actual medicament delivery device. The simulated medicament delivery device includes a housing, a safety lock and a cover. The safety lock is configured to simulate a safety lock of the actual medicament delivery device. The cover is removably disposed about at least a portion of the housing. The electronic circuit system is configured to output a first plurality of electronic outputs when the cover is removed from the housing a first time. The electronic circuit system is configured to output a second plurality of electronic outputs when the cover is removed from the housing a second time. The second plurality of electronic outputs are different from the first plurality of electronic outputs. In some embodiments, the first and/or the second plurality of electronic outputs can be, for example, visual outputs, audible outputs, and/or haptic outputs.

In some embodiments, a processor-readable medium storing code representing instructions to cause a processor to perform a process includes code to output a first electronic output associated with a use of a simulated medicament delivery device when a cover is removed from the simulated medicament delivery device a first time. The processor-readable medium includes code to output a second electronic output associated with a use of the simulated medicament delivery device when the cover is removed a second time. The second electronic output is different from the first electronic output. The simulated medicament delivery device can be, for example, a pen injector, an auto-injector, an inhaler or a transdermal delivery device. In some embodiments, the first and/or second electronic outputs can be, for example, a visual output, an audible output, and/or a haptic output.

As used in this specification and the appended claims, the words "proximal" and "distal" refer to direction closer to and away from, respectively, an operator (e.g., surgeon, physician, nurse, technician, etc.) of the medical device. Thus, for example, the end of the medicament delivery device contacting the patient's body would be the distal end of the medicament delivery device, while the end opposite the distal end would be the proximal end of the medicament delivery device.

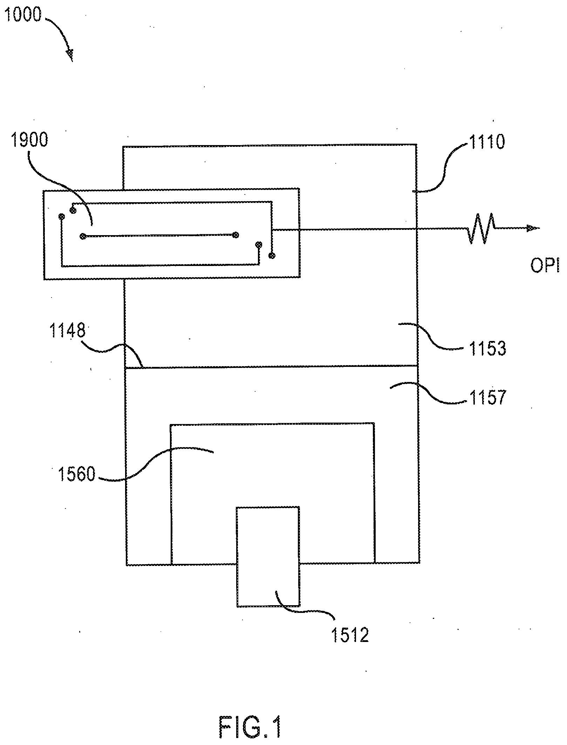

FIG. 1 is a schematic illustration of a medical injector 1000, according to an embodiment of the invention. The medical injector 1000 includes a housing 1110, a medicament container 1560, a medicament delivery member 1512 and an electronic circuit system 1900. The housing 1110 includes a sidewall 1148 that defines a first region 1157 and a second region 1153 within the housing 1110. More particularly, the sidewall 1148 physically isolates the first region 1157 from the second region 1153. Said another way, the sidewall 1148 is devoid of openings such that the first region 1157 is fluidically and/or physically isolated from the second region 1153. Said yet another way, the sidewall 1148 is disposed between the first region 1157 and the second region 1153 such that the first region 1157 is separated from the second region 1153. Although the first region 1157 and the second region 1153 are shown in FIG. 1 as being two-dimensional areas, in some embodiments, the first region 1157 and/or the second region 1153, can be fully enclosed volumes within the housing, and/or volumes within the housing 1110 having an opening to an area outside of the housing. Similarly stated, the first region and/or the second region can be cavities, defined by the housing 1110 and/or the sidewall 1148.

The medicament container 1560, which can be, for example, a pre-filled cartridge, a vial, an ampule or the like, is disposed within the first region 1157 of the housing 1110. At least a portion of the medicament delivery member 1512 is disposed within the first region 1157 of the housing 1110. In some configurations, the medicament delivery member 1512 can be in fluid communication with the medicament container 1560. In this manner, a medicament can be conveyed from the medicament container 1560 to a region outside the housing 1110 via the medicament delivery member 1512. The medicament delivery member 1512 can include, for example, a needle and/or a nozzle.

At least a portion of the electronic circuit system 1900 is disposed within the second region 1153 of the housing 1110. Accordingly, the portion of the electronic circuit system 1900 is disposed within the housing 1110 such that the portion of the electronic circuit system 1900 is fluidically and/or physically isolated from the medicament container 1560 and/or the medicament delivery member 1512.

The electronic circuit system 1900 is configured to output an electronic output OP1 associated with a use of the medical injector 1000. For example, in some embodiments, the electronic output OP can be associated with an instruction for using the medical injector 1000. In other embodiments, the electronic output OP1 can be a post-use instruction, such as, for example, a recorded message notifying the user that the injection is complete, instructing the user on post-injection disposal and safety procedures, instructing the user to seek post-injection medical treatment, and/or the like. In yet other embodiments, the electronic output OP1 can be associated with the patient's compliance in using medical injector 1000. In some embodiments, the electronic output OP1 can be associated with an actuation of the medical injector 1000. Said another way, the electronic circuit system 1900 can be configured to output the electronic output OP1 in response to actuation of the medical injector 1000.

The electronic output OP1 can be, for example, a visual output such as, for example, a text message to display on a screen (not shown), and/or an LED. In some embodiments, the electronic output OP1 can be an audio output, such as, for example, recorded speech, a series of tones, and/or the like. In other embodiments, the electronic output OP1 can be a wireless signal configured to be received by a remote device.

The medical injector 1000 can be any suitable medical injector for injecting medicament into a body of a patient. For example, the medical injector 1000 can be a syringe, pen injector, auto-injector or the like. In some embodiments, the medical injector 1000 can be a chronic-care injector. Said another way, the medical injector 1000 can be a reusable device containing multiple doses of medicament. For example, a medical injector 1000 having multiple doses of medicament can be used to manage insulin delivery or the delivery of other medicaments (e.g., to treat Multiple Sclerosis, Anemia, Rhuematoid Arthritis, Osteoporosis or the like), which can require daily, weekly, and/or monthly injections. In other embodiments, the medical injector 1000 can be a single-use device. Said another way, the medical injector 1000 can contain a single dose of medicament. In some embodiments, medical injector 1000 can include the same dosage of a medicament, and can be prescribed as a part of a chronic-care medicament regimen, clinical trial, or the like. In other embodiments, medical injector 1000 can include different dosages, and/or different medicament compositions.

The sidewall 1148 can be any suitable structure to isolate the first region 1157 within the housing 1110 from the second region 1153 within the housing 1110. In some embodiments, the sidewall 1148 can be rigid. In other embodiments, the sidewall 1148 can be a movable member such as, for example, a piston. In yet other embodiments, the sidewall 1148 can be a flexible member such as, for example, a diaphragm. In some embodiments, the sidewall 1148 can be constructed from a transparent material such that light can pass from the first region 1157 to the second region 1153, and vice versa. A transparent sidewall can be used in conjunction with an optical sensor. The sidewall 1148 can be integrally formed with the housing 1110 or can be formed separately from the housing 1110.

The electronic circuit system 1900 can include any suitable electronic components operatively coupled to produce and/or output the electronic output OP1 and/or to perform the functions described herein. The electronic circuit system 1900 can be similar to the electronic circuit systems described in U.S. Patent Application Serial Number 11/621,236, entitled "Devices, Systems and Methods for Medicament Delivery," filed January 9, 2007.

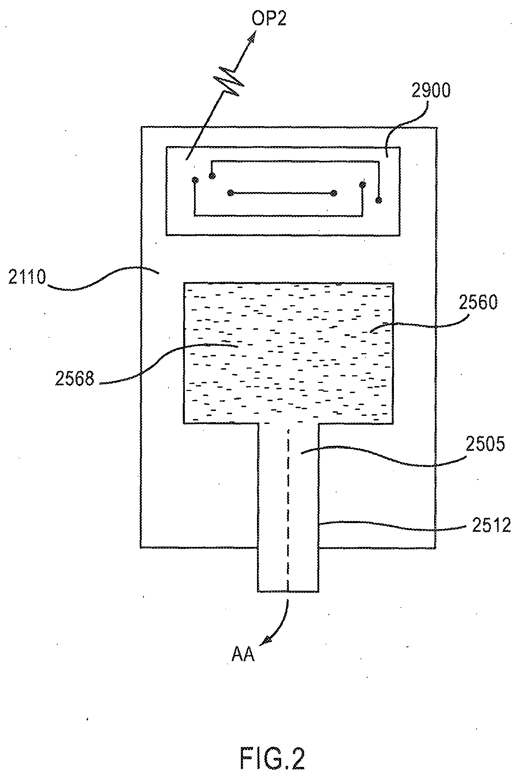

FIG. 2 is a schematic illustration of a medicament delivery device 2000, according to an embodiment of the invention. The medicament delivery device 2000 includes a housing 2110, a medicament container 2560, a medicament delivery member 2512 and an electronic circuit system 2900. The medicament container 2560, which can be, for example, a pre-filled cartridge, a vial, an ampule or the like, is disposed within the housing 2110. At least a portion of the medicament delivery member 2512 is disposed within the housing 2110. The medicament delivery member 2512 can include any suitable member configured to convey a medicament from the medicament container 2560 to a location within a patient's body. For example, in some embodiments, the medicament delivery member 2512 can be a needle, a nozzle, and/or an inhaler mouth piece.

In use, the medicament delivery member 2512 can be in fluid communication with the medicament container 2560. In this manner, the medicament delivery member 2512 and the medicament container 2560 can define a medicament delivery path 2505 through which a medicament 2568 can be conveyed from the medicament container 2560 to a location outside the housing 2110 via the medicament delivery member 2512 as shown by arrow AA. In some embodiments, the medicament delivery path 2505 can include portions of a lumen defined by the medicament delivery member 2512 and/or the connection between the medicament delivery member 2512 and the medicament container 2560.

The electronic circuit system 2900 is coupled to the housing 2110 and is fluidically and/or physically isolated from the medicament delivery path 2505. The electronic circuit system 2900 is configured to output an electronic output OP2 in response to a delivery of the medicament 2568 via the medicament delivery path 2505. In this manner, the electronic circuit system 2900 can output the electronic output OP2 in an unobtrusive manner and/or without impeding the delivery of the medicament 2568 through the medicament delivery path 2505. In some embodiments, for example, the electronic output OP2 can be a post-use instruction, such as, for example, a recorded message notifying the user that the injection is complete, instructing the user on post-injection disposal and safety procedures, instructing the user on post-injection medical treatment, and/or the like. In other embodiments, the electronic output OP2 can be associated with the patient's compliance in using the medicament delivery device 2000. For example, in some embodiments, the electronic output OP2 can be a signal sent to a compliance tracking monitor to record the data and/or time of use of the medicament delivery device 2000.

The electronic output OP2 can be, for example, a visual output such as, for example, a text message to display on a screen (not shown), and/or an LED. In some embodiments, the electronic output OP2 can be an audio output, such as, for example, recorded speech, a series of tones, and/or the like. In other embodiments, the electronic output OP2 can be a wireless signal configured to be received by a remote device.

The medicament delivery device 2000 can be any suitable medicament delivery device for delivering the medicament 2568 to a body of a patient. For example, the medicament delivery device 2000 can be a syringe, pen injector, auto-injector, inhaler or the like. In some embodiments, the medicament delivery device 2000 can be a chronic-care delivery device. Said another way, the medicament delivery device 2000 can be a reusable device containing multiple doses of medicament 2568. In other embodiments, the medicament delivery device 2000 can be a single-use device. Said another way, the medicament delivery device 2000 can contain a single dose of medicament 2568.

The electronic circuit system 2900 can include any suitable electronic components operatively coupled to produce and/or output the electronic output OP2 and/or to perform the functions described herein. The electronic circuit system 1900 can be similar to the electronic circuit system 1900 as described above with reference to FIG. 1.

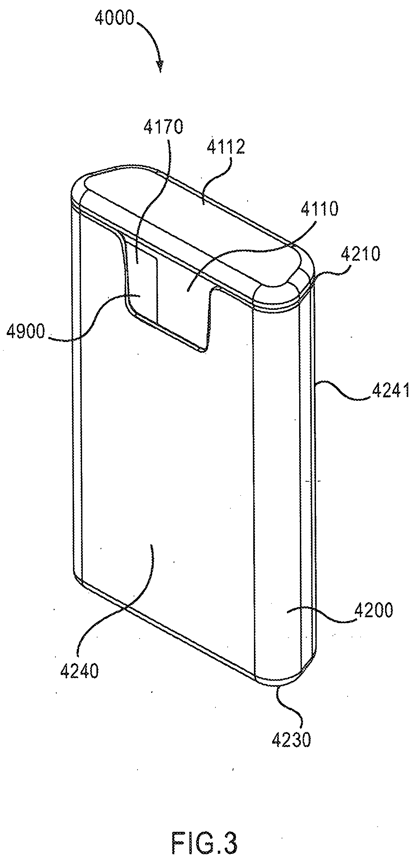

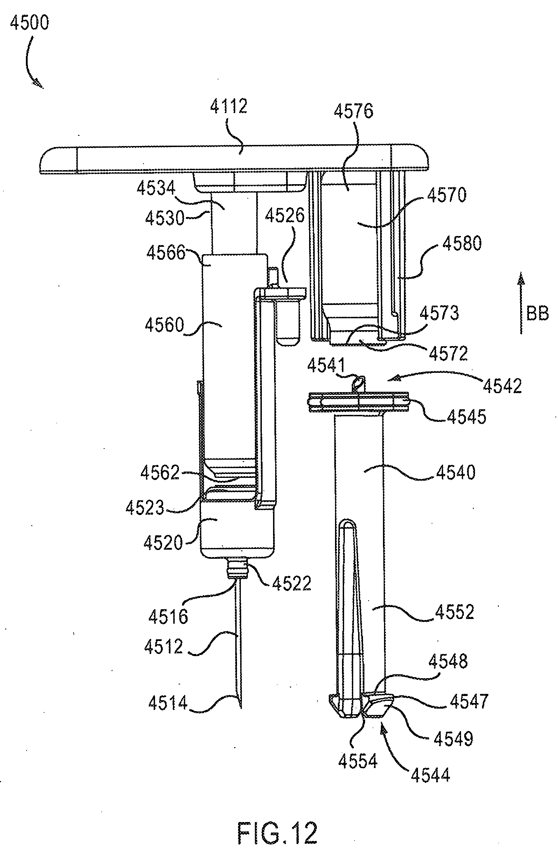

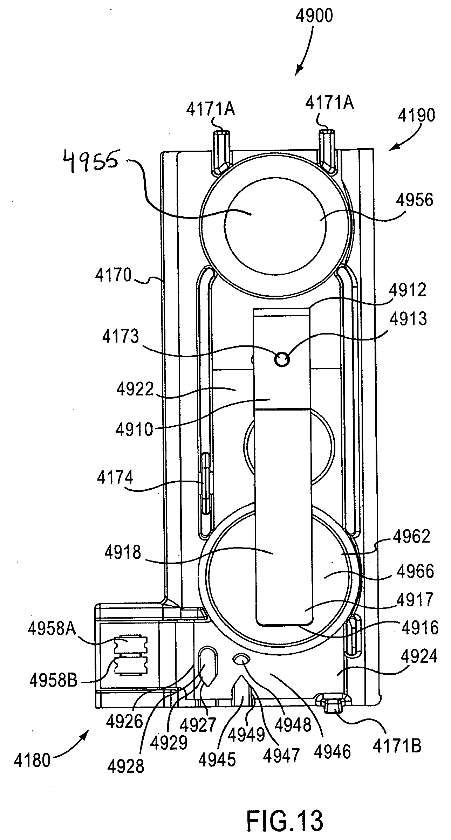

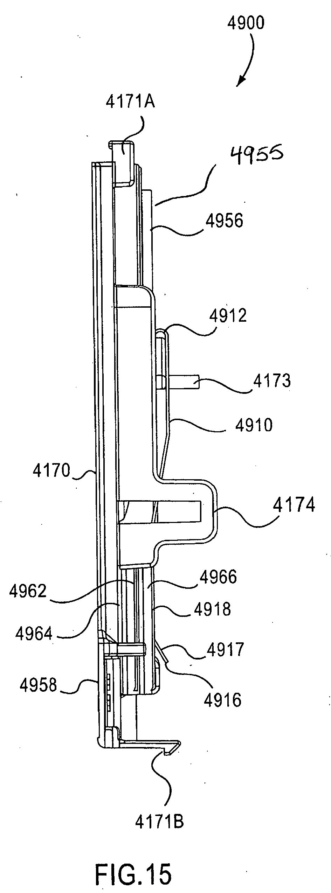

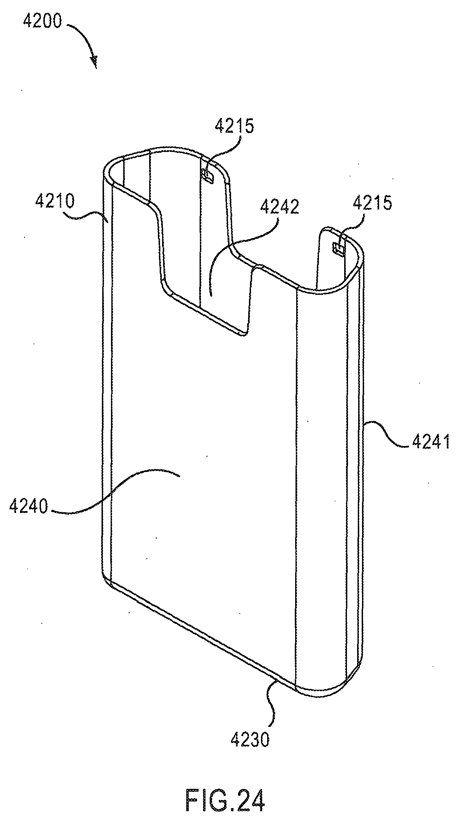

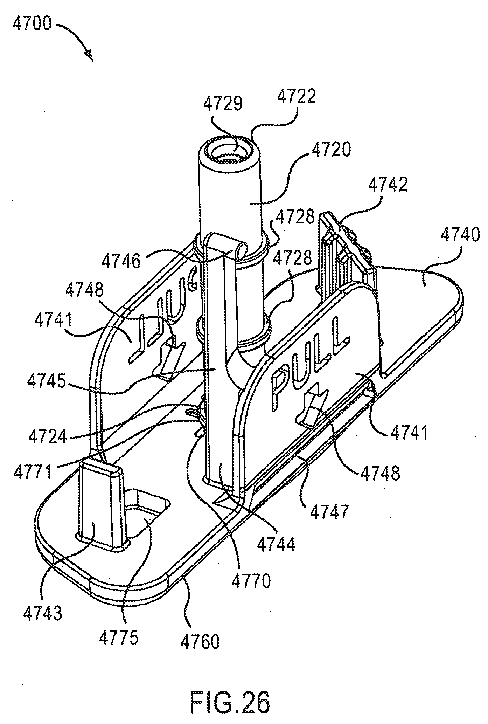

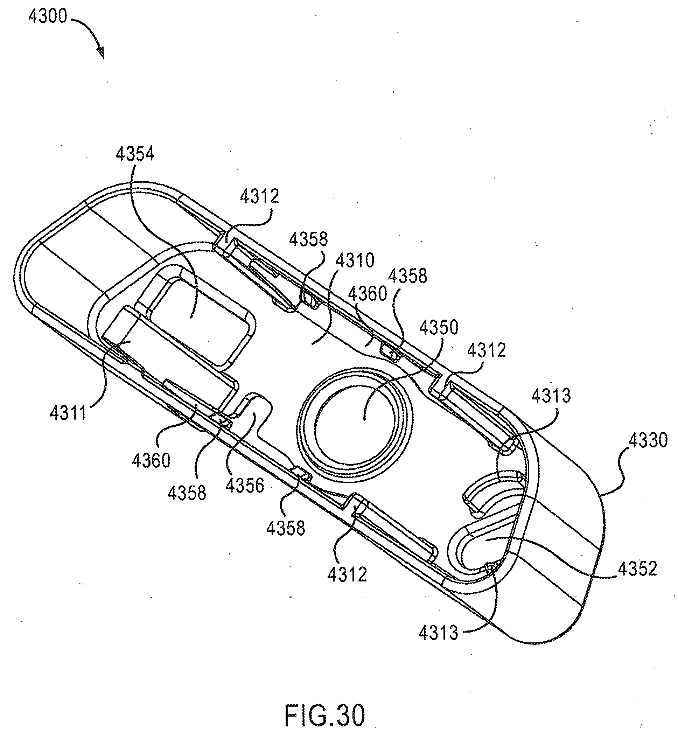

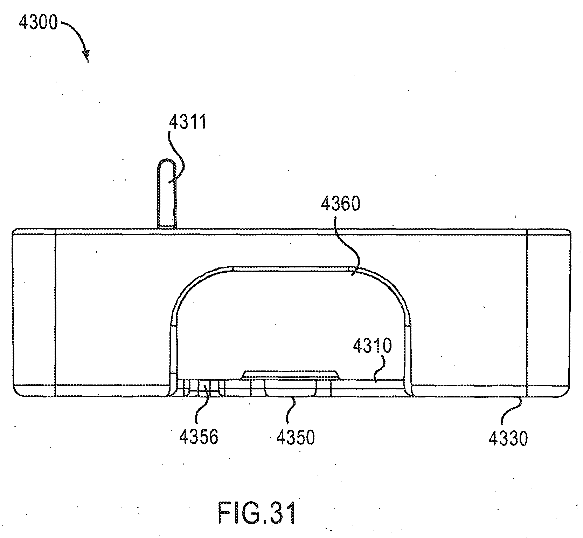

FIGS. 3-34 show a medical injector 4000, according to an embodiment of the invention. FIGS. 3-4 are perspective views of the medical injector 4000 in a first configuration (i.e., prior to use). The medical injector 4000 includes a housing 4110, a delivery mechanism 4500 (see e.g., FIG. 12), an electronic circuit system 4900 (see e.g., FIGS. 13-23), a cover 4200 (see e.g., FIGS. 24-25), a safety lock 4700 (see e.g., FIGS. 26-29) and a base 4300 (see e.g., FIGS. 30-31). A discussion of the components of the medical injector 4000 will be followed by a discussion of the operation of the medical injector 4000.

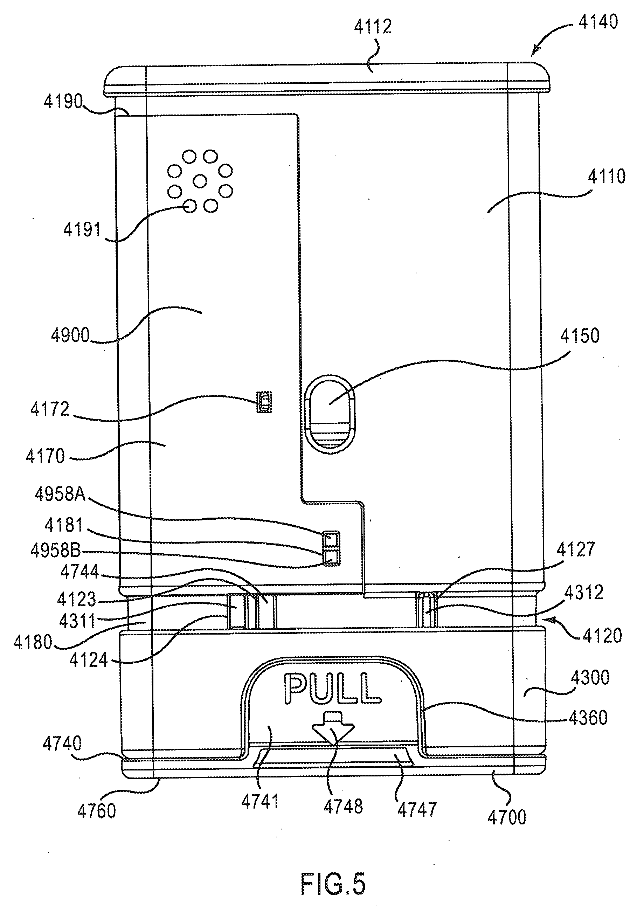

As shown in FIGS. 5-11, the housing 4110 has a proximal end portion 4140 and a distal end portion 4120. The housing 4110 defines a first status indicator aperture 4150 and a second status indicator aperture 4151. The first status indicator aperture 4150 defined by the housing 4110 is located on a first side of the housing 4110, and the second status indicator aperture 4151 of the housing 4110 is located on a second side of the housing 4110. The status indicator apertures 4150, 4151 can allow a patient to monitor the status and/or contents of a medicament container 4560. For example, by visually inspecting the status indicator apertures 4150, 4151, a patient can determine whether the medicament container 4560 contains a medicament and/or whether a medicament has been dispensed.

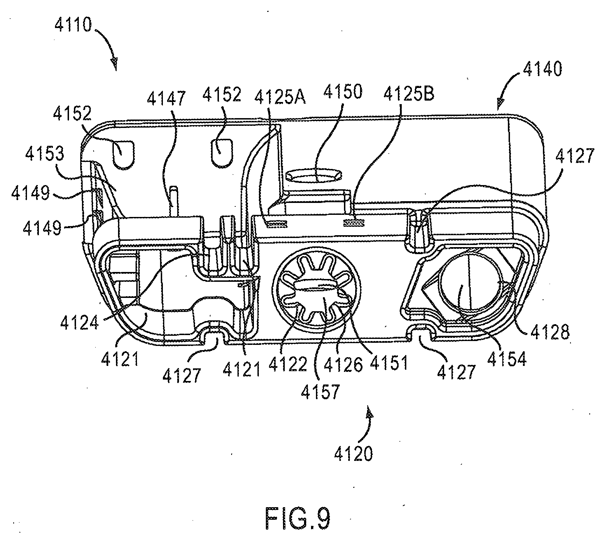

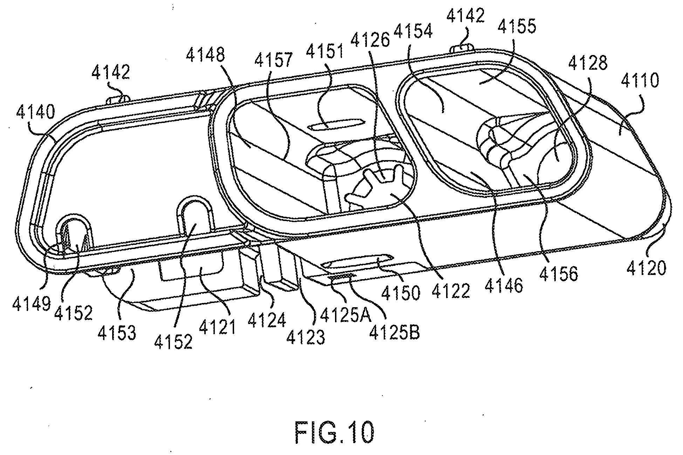

As shown in FIGS. 9 and 10, the housing 4110 defines a gas cavity 4154, a medicament cavity 4157 and an electronic circuit system cavity 4153. The gas cavity 4154 has a proximal end portion 4155 and a distal end portion 4156. The gas cavity 4154 is configured to receive the gas container 4570 and the release member 4540 of the medicament delivery mechanism 4500 (see e.g., FIG. 12) as described in further detail herein. The proximal end portion 4155 of the gas cavity 4154 is configured to receive the gas container retention member 4580 of the proximal cap 4112 of the housing 4110, as described in further detail herein. The gas cavity 4154 is in fluid communication with the medicament cavity 4157 via a gas passageway 4144, as described in further detail herein, and the gas cavity 4154 is in fluid communication with a region outside the housing 4110 via a safety lock aperture 4128.

The medicament cavity 4157 is configured to receive a portion of the delivery mechanism 4500. In particular, the carrier 4520, the moveable member 4530 and the needle 4512 of the medicament delivery mechanism 4500 are movably disposed in the medicament cavity 4157. The medicament cavity 4157 is in fluid communication with a region outside the housing 4110 via a needle aperture 4122.

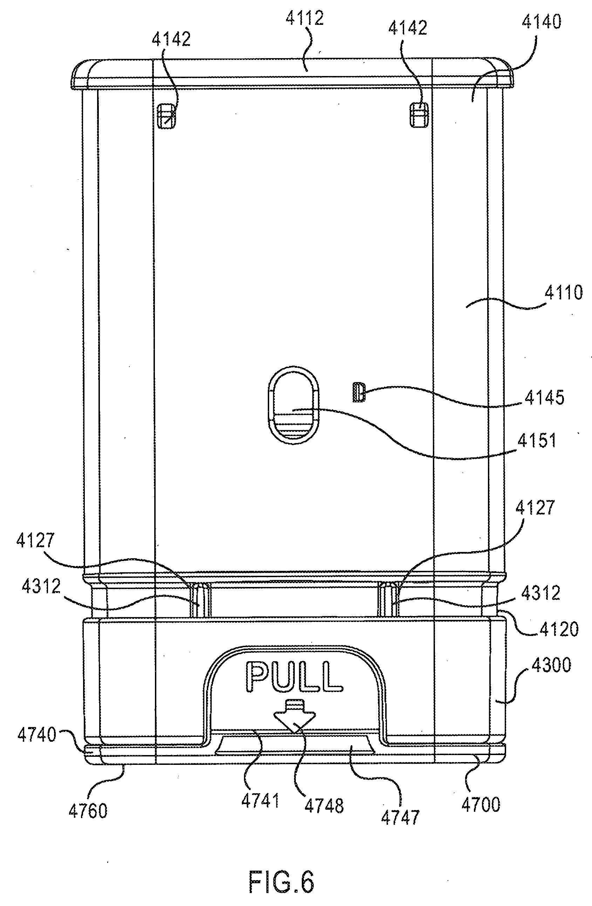

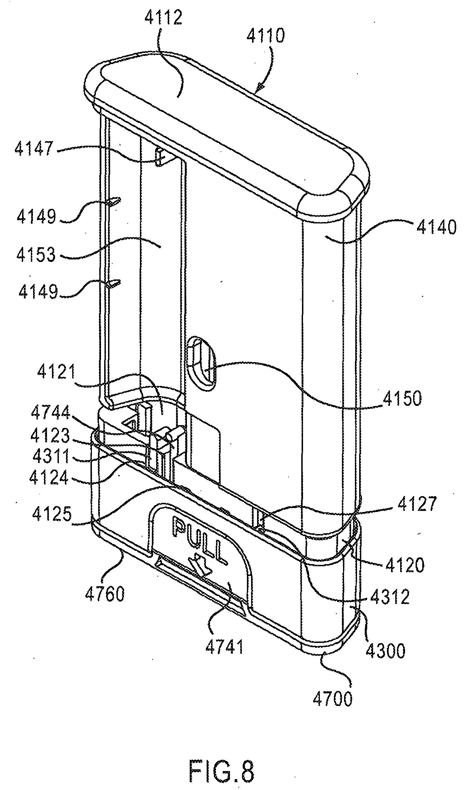

The electronic circuit system cavity 4153 is configured to receive the electronic circuit system 4900. The housing 4110 has protrusions 4149 (see e.g., FIG. 8) configured to stabilize the electronic circuit system 4900 when the electronic circuit system 4900 is disposed within the electronic circuit system cavity 4153. The housing 4110 also defines connection apertures 4152 configured to receive connection protrusions 4171A and 4171B (see e.g., FIG. 13) of the electronic circuit system 4900, and aperture 4145 (see e.g., FIG. 6) configured to receive a portion of a protrusion 4174 of the electronic circuit system 4900. In this manner, the electronic circuit system 4900 can be coupled to the housing 4110 within the electronic circuit system cavity 4153. In other embodiments, the electronic circuit system 4900 can be coupled within the electronic circuit system cavity 4153 by other suitable means such as an adhesive, a clip and/or the like.

The electronic circuit system cavity 4153 is fluidically and/or physically isolated from the gas cavity 4154 and/or the medicament cavity 4157 by a sidewall 4148. Said another way, the electronic circuit system cavity 4153 is acoustically separated from the gas cavity 4154 and/or the medicament cavity 4157. As described in more detail herein, the electronic circuit system cavity 4153 can function as an acoustic enclosure to enhance the magnitude and/or quality of the audible output produced by the electronic circuit system 4900. The sidewall 4148 can be any suitable structure to isolate the electronic circuit system cavity 4153 within the housing 4110 from the gas cavity 4154 and/or the medicament cavity 4157 within the housing 4110. Similarly, the gas cavity 4154 and the medicament cavity 4157 are separated by a sidewall 4146. In some embodiments, sidewall 4146 can be similar to the sidewall 4148, which isolates the gas cavity 4154 and the medicament cavity 4157 from the electronic circuit system cavity 4153. In other embodiments, the gas cavity 4154 can be fluidically, acoustically and/or physically isolated from the medicament cavity 4157.

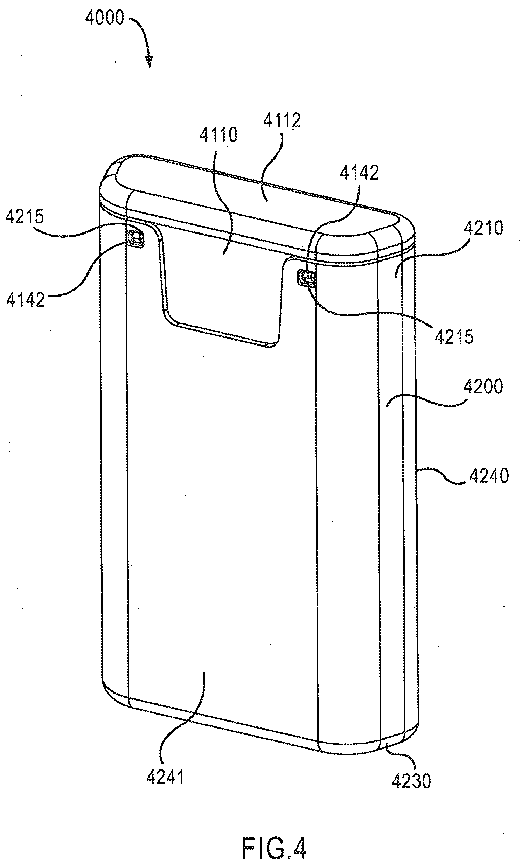

The proximal end portion 4140 of the housing 4110 includes a proximal cap 4112, a speaker protrusion 4147 (see e.g., FIGS. 8 and 9), and cover retention protrusions 4142 (see e.g., FIGS. 4 and 6). The speaker protrusion 4147 is configured to maintain a position of an audible output device 4956 of the electronic circuit system 4900 relative to the housing 4110 and/or an electronic circuit system cover 4170 when the electronic circuit system 4900 is attached to the housing 4110, as described herein. As described in more detail below, in some embodiments, the speaker protrusion 4147 can press the front portion 4957 of the audible output device 4956 against the electronic circuit system cover 4170 to form a substantially airtight (e.g., a substantially hermetic) seal between the front portion 4957 of the audible output device 4956 and the electronic circuit system cover 4170. A substantially airtight seal can reduce undesirable audible noise that can result from air leaking through a gap between the front portion 4957 of the audible output device 4956 and the electronic circuit system cover 4170. Moreover, the speaker protrusion 4147 can reduce or minimize undesirable vibration of the audible output device 4956 by holding the audible output device 4956 in a substantially fixed position relative to the electronic circuit system cover 4170. Cover retention protrusions 4142 are configured to be received within corresponding openings 4215 on the cover 4200. In this manner, as described in more detail herein, the cover 4200 can be removably coupled to and disposed about at least a portion of the housing 4110.

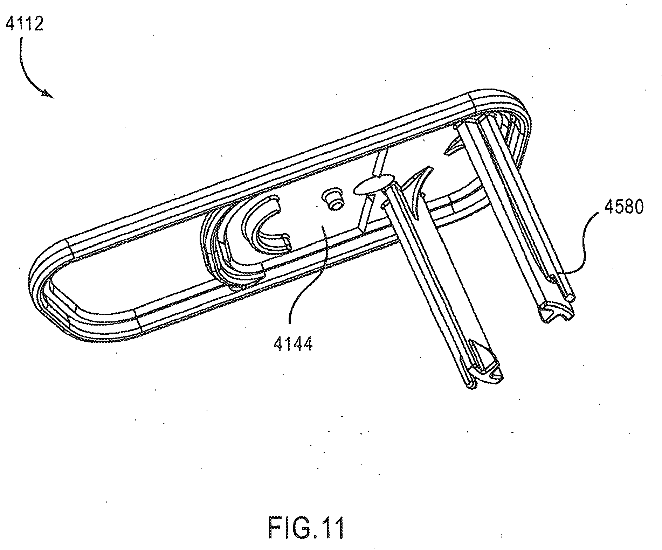

As shown in FIG. 11, the proximal cap 4112 includes a gas container retention member 4580 and defines a gas passageway 4144. The gas container retention member 4580 is configured to receive and/or retain a gas container 4570 that can contain a pressurized gas. The gas passageway 4144 is configured to allow for the passage of gas contained in the gas container 4570 from the gas cavity 4154 to the medicament cavity 4157, as further described herein. Said another way, the gas passageway 4144 places the gas cavity 4154 in fluid communication with the medicament cavity 4157.

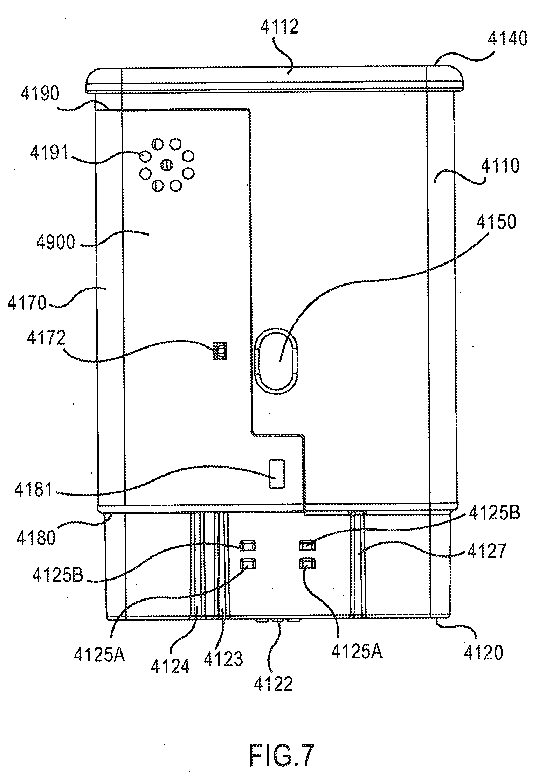

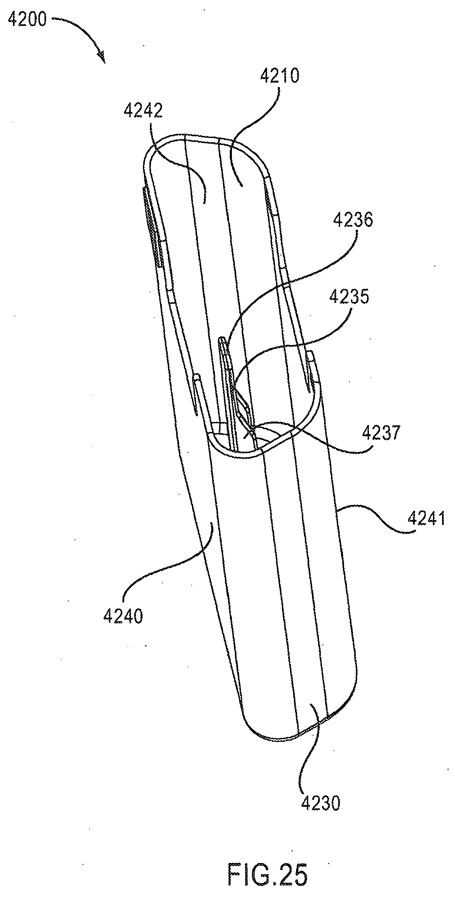

As shown in FIGS. 7 and 9, the distal end portion 4120 of the housing 4110 defines a battery isolation protrusion aperture 4121, a needle aperture 4122, a safety lock actuator groove 4123, a safety lock aperture 4128, a base actuator groove 4124, base retention recesses 4125A, 4125B, and base rail grooves 4127. The battery isolation protrusion aperture 4121 is configured to receive the battery isolation protrusion 4235 of the cover 4200 (see e.g., FIG. 25), as described in further detail herein.

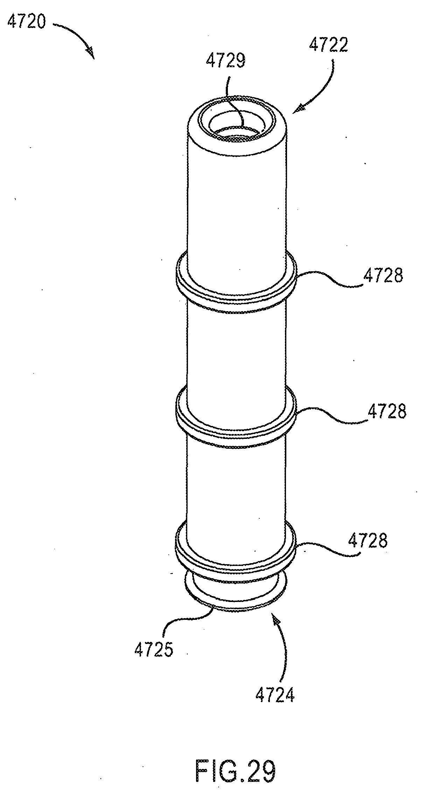

The needle aperture 4122 is configured to allow the needle 4512 (see e.g., FIG. 12) to exit the housing 4110 when the medical injector 4000 is actuated. The portion of the sidewall of the housing 4110 that defines the needle aperture 4122 includes multiple sheath retention protrusions 4126. In some embodiments, the sheath retention protrusions can interact with the a plurality of ribs 4728 of the needle sheath 4720 (see e.g. FIG 29) to maintain a position of the needle sheath 4720 relative to the safety lock 4700 when the safety lock 4700 is coupled to the housing 4110 and/or when the safety lock 4700 is being removed from the housing 4110.

The safety lock actuator groove 4123 is configured to receive an actuator 4744 of the safety lock 4700. As described in more detail herein, the actuator 4744 is configured to engage and/or activate the electronic circuit system 4900 when the safety lock 4700 is moved with respect to the housing 4110. The safety lock aperture 4128 is configured to receive a safety lock protrusion 4742 (see e.g., FIGS. 25 and 26). As described in more detail below, the safety lock protrusion 4742 is received within an opening 4554 between extensions 4552 of a release member 4540 such that activation of the medical injector 4000 is prevented when the safety lock 4700 is in place. The safety lock 4700, its components and functions are further described herein.

The distal base retention recesses 4125A are configured to receive the base connection knobs 4358 of the base 4300 (see e.g., FIG. 30) when the base 4300 is in a first position relative to the housing 4110. The proximal base retention recesses 4125B are configured to receive the base connection knobs 4358 of the base 4300 when the base 4300 is in a second position relative to the housing 4110. The base retention recesses 4125A, 4125B have a tapered proximal sidewall and a non-tapered distal sidewall. This allows the base retention recesses 4125A, 4125B to receive the base connection knobs 4358 such that the base 4300 can move proximally relative to the housing 4110, but cannot move distally relative to the housing 4110. Said another way, the distal base retention recesses 41125A are configured to prevent the base 4300 from moving distally when the base 4300 is in a first position and the proximal base retention recesses 4125B are configured to prevent the base 4300 from moving distally when the base 4300 is in a second position. Similarly stated, the proximal base retention recesses 4125B and the base connection knobs 4358 cooperatively prevent "kickback" after the medical injector 4000 is actuated.

The base actuator groove 4124 is configured to receive an actuator 4311 of the base 4300. As described in more detail herein, the actuator 4311 of the base 4300 is configured to engage the electronic circuit system 4900 when the base 4100 is moved with respect to the housing 4110. The base rail grooves 4127 are configured to receive the guide members 4312 of the base 4300. The guide members 4312 of the base 4300 and the base rail grooves 4127 of the housing 4110 engage each other in a way that allows the guide members 4312 of the base 4300 to slide in a proximal and/or distal direction within the base rail grooves 4127 while limiting lateral movement of the guide members 4312. This arrangement allows the base 4300 to move in a proximal and/or distal direction with respect to the housing 4110 but prevents the base 4300 from moving in a lateral direction with respect to the housing 4110.

FIG. 12 shows the medicament delivery mechanism 4500 of the medical injector 4000. The medical injector 4000 is similar to the auto-injectors described in U.S. Patent Application Publication Number 2007/01459925, entitled "Devices, Systems and Methods for Medicament Delivery," filed November 21, 2006. Accordingly, only an overview of the medicament delivery mechanism 4500 and related operation of the medical injector 4000 is included below.

The medicament delivery mechanism 4500 includes a needle 4512, a carrier 4520, a movable member 4530, a medicament container 4560, a gas container 4570, and a release member 4540. As described above, the needle 4512, carrier 4520, movable member 4530 and medicament container 4560 are disposed within the medicament cavity 4157 of the housing 4110. The gas container 4570 and the release member 4540 are disposed within the gas cavity 4154 of the housing 4110.

The release member 4540 has a proximal end portion 4542 and a distal end portion 4544, and is movably disposed within the distal end portion 4156 of the gas cavity 4154. The proximal end portion 4542 of the release member 4540 includes a sealing member 4545 and a puncturer 4541. The sealing member 4545 is configured to engage the sidewall of the housing 4110 defining the gas cavity 4154 such that the proximal end portion 4155 of the gas cavity 4154 is fluidically isolated from the distal end portion 4156 of the gas cavity 4154. In this manner, when gas is released from the gas container 4570, the gas contained in the proximal end portion 4155 of the gas cavity 4154 is unable to enter the distal end portion 4156 of the gas cavity 4154. The puncturer 4541 of the proximal end portion 4542 of the release member 4540 is configured to contact and puncture a frangible seal 4573 on the gas container 4570 when the release member 4540 moves proximally within the gas cavity 4154, as shown by the arrow BB in FIG. 12.

The distal end portion 4544 of the release member 4540 includes extensions 4552. The extensions 4552 include projections 4547 that include tapered surfaces 4549 and engagement surfaces 4548. Further, the extensions 4552 define an opening 4554 between the extensions 4552. The tapered surfaces 4549 of the projections 4547 are configured to contact protrusions 4313 on a proximal surface 4310 of the base 4300 (see e.g., FIG. 30). The engagement surfaces 4548 of the projections 4547 are configured to extend through the safety lock aperture 4128 of the housing 4110 and contact a distal surface of the housing 4110. In this manner, the engagement surfaces 4548 of the projections 4547 limit proximal movement of the release member 4540 when the engagement surfaces 4548 are in contact with the distal surface of the housing 4110. The tapered surfaces 4549 of the projections 4547 are configured to contact protrusions 4313 on a proximal surface 4310 of the base 4300 (see e.g., FIG. 30). In this manner, proximal movement of the base 4300 causes the extensions 4552 to move together, thereby releasing the engagement surfaces 4548 from the housing 4110 and allowing the release member 4540 to move proximally within the gas cavity 4154.

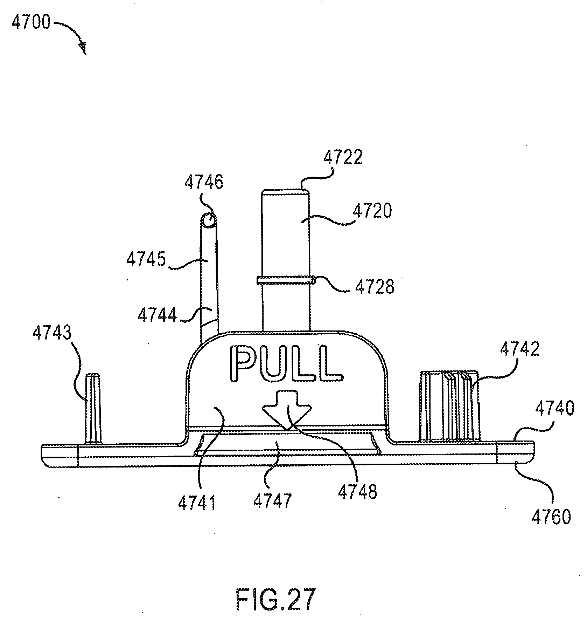

The opening 4554 defined by the extensions 4552 is configured to receive the safety lock protrusion 4742 of the safety lock 4700 (see e.g., FIG. 27). The safety lock protrusion 4742 is configured to prevent the extensions 4552 from moving closer to each other. Said another way, the safety lock protrusion 4742 is configured to ensure that the extensions 4552 remain apart and the engagement surfaces 4548 of the projections 4547 remain in contact with the distal end portion 4120 of the housing 4110. In some embodiments, for example, the release member 4540 and/or the extensions 4552 can be constructed from any suitable material configured to withstand deformation that may occur when exposed to a load over an extended period of time. In some embodiments, for example, the release member 4540 and/or the extensions 4552 can be constructed from brass.

The gas container 4570 includes a distal end portion 4572 and a proximal end portion 4576, and is configured to contain a pressurized gas. The distal end portion 4572 of the gas container 4570 contains a frangible seal 4573 configured to break when the puncturer 4541 of the proximal end portion 4542 of the release member 4540 contacts the frangible seal 4573. The gas container retention member 4580 of the proximal cap 4112 of the housing 4110 is configured to receive and/or retain the proximal end portion 4576 of the gas container 4570. Said another way, the position of the gas container 4570 within the gas cavity 4154 is maintained by the gas container retention member 4580.

The medicament container 4560 of the medicament delivery mechanism 4500 has a distal end portion 4562 and a proximal end portion 4566, and is configured to contain a medicament. The distal end portion 4562 of the medicament container 4560 contains a seal 4523. The seal 4523 is configured to burst when punctured by the proximal end 4516 of the needle 4512, as described below. The proximal end portion 4566 of the medicament container 4560 is configured to receive a piston portion 4534 of the movable member 4530.

The movable member 4530 of the medicament delivery mechanism 4500 is movably disposed within the medicament cavity 4157. The movable member 4530 includes a piston portion 4534 having a plunger at the distal end portion of the piston portion 4534. The piston portion 4534 is configured to move within the medicament container 4560. In this manner, the piston portion 4534 of the movable member 4530 can apply pressure to a medicament contained in the medicament container 4560. The piston portion 4534 can be constructed of a resilient, durable, and/or sealing material, such as a rubber.

The carrier 4520 of the medicament delivery mechanism 4500 includes a distal end portion 4522 and a proximal end portion 4526. The medicament container 4560 is coupled to the carrier 4520 via a "snap-fit" connection (not shown) such that the medicament container 4560 can move relative to the carrier 4520 between a first configuration and a second configuration during an injection event. In the first configuration, the carrier 4520 is configured to move within the medicament cavity 4157 such that movement of the carrier 4520 within the medicament cavity 4157 causes contemporaneous movement of the medicament container 4560 within the medicament cavity 4157. The proximal end portion 4516 of the needle 4512 is spaced apart from the seal 4523 of the medicament container 4560 when the carrier 4520 is in the first configuration. In the second configuration, the medicament container 4560 releases from the "snap-fit" causing the medicament container 4560 to move distally with respect to the carrier 4520, causing the proximal end portion 4516 of the needle 4512 to pierce the seal 4523. In this manner, the needle 4512 can be selectively placed in fluid communication with the medicament container 4560 to define a medicament delivery path (not shown).

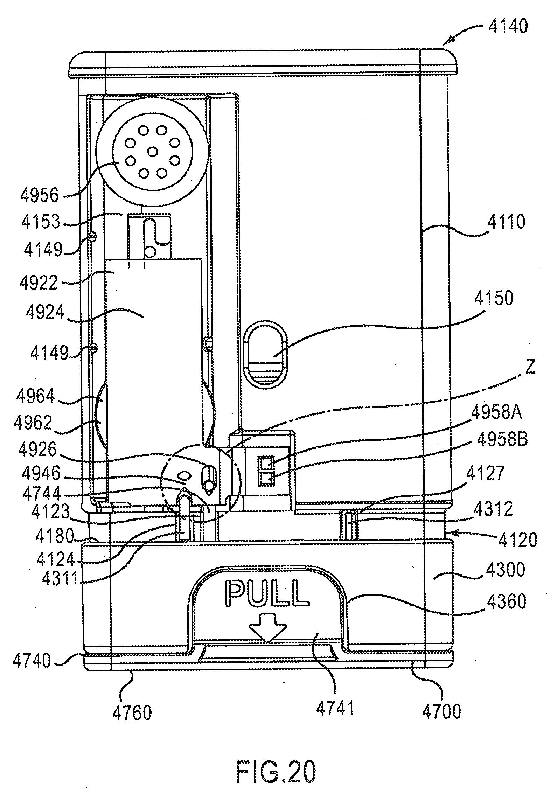

FIGS. 13-22 show the electronic circuit system 4900. The electronic circuit system 4900 of the medical injector 4000 includes an electronic circuit system housing 4170 (also referred to as the electronic circuit system cover), a printed circuit board 4922, a battery assembly 4962, an audible output device 4956, two light emitting diodes (LEDs) 4958A, 4958B and a battery clip 4910. As shown in FIG. 20, the electronic circuit system 4900 is configured to fit within the electronic circuit system cavity 4153 of the housing 4110. Accordingly, as described above, the electronic circuit system 4900 is physically, acoustically and/or fluidically isolated from the medicament cavity 4157, the gas cavity 4154 and/or the medicament delivery device 4500. As described herein, the electronic circuit system 4900 is configured to output an electronic output associated with the use of the medical injector 4000.

The electronic circuit system housing or cover 4170 of the electronic circuit system 4900 includes a distal end portion 4180 and a proximal end portion 4190. The proximal end portion 4190 includes connection protrusions 4171 A and a battery clip protrusion 4173. The connection protrusions 4171A extend from the proximal end portion 4190 of the electronic circuit system housing 4170, and are configured to be disposed within the connection apertures 4152 of the housing 4110, as described above. In this manner, the electronic circuit system 4900 can be coupled to the housing 4110 within the electronic circuit system cavity 4153. In other embodiments, the electronic circuit system 4900 can be coupled to the housing 4110 by other suitable means such as an adhesive, a clip and/or the like. As described in more detail herein, the battery clip protrusion 4173 is configured to hold the battery clip 4910 in place.

The proximal end portion 4190 of the electronic circuit system housing or cover 4170 defines multiple sound apertures 4191. The audible output device 4956 is disposed against the proximal end portion 4190 of the electronic circuit system housing 4170 such that the front portion 4957 of the audible output device 4956 is disposed adjacent the sound apertures 4191. In this manner, sound waves from the audible output device 4956 can travel from the audible output device 4956 to a region outside of the housing 4110 via the sound apertures 4191. As described above, in some embodiments, a sealing material (e.g., a compressible adhesive foam, an elastomeric o-ring, or the like) can be disposed on an outer perimeter of the front portion 4957 of the audible output device 4956 such that when the audible output device 4956 is pressed against the electronic circuit system cover 4170 by the speaker protrusion 4147, a substantially airtight seal is formed between the front portion 4957 of the audible output device 4956 and the electronic circuit system cover 4170. In this manner, sound waves produced by the front portion 4957 of the audible output device 4956 are directed through the multiple sound apertures 4191 and not through a breach or gap between the audible output device 4956 and the electronic circuit system cover 4170. In some embodiments, for example, the sealing material is a compressible adhesive foam approximately % millimeters in thickness that covers approximately 10 percent of the front portion 4957 of the audible output device 4956.

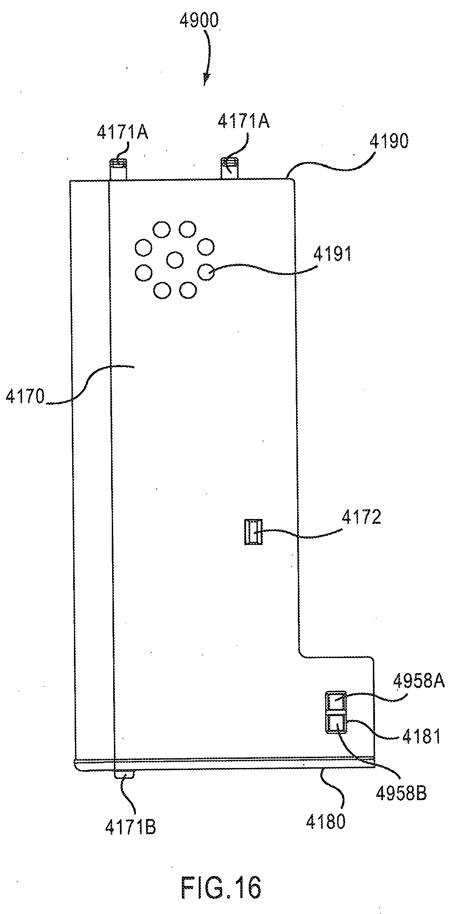

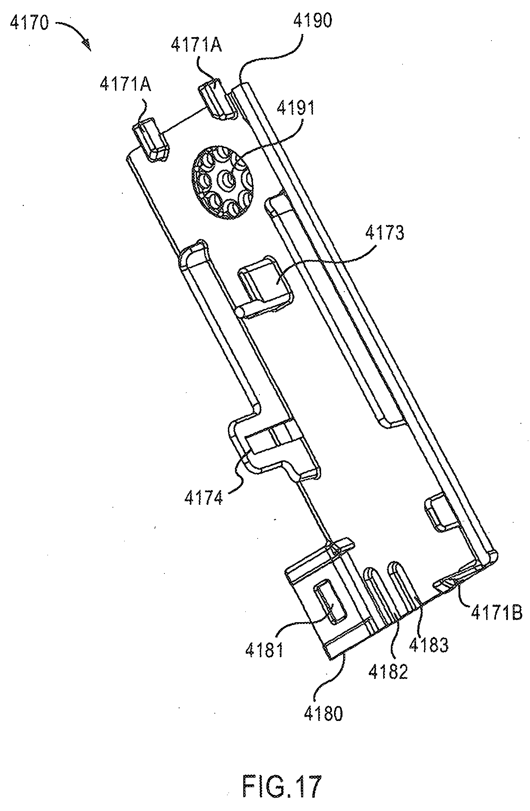

As shown in FIGS. 16 and 17, the distal end portion 4180 of the electronic circuit system housing 4170 includes a connection protrusion 4171B, a stiffening protrusion 4174, and defines an LED aperture 4181, an aperture 4172, a safety lock actuator groove 4182, and a base actuator groove 4183. The LED aperture 4181 is configured to receive the LEDs 4958A, 4958B such that a user can view the LEDs 4958A, 4958B, which are described in more detail herein.

The connection protrusion 4171B extends from the distal end portion 4180 of the electronic circuit system housing 4170, and is configured to attach the electronic circuit system 4900 to the housing 4110, as described above. The stiffening protrusion 4174 is configured to have at least a portion received within and/or accessible via the aperture 4145 in the housing 4110 (see e.g., FIG. 6). The stiffening protrusion 4174 is configured to limit the bending (e.g., buckling) of the electronic circuit system housing 4170 when the electronic circuit system housing 4170 is coupled to the housing 4110. Moreover, a user can access the stiffening protrusion 4174 via the aperture 4172. In this manner, for example, the user can disengage the stiffening protrusion 4174 from the aperture 4145.

The safety lock actuator groove 4182 of the electronic circuit system housing 4170 is configured to be disposed adjacent the safety lock actuator groove 4123 of the distal end portion 4120 of the housing 4110. In this manner, the safety lock actuator groove 4182 of the electronic circuit system housing 4170 and the safety lock actuator groove 4123 of the distal end portion 4120 of the housing 4110 collectively receive the actuator 4744 of the safety lock 4700, which is described in more detail herein. Similarly, the base actuator groove 4183 of the electronic circuit system housing 4170 is configured to be disposed about the base actuator groove 4124 of the distal end portion 4120 of the housing 4110. The base actuator groove 4183 of the electronic circuit system housing 4170 and the base actuator groove 4124 of the distal end portion 4120 of the housing 4110 collectively receive the actuator 4311 of the base 4300, which is described in more detail herein.

The electronic circuit system housing or cover 4170 is matingly coupled to the housing 4110 such that the electronic circuit system housing or cover 4170 and the electronic circuit system cavity 4153 collectively define an acoustic enclosure within which the audible output device 4956 is disposed. Said another way, the electronic circuit system cover 4170 and the electronic circuit system cavity 4153 collectively form a region, volume and/or space that is configured to minimize or attenuate noise and/or enhance the audible output of the audible output device 4956. Moreover, the volume associated with the region defined by the electronic circuit system cover 4170 and the electronic circuit system cavity 4153 is larger than the volume of the audible output device 4956 and/or the electronic circuit system 4900 disposed within the region. In this manner, the acoustic enclosure defined by the electronic circuit system cover 4170 and the electronic circuit system cavity 4153 is configured to contain a volume of air behind the audible output device 4956.

Moreover, the audible output device 4956 and the electronic circuit system cavity 4153 are collectively configured to enhance the quality and/or magnitude of the sound produced by the audible output device 4956. For example, in some embodiments, the size and/or shape of the electronic circuit system cavity 4153 can be configured such that the electronic circuit system cavity 4153 defines an acoustic resonant frequency that is within a predefined frequency range of the audible output device 4956. More particularly, in some embodiments, the electronic circuit system cavity 4153 can be configured such that the electronic circuit system cavity 4153 defines an acoustic resonant frequency that is substantially the same as a resonant frequency of the audible output device 4956. In this manner, the quality and/or magnitude of the sound produced by the audible output device 4956 can be enhanced for a particular range of frequencies. In some embodiments, the quality and/or magnitude of the sound produced by the audible output device 4956 can be enhanced for a frequency range corresponding to the frequency range of a recorded speech output.

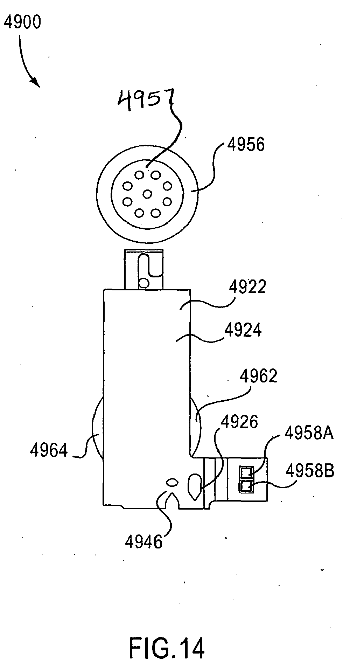

The printed circuit board 4922 of the electronic circuit system 4900 provides the structure upon which at least a portion of the electronic components of the electronic circuit system 4900 are mounted. The printed circuit board 4922 includes a substrate 4924, a first actuation portion 4926 and a second actuation portion 4946. The substrate 4924 of the printed circuit board 4922 provides a mounting surface for the electrical components necessary for the electronic circuit system 4900 to operate as desired. Moreover, the substrate 4924 includes conductive portions (e.g., copper traces, insulated wires, flexible wires or the like) to electronically couple the components. For example, the electrical components can be resistors, capacitors, inductors, switches, microcontrollers, processors and/or the like. The processor (not shown in FIGS. 14-20) can be any suitable device configured to process electronic inputs (e.g., inputs from switches or sensors) and produce electronic signals and/or outputs. The processor can be, for example, similar to the processor 5950 shown and described herein below.

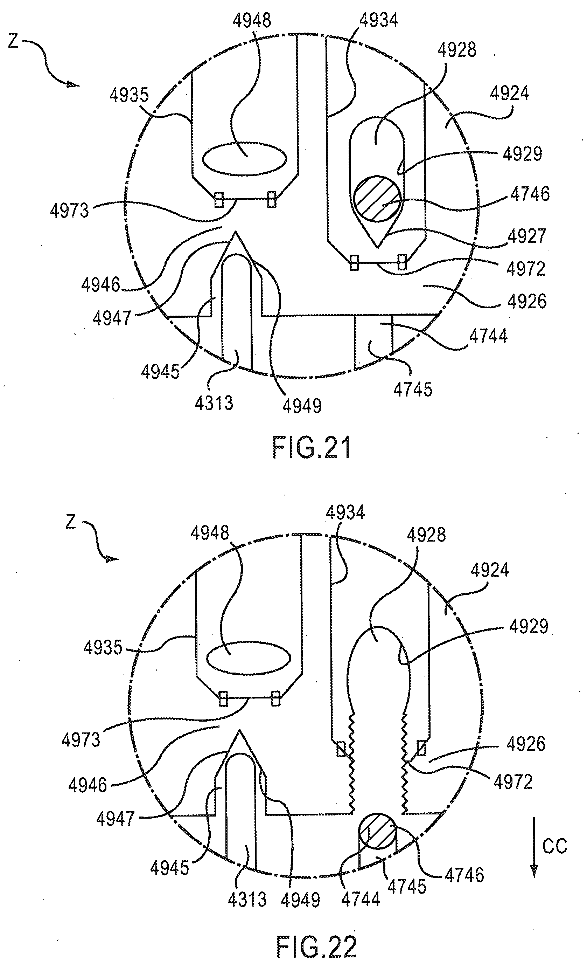

As shown in FIGS. 21-23, the first actuation portion 4926 includes a first electrical conductor 4934 and defines an opening 4928 having a boundary 4929. The opening 4928 of the first actuation portion 4926 is configured to receive a protrusion 4746 of the actuator 4744 of the safety lock 4700. The boundary 4929 of the first opening 4928 has a discontinuous shape, such as, for example, a teardrop shape, that includes a stress concentration riser 4927. The discontinuity and/or the stress concentration riser 4927 of the boundary 4929 can be of any suitable shape to cause the substrate 4924 to deform in a predetermined direction when the protrusion 4746 of the actuator 4744 of the safety lock 4700 is moved relative to the opening 4928, as shown by the arrow CC in FIG. 22.

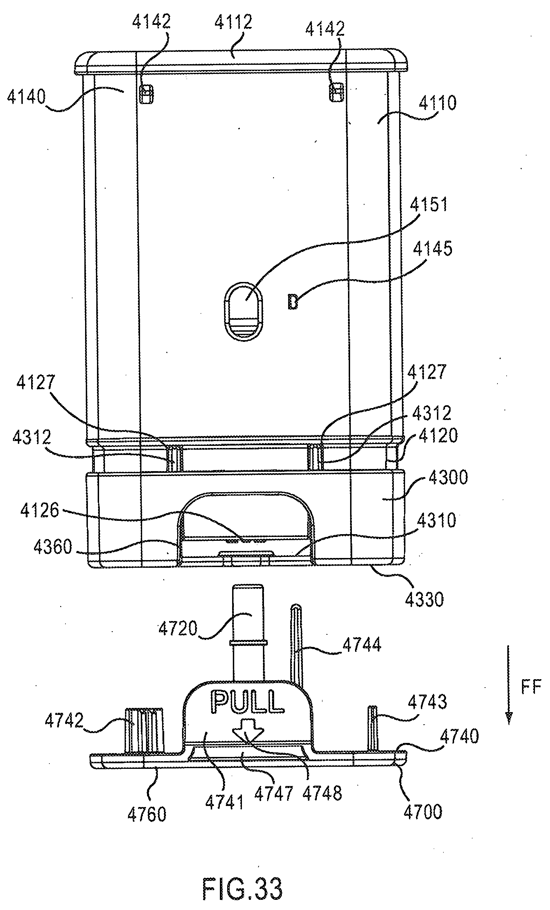

The opening 4928 is defined adjacent the first electrical conductor 4934 that electronically couples the components included in the electronic circuit system 4900. The first electrical conductor 4934 includes a first switch 4972, which can be, for example a frangible portion of the first electrical conductor 4934. In use, when the safety lock 4700 is moved from a first position (see e.g., FIG. 21) to a second position (see e.g., FIG. 22), the actuator 4744 moves in a direction substantially parallel to a plane defined by a surface of the first actuation portion 4926 of the substrate 4924. The movement of the actuator 4744 causes the protrusion 4746 to move within the first opening 4928, as indicated by the arrow CC in FIG. 22. The movement of the protrusion 4746 tears the first actuation portion 4926 of the substrate 4924, thereby separating the portion of the first electrical conductor 4934 including the first switch 4972. Said another way, when the safety lock 4700 is moved from its first position to its second position (see e.g., FIG. 33), the actuator 4744 moves irreversibly the first switch 4972 from a first state (e.g., a state of electrical continuity) to a second state (e.g., a state of electrical discontinuity). Said yet another way, when the safety lock 4700 is moved from its first position to its second position, the actuator 4744 disrupts the first electrical conductor 4934.

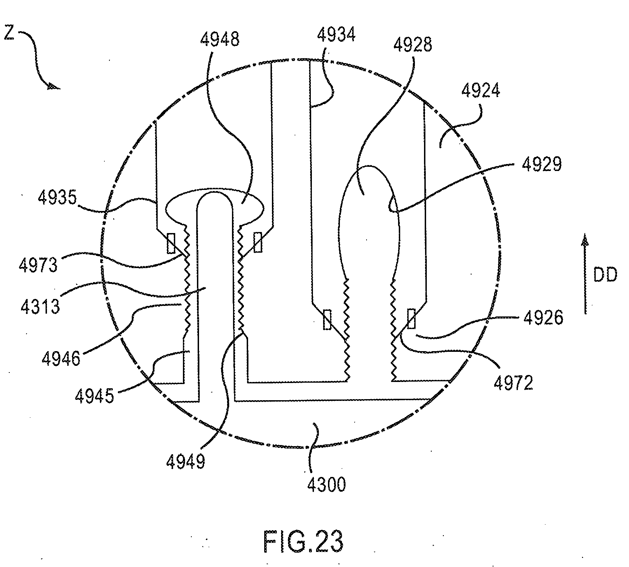

The second actuation portion 4946 includes a second electrical conductor 4935 and defines an opening 4945, having a boundary 4949 and a tear propagation limit aperture 4948. As shown in FIGS. 20-23, the opening 4945 of the second actuation portion 4946 is configured to receive a portion of an actuator 4311 of the base 4300. The boundary 4949 of the opening 4945 has a discontinuous shape that includes a stress concentration riser 4947. The discontinuity and/or the stress concentration riser 4947 of the boundary 4949 can be of any suitable shape to cause the substrate 4924 to deform in a predetermined direction when the actuator 4311 of the base 4300 is moved in a proximal direction relative to the opening 4945, as shown by the arrow DD in FIG. 23.

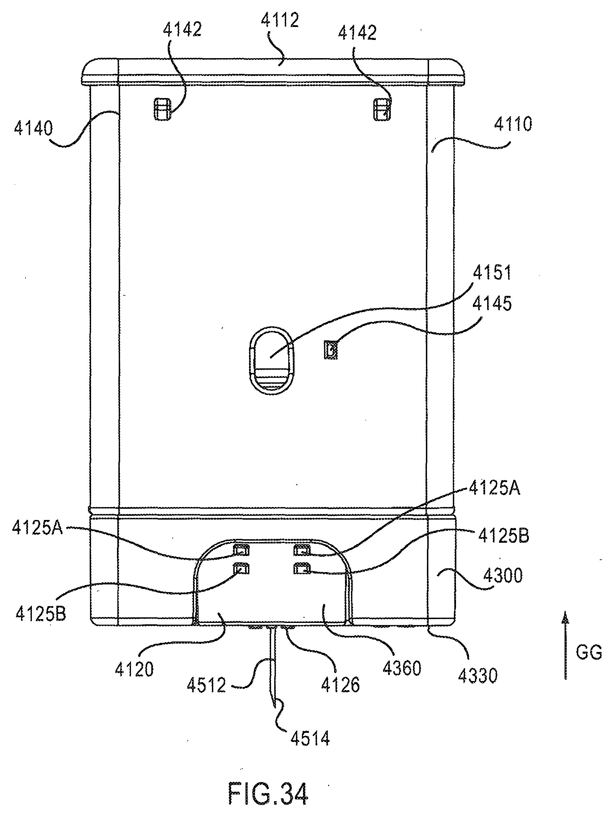

The second electrical conductor 4935 includes a second switch 4973 disposed between the opening 4945 and the tear propagation limit aperture 4948, which can be, for example, a frangible portion of the second electrical conductor 4935. In use, when the base 4300 is moved from its first position to its second position (see e.g., FIG. 34), the actuator 4311 moves in a proximal direction, substantially parallel to a plane defined by a surface of the second actuation portion 4946 of the substrate 4924. The proximal movement of the actuator 4311 tears the second actuation portion 4946 of the substrate 4924, thereby separating the portion of the second electrical conductor 4935 including the second switch 4973. Said another way, when the base 4300 is moved from its first position to its second position, the actuator 4311 moves irreversibly the second switch 4973 from a first state (e.g., a state of electrical continuity) to a second state (e.g., a state of electrical discontinuity). The tear propagation limit aperture 4948 is configured to limit the propagation of the tear in the substrate 4924 in the proximal direction. Said another way, the tear propagation limit aperture 4948 is configured to ensure that the tear in the substrate 4924 does not extend beyond the tear propagation limit aperture 4948. The tear propagation limit aperture 4948 can be any shape configured to stop the propagation of a tear and/or disruption, of the substrate 4924. For example, the tear propagation limit aperture 4948 can be oval shaped. In other embodiments, the proximal boundary of the tear propagation limit aperture 4948 can be reinforced to ensure that the tear in the substrate 4924 does not extend beyond the tear propagation limit aperture 4948.

The battery assembly 4962 of the electronic circuit system 4900 includes two batteries stacked on top of one another. The battery assembly 4962 has a first surface 4964 and a second surface 4966. The first surface 4964 of the battery assembly 4962 can contact an electrical contact (not shown) disposed on the substrate 4924. The second surface 4966 of the battery assembly 4962 is configured to contact a contact portion 4918 of a distal end portion 4916 of a battery clip 4910. When both the electrical contact of the substrate 4924 and the contact portion 4918 of the distal end portion 4916 of the battery clip 4910 contact the battery assembly 4962, the batteries of the battery assembly 4962 are placed in electrical communication with the electronic circuit system 4900. Said another way, when the electrical contact of the substrate 4924 and the contact portion 4918 of the distal end portion 4916 of the battery clip 4910 contact the battery assembly 4962, the battery assembly 4962 is configured to supply power to the electronic circuit system 4900.

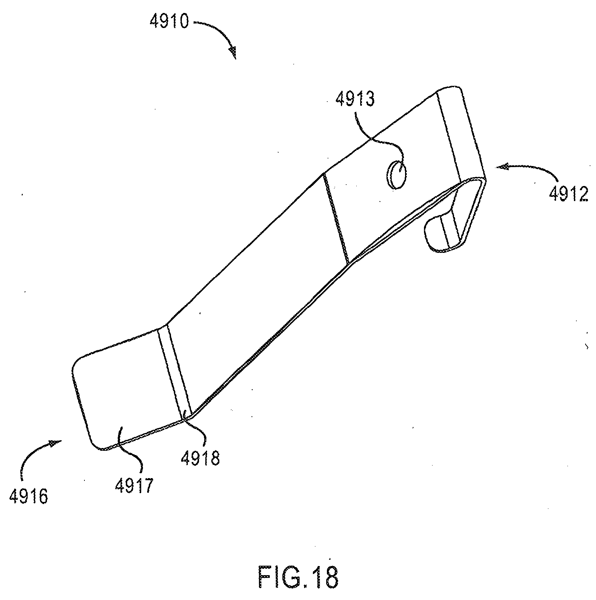

The battery clip 4910 (shown in FIG. 18) includes a proximal end portion 4912 and a distal end portion 4916. The proximal end portion 4912 defines a retention aperture 4913. The retention aperture 4913 is configured to receive the battery clip protrusion 4173 of the electronic circuit system housing 4170. In this manner, the battery clip protrusion 4173 maintains the position of the battery clip 4910 with respect to the electronic circuit system housing 4170 and/or the battery assembly 4962.

The distal end portion 4916 of the battery clip 4910 includes a contact portion 4918 and an angled portion 4917. As described above, the contact portion 4918 is configured to contact the second surface 4916 of the battery assembly 4962 to place the battery assembly 4962.in electrical communication with the electronic circuit system 4900. The angled portion 4917 of the distal end portion 4916 of the battery clip 4910 is configured to allow a proximal end portion 4236 of a battery isolation protrusion 4235 (see e.g., FIG. 25) to be disposed between the second surface 4966 of the battery assembly 4962 and the contact portion 4918 of the distal end portion 4916 of the battery clip 4910. When the battery isolation protrusion 4235 is disposed between the second surface 4966 of the battery assembly 4962 and the contact portion 4918 of the distal end portion 4916 of the battery clip 4910, the electrical path between the battery assembly 4962 and the remainder of the electrical circuit system 4900 is severed, thereby removing power from the electronic circuit system 4900. The contact portion 4918 of the distal end portion 4916 of the battery clip 4910 is biased such that when the battery isolation protrusion 4235 is removed, the contact portion 4918 will move into contact the second surface 4916 of the battery assembly 4962, thereby restoring electrical communication between the battery assembly 4962 and the electronic circuit system 4900. In some embodiments, the battery isolation protrusion 4235 can be repeatedly removed from between the second surface 4966 of the battery assembly 4962 and the contact portion 4918 of the distal end portion 4916 of the battery clip 4910 and reinserted. Said another way, the battery isolation protrusion 4235 and the battery clip 4910 collectively form a reversible on/off switch.

When the battery isolation protrusion 4235 is disposed between the second surface 4966 of the battery assembly 4962 and the contact portion 4918 of the distal end portion 4916 of the battery clip 4910, a portion of the battery isolation protrusion 4235 is also disposed within the battery isolation protrusion aperture 4121 (see e.g., FIG. 9). Conversely, when the battery isolation protrusion 4235 is removed from between the second surface 4966 of the battery assembly 4962 and the contact portion 4918 of the distal end portion 4916 of the battery clip 4910 the battery isolation protrusion aperture 4121 is opened. In this manner, the battery isolation protrusion 4235 can selectively open and/or close the battery isolation protrusion aperture 4121. In this regard, the battery isolation protrusion aperture 4121 can selectively function as a port to allow sound waves produced by the audible output device 4956 to exit the electronic circuit system cavity 4153 to an area outside the housing 4110. As described herein, in some embodiments, the location of the battery isolation protrusion aperture 4121 with respect to the location of the audible output device 4956 and/or the sound apertures 4191 is such that the sound waves that exit through the multiple sound apertures 4191 are substantially in phase with the sound waves that exit through the battery isolation protrusion aperture 4121.

The audible output device 4956 of the electronic circuit system 4900 is configured to output audible sound to a user in response to a use of the medical injector 4000. The audible output device 4956 can have any suitable performance characteristics to produce the desired audible output (e.g., an audible output having a predefined frequency range, a predefined sound pressure level, etc.) based on the electronic signals produced by the processor 4950, as described above. For example, the audible output device 4956 can have a specified resonance frequency, a specified output sound pressure level (e.g., Watts/meter), a specified maximum input power rating, and/or a specified frequency response range. The audible output device 4956 can have one or more resonant frequencies within the specified frequency response range. In some embodiments, the audible output device 4956 can be a commercially-available micro-speaker such as an M0015N07K01F micro-speaker manufactured by Dain (International) Co., Ltd. In other embodiments, the audible output device 4956 can be an RS-1511A micro-speaker manufactured by Regal Electronics, Inc., for example.

As shown in FIGS. 13 and 14, the audible output device 4956 includes a front portion 4957 and a back portion 4955. The audible output device 4956 can include a movable portion (e.g., a cone, membrane, diaphragm, or the like; not shown in FIGS. 13 and 14) such that the audible output device 4956 can produce a first set of sound waves from the front portion 4957 and a second set of sound waves from the back portion 4955. The sound waves produced by the front portion 4957 and the sound waves produced by the back portion 4955 collectively define the audible output of the audible output device 4956. More particularly, the audible output of the audible output device 4956 is characterized by the sound waves produced by the front portion 4957 and the sound waves produced by the back portion 4955 as they exit the electronic circuit system cavity 4153 through the sound apertures 4191 and the battery isolation protrusion aperture 4121, respectively.

As described above, the audible output device 4956 and the electronic circuit system cavity 4153 are collectively configured to enhance the quality and/or magnitude of the sound produced by the audible output device 4956. Similarly stated, the size, shape and/or number and location of openings (e.g., the sound apertures 4191 and the battery isolation protrusion aperture 4121) of the electronic circuit system cavity 4153 are configured to enhance the quality of the audible output produced by the audible output device 4956 having predefined performance characteristics. In some embodiments, for example, the electronic circuit system cavity 4153 and the electronic circuit system cover 4170 are configured such that the sound waves produced by the front portion 4957 as they exit through the sound apertures 4191 are substantially in phase with the sound waves produced by the back portion 4955 as they exit through the battery isolation protrusion aperture 4121. This can be accomplished, for example, by spacing the sound apertures 4191 apart from the battery isolation protrusion aperture 4121 by a predetermined distance. In this manner, the distance through which the sound waves produced by the front portion 4957 travel to exit through the sound apertures 4191 and the distance through which the sound waves produced by the back portion 4955 travel to exit through the battery isolation protrusion aperture 4121 can be set to predetermined values such that the sound waves produced by the front portion 4957 are substantially in phase with the sound waves produced by the back portion 4955 as they exit the sound apertures 4191 and the battery isolation protrusion aperture 4121, respectively. For example, in some embodiments, the sound apertures 4191 can be spaced apart from the battery isolation protrusion aperture 4121 by approximately 6.25cm to 7.5cm (2.5 inches to 3 inches). In other embodiments, the sound apertures 4191 can be spaced apart from the battery isolation protrusion aperture 4121 by approximately 2.5cm to 7.5cm (1 inch to 3 inches).

In some embodiments, the audible output device 4956 and the electronic circuit system cavity 4153 can be collectively "tuned" to enhance the quality of an audible output having a specific frequency range. For example, in some embodiments, the audible output device 4956 and the electronic circuit system cavity 4153 can be collectively "tuned" to enhance the quality of a recorded speech output. As described above, in some embodiments, the size and/or shape of the electronic circuit system cavity 4153 can be configured such that the electronic circuit system cavity 4153 defines an acoustic resonant frequency that is within a frequency range of the audible output device 4956 and/or a frequency range of a recorded speech output. In some embodiments, for example, the electronic circuit system cavity 4153 can define at least one acoustic resonant frequency of between about 100 hertz and about 1000 hertz. In other embodiments, the electronic circuit system cavity 4153 can define at least one acoustic resonant frequency of between about 100 hertz and about 3000 hertz.

By enhancing the audible output produced by the audible output device 4956 as described above, the medicament delivery device 4000 can produce audible outputs associated with recorded speech having sufficient volume (e.g., sound pressure level) and without external amplification. In this manner, the power required to produce such audible outputs can be minimized. For example, in some embodiments, the processor 4950 can be configured to output an electronic output to the audible output device 4956 such that the audible output device 4956 outputs an audible output having a sound pressure level in a range between about 61 decibels (dB) and about 65 dB. In another embodiment, the audible output can have a sound pressure level in a range between about 61 decibels (dB) and about 65 dB at a distance of about 15cm (6 inches) from the audible output device 4956. In yet other embodiments, the audible output can have a sound pressure level in a range between about 61 decibels (dB) and about 65 dB within a distance of about 6m (20 feet) from the audible output device 4956.

In other embodiments, the processor 4950 can be configured to output an electronic output to the audible output device 4956 such that the audible output device 4956 outputs an audible output that has a sound pressure level greater than about 61 dB. In yet other embodiments, the audible output can have a sound pressure level greater than about 61 dB at a distance of about 15cm (6 inches) from the audible output device 4956. In yet other embodiments, the audible output can have a sound pressure level greater than about 61 dB within a distance of about 6m (20 feet) from the audible output device 4956.

In some embodiments, the processor 4950 can output an electronic output having a power of less than 100 milliwatts (mW) to the audible output device 4956, and the audible output device 4956 can output an audible output that has a sound pressure level greater than about 61 dB. In yet another embodiment, the electronic output can have a power of less than 100 mW and the audible output can have a sound pressure level greater than about 61 dB at a distance of about 15cm (6 inches) from the audible output device 4956. In yet another embodiment, the electronic output can have a power of less than 100 mW and the audible output can have a sound pressure level greater than about 61 dB within a distance of about 6m (20 feet) from the audible output device 4956.

In other embodiments, the medical injector 4000 can have a network interface device (not shown) configured to operatively connect the electronic circuit system 4900 to a remote device (not shown) and/or a communications network (not shown). In this manner, the electronic circuit system 4900 can send information to and/or receive information from the remote device. The remote device can be, for example, a remote communications network, a computer, a compliance monitoring device, a cell phone, a personal digital assistant (PDA) or the like. Such an arrangement can be used, for example, to download replacement processor-readable code from a central network to the electronic circuit system 4900. In some embodiments, for example, the electronic circuit system 4900 can download information associated with a medical injector 4000, such as an expiration date, a recall notice, updated use instructions or the like. Similarly, in some embodiments, the electronic circuit system 4900 can upload compliance information associated with the use of the medical injector 4000 via the network interface device.

FIGS. 24 and 25 show the cover 4200 of the medical injector 4000. The cover 4200 includes a proximal end portion 4210 and a distal end portion 4230, and defines a cavity 4242. The cavity 4242 of the cover 4200 is configured to receive at least a portion of the housing 4110. The proximal end portion 4210 defines apertures 4215 configured to receive the cover retention protrusions 4142 of the housing 4110 (shown in FIGS. 4 and 6). In this manner, the apertures 4215 and the cover retention protrusions 4142 of the housing 4110 removably retain the cover 4200 about at least a portion of the housing 4110. Said another way, the apertures 4215 and the cover retention protrusions 4142 of the housing 4110 are configured such that the cover 4200 can be removed from a portion of the housing 4110 and then replaced about the portion of the housing 4110.

The distal end portion 4230 of the cover 4200 includes a battery isolation protrusion 4235. The battery isolation protrusion 4235 includes a proximal end portion 4236 and a tapered portion 4237. The proximal end portion 4236 of the battery isolation protrusion 4235 is configured to be removably disposed between the second surface 4966 of the battery assembly 4962 and the contact portion 4918 of the distal end portion 4916 of the battery clip 4910, as described above.

FIGS. 26-29 show the safety lock 4700 of the medical injector 4000. The safety lock 4700 of the medical injector 4000 includes a proximal surface 4740, a distal surface 4760 opposite the proximal surface 4740 and a needle sheath 4720. The safety lock 4700 defines a needle sheath aperture 4770 and a battery isolation protrusion aperture 4775. The battery isolation protrusion aperture 4775 is configured to receive the battery isolation protrusion 4235 of the cover 4200 such that the battery isolation protrusion 4235 can be disposed within the electronic circuit system cavity 4153 or the electronic circuit system 4900, as described above. Similarly stated, the battery isolation protrusion aperture 4775 of the safety lock 4700 is aligned with the battery isolation protrusion aperture 4121 of the housing 4110, such that the battery isolation protrusion 4235 can be disposed within the electronic circuit system cavity 4153 when the cover 4200 is disposed about a portion of the housing 4110. The battery isolation protrusion aperture 4775 is aligned with the battery isolation protrusion aperture 4121 such that sound waves produced by the audible output device 4956 within the electronic circuit system cavity 4153 that exit through the battery isolation protrusion aperture 4121 can travel through the battery isolation protrusion aperture 4775.

The proximal surface 4740 of the safety lock 4700 includes a safety lock protrusion 4742, a stopper 4743, an actuator 4744 and two opposing pull tabs 4741. As described above, when the safety lock 4700 is in a first (locked) position, the safety lock protrusion 4742 is configured to be disposed in the opening 4554 defined by the extensions 4552 of the distal end portion 4544 of the release member 4540. Accordingly, the safety lock protrusion 4742 is configured to prevent the extensions 4552 from moving closer to each other, thereby preventing proximal movement of the release member 4540 of the medicament delivery mechanism 4500 and/or delivery of a medicament. The stopper 4743 of the safety lock 4700 is a protrusion extending from the proximal surface 4740 of the safety lock 4700. The stopper 4743 is configured to contact a portion of the housing 4110 to limit the proximal movement of the safety lock 4700 relative to the housing 4110. In other embodiments, the stopper 4743 can be any structure configured to limit the proximal movement of the safety lock 4700.

The actuator 4744 of the safety lock 4700 has an elongated portion 4745 and a protrusion 4746. The elongated portion 4745 extends in a proximal direction from the proximal surface 4740. In this manner, the elongated portion 4745 can extend through a safety lock actuator opening 4356 of the base 4300 (see e.g., FIG. 30) and within the safety lock actuator groove 4123 of the housing 4110 and the safety lock actuator groove 4182 of the electronic circuit system housing 4170. The protrusion 4746 extends in a direction substantially transverse to the elongated portion 4745 and/or substantially parallel to the proximal surface 4740 of the safety lock 4700. As described above, the opening 4928 of the first actuation portion 4926 is configured to receive the protrusion 4746 of the actuator 4744 of the safety lock 4700.

The pull tabs 4741 of the safety lock 4700 include a grip portion 4747 and indicia 4748. The grip portion 4747 of the pull tabs 4741 provides an area for the user to grip and/or remove the safety lock 4700 from the rest of the medicament delivery system 4700. The indicia 4748 provides instruction on how to remove the safety lock 4700. In some embodiments, for example, the indicia 4748 can indicate the direction the user should pull the safety lock 4700 to remove the safety lock 4700.

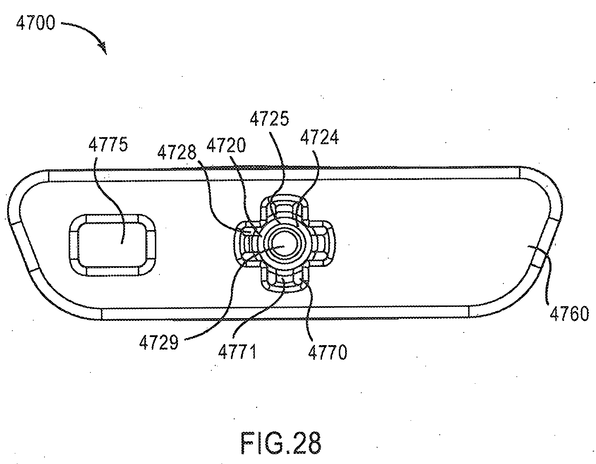

As shown in FIG. 28, the needle sheath 4720 of the safety lock 4700 includes a distal end portion 4724, a proximal end portion 4722 and a plurality of ribs 4728. The needle sheath 4720 can also define a lumen 4729. The lumen 4729 of the safety lock 4700 is configured to receive the needle 4512. In this manner, the needle sheath 4720 can protect the user from the needle 4512 and/or can keep the needle 4512 sterile before the user uses the medical injector 4000. The proximal end portion 4722 of the needle sheath is configured to contact the distal end portion 4522 of the carrier 4520 of the medicament delivery mechanism 4500.

The distal end portion 4724 of the needle sheath 4720 has an angled ridge 4725. The angled ridge 4725 is configured to allow the proximal end portion 4722 of the needle sheath 4720 to irreversibly move through the needle sheath aperture 4770 of the safety lock 4700 in a distal direction. Said another way, the angled ridge 4725 can be configured in such a way as to allow the proximal end portion 4722 of the needle sheath 4720 to move through the needle sheath aperture 4770 in a distal direction, but not in a proximal direction. The needle sheath aperture 4770 has retaining tabs 4771 configured to engage the proximal end of the angled ridge 4725 when the needle sheath 4720 is moved in a proximal direction. In this manner, the retaining tabs 4771 prevent the proximal movement of the needle sheath with respect to the safety lock 4700. Further, the retaining tabs 4771 are configured to engage the proximal end of the angled ridge 4725 when the safety lock 4700 is moved in a distal direction. Said another way, as shown in FIG. 33, the needle sheath 4720 is removed from the needle 4512 when the safety lock 4700 is moved in a distal direction with respect to the housing 4110.

FIGS. 30-31 show the base 4300 of the medical injector 4000. The base 4300 includes a proximal surface 4310, a distal surface 4330 and base connection knobs 4358. The base 4300 defines a needle aperture 4350, a safety lock protrusion aperture 4352, a battery isolation protrusion aperture 4354, a safety lock actuator opening 4356, and pull tab openings 4360. The needle aperture 4350 is configured to receive the needle 4512 when the medical injector 4000 is actuated. The safety lock protrusion aperture 4352 of the base 4300 receives the safety lock protrusion 4742 of the safety lock 4700. The battery isolation protrusion aperture 4354 of the base 4300 receives the battery isolation protrusion 4235 of the cover 4200 and the stopper 4743 of the safety lock 4700. The battery isolation protrusion aperture 4354 is aligned with the battery isolation protrusion aperture 4121 such that sound waves produced by the audible output device 4956 within the electronic circuit system cavity 4153 that exit through the battery isolation protrusion aperture 4121 can travel through the battery isolation protrusion aperture 4354. The safety lock actuator opening 4356 receives the safety lock actuator 4744 of the safety lock 4700. The pull tab openings 4360 are configured to receive the pull tabs 4741 of the safety lock 4700.

The proximal surface 4310 of the base 4300 includes an actuator 4311, guide members 4312, and protrusions 4313. The actuator 4311 is an elongate member configured to engage the substrate 4924 of the electronic circuit system 4900. As described above, the opening 4945 of the second actuation portion 4946 is configured to receive the actuator 4311 of the base 4300. The guide members 4312 of the base 4300 are configured to engage and/or slide within the base rail grooves 4127 of the housing 4110, as described above. The protrusions 4313 of the base 4300 are configured to engage the tapered surfaces 4549 of the extensions 4552 of the release member 4540. As described in further detail herein, when the safety lock 4700 is removed and the base 4300 is moved in a proximal direction with respect to the housing 4110, the protrusion 4313 of the base 4300 are configured to move the extensions 4552 of the release member 4540 closer to each other, actuating the medicament delivery mechanism 4500. As described above, the base connection knobs 4358 are configured to engage the base retention recesses 4125A, 4125B in a way that allows proximal movement of the base 4300 but limits distal movement of the base 4300.

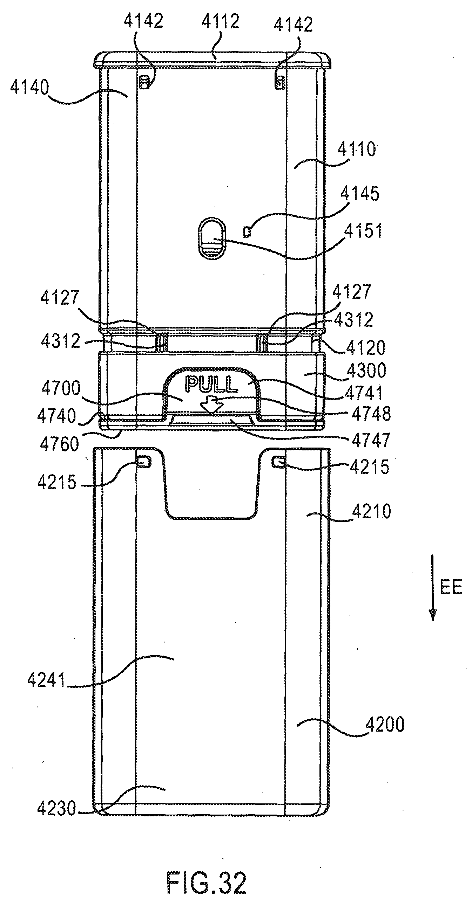

As shown in FIG. 32, the medical injector 4000 is first enabled by moving the medicament delivery device from a first configuration to a second configuration by moving the cover 4200 from a first position to a second position. The cover 4200 is moved from the first position to the second position by moving it with respect to the housing 4110 in the direction shown by the arrow EE in FIG. 32. When the cover 4200 is moved with respect to the housing 4110 in the direction EE, the battery isolation protrusion 4235 is removed from the area between the battery clip 4910 and the second surface 4966 of the battery assembly 4962. In this manner, the battery assembly 4962 can be operatively coupled to the electronic circuit system 4900 when the cover 4200 is removed, thereby providing power to the electronic circuit system 4900.

When power is provided, as described above, the electronic circuit system 4900 can output one or more predetermined electronic outputs. For example, in some embodiments, the electronic circuit system 4900 can output an electronic signal associated with recorded speech to the audible output device 4956. Such an electronic signal can be, for example, associated with a .WAV file that contains a recorded instruction instructing the user in the operation of the medical injector 4000. Such an instruction can state, for example, "remove the safety tab near the base of the auto-injector." The electronic circuit system 4900 can simultaneously output an electronic signal to one and/or both of the LEDs 4958A, 4958B thereby causing one and/or both of the LEDs 4958A, 4958B to flash a particular color. In this manner, the electronic circuit system 4900 can provide both audible and visual instructions to assist the user in the initial operation of the medical injector 4000. Further, because the battery isolation protrusion 4235 has been removed from the battery isolation protrusion aperture 4121, an audible output by the audible output device 4956 can include sound waves produced within the electronic circuit system cavity 4153 that exit through the battery isolation protrusion aperture 4121.

In other embodiments, the electronic circuit system 4900 can output an electronic output associated with a description and/or status of the medical injector 4000 and/or the medicament contained therein. For example, in some embodiments, the electronic circuit system 4900 can output an audible message indicating the type of medicament contained in the medical injector 4000, the expiration date of the medicament, the dosage of the medicament or the like.

As described above, the medical injector 4000 can be can be repeatedly moved between the first configuration and the second configuration when the cover 4200 is moved repeatedly between the first position and the second position respectively. Said another way, the cover 4200 can be removed and replaced about the housing 4110 any number of times. When the cover 4200 is moved from the second position to the first position, the battery isolation protrusion 4235 is inserted between the battery clip 4910 and the second surface 4966 of the battery assembly 4962, deactivating the electronic circuit system 4900 and closing the acoustic port associated with the battery isolation protrusion aperture 4121. When the cover is moved from the first position to the second position a second time, the electronic circuit system 4900 is once again activated and the acoustic port is opened. In this manner, the cover 4200 can be removed and the electronic circuit system 4900 can output an electronic output without compromising the sterility of the needle 4512.

After the cover 4200 is removed from the housing 4110, the medical injector 4000 can be moved from the second configuration to a third configuration by moving the safety lock 4700 from a first position to a second position. The safety lock 4700 is moved from a first position to a second position by moving the safety lock 4700 with respect to the housing 4110 in the direction shown by the arrow FF in FIG. 33. When the safety lock 4700 is moved from the first position to the second position, the safety lock protrusion 4742 is removed from between the extensions 4552 of the release member 4540, thereby enabling the medicament delivery member 4500. Moreover, as shown in FIGS. 21 and 22, when the safety lock 4700 is moved from the housing 4110, the actuator 4744 of the safety lock 4700 moves in the direction CC as shown in FIG. 22, irreversibly moving the first switch 4972 from a first state (e.g., a state of electrical continuity) to a second state (e.g., a state of electrical discontinuity). When the actuator 4744 of the safety lock 4700 moves irreversibly the first switch 4972 of the electronic circuit system 4900 to the second state, the electronic circuit system 4900 can output one or more predetermined electronic outputs. For example, in some embodiments, a processor (not shown) can output an electronic signal associated with recorded speech to the audible output device 4956. Such an electronic signal can be, for example, associated with a recorded message notifying the user of the status of the medical injector 4000. Such a status message can state, for example, "The medical injector is now enabled." The electronic circuit system 4900 can also simultaneously output an electronic signal to one and/or both of the LEDs 4958A, 4958B, thereby causing one and/or both of the LEDs 4958A, 4958B to stop flashing, change color or the like.

In some embodiments, the first actuation portion 4926 and the actuator 4744 can be configured such that the actuator 4744 must move a predetermined distance before the actuator 4744 engages the boundary 4929 of the opening 4928. For example, in some embodiments, the actuator 4744 must move approximately 0.5cm (0.200 inches) before the actuator 4744 engages the boundary 4929 of the opening 4928. In this manner, the safety lock 4700 can be moved slightly without irreversibly moving the first switch 4972 of the electronic circuit system 4900 to the second state. Accordingly, this arrangement will permit the user to inadvertently and/or accidentally move the safety lock 4700 without actuating the electronic circuit system 4900.

In some embodiments, the electronic circuit system 4900 can be configured to output the status message for a predetermined time period, such as, for example, five seconds. After the predetermined time period has elapsed, the electronic circuit system 4900 can output an audible message further instructing the user in the operation of the medical injector 4000. Such an instruction can state, for example, "Place the base of the auto-injector against the patient's thigh. To complete the injection, press the base firmly against the patient's thigh." In some embodiments, the electronic circuit system 4900 can simultaneously output an electronic signal to one and/or both of the LEDs 4958A, 4958B, thereby causing one and/or both of the LEDs 4958A, 4958B to flash a particular color. In this manner, the electronic circuit system 4900 can provide both audible and/or visual instructions to assist the user in the placement and actuation of the medical injector 4000. In some embodiments, the electronic circuit system 4900 can be configured to repeat the instructions after a predetermined time period has elapsed.

As described above, in other embodiments, the medical injector 4000 can have a network interface device (not shown) configured to operatively connect the electronic circuit system 4900 to a remote device (not shown) and/or a communications network (not shown). In this manner, the electronic circuit system 4900 can send a wireless signal notifying a remote device that the safety lock 4700 of the medical injector 4000 has been removed and that the medical injector 4000 has been armed.

After the safety lock 4700 is moved from the first position to the second position, the medical injector 4000 can be moved from the third configuration to a fourth configuration by moving the base 4300 from a first position to a second position. The base 4300 is moved from its first position to its second position by placing the medical injector 4000 against the body of the patient and moving the base 4300 with respect to the housing 4110 in the direction shown by the arrow GG in FIG. 34. Moving the base 4300 from the first position to the second position causes the protrusions 4313 on the proximal surface 4310 of the base 4300 to engage the tapered surfaces 4549 of the extensions 4552 of the release member 4540, causing the release member 4540 to actuate the medicament delivery mechanism 4500 and deliver a medicament to a body of a patient.

When the base 4300 is moved from the first position to the second position, the medicament delivery mechanism 4500 is actuated such that the puncturer 4541 of the release member 4540 is brought in contact with and/or punctures the frangible seal 4573 of the gas container 4570. In some embodiments, the movement of the release member 4540 can be caused by a spring (not shown in FIG. 12). After the frangible seal 4573 has been punctured, an actuating portion of a compressed gas can escape from the gas container 4570 and flow via the gas passageway 4144 into the medicament cavity 4157. The gas applies gas pressure to the movable member 4530 causing the movable member 4530 and the carrier 4520 to move in a distal direction within the medicament cavity 4157. When the carrier 4520 moves distally within the medicament cavity 4157, the carrier 4520 and the medicament container 4560 are in a first configuration. Accordingly, as described above, the medicament container 4560 is connected to the carrier 4520 by a "snap fit" connection. In this manner, the medicament container 4560 and the needle 4512 contemporaneously move with movable member 4530 and/or the carrier 4520 in a distal direction. As described above, the proximal end portion 4516 of the needle 4512 is connected to the distal end portion 4522 of the carrier 4520 and is spaced from the seal 4523 of the medicament container 4560 when the carrier 4520 is in its first configuration. Said another way, the medicament container 4560 and the needle 4512 do not define a medicament delivery path when the carrier 4520 is in the first configuration. The movement of the needle 4512 in a distal direction causes the proximal end portion 4516 of the needle 4512 to exit the housing 4110 and enter the body of a patient prior to administering a medicament.

After the carrier 4520 and/or the needle 4512 have moved within the medicament cavity 4157 a predetermined distance, the carrier 4520 and the medicament container 4560 are moved from the first configuration to a second configuration. In the second configuration of the carrier 4520, the medicament container 4560 is released from the "snap-fit" allowing the medicament container 4560 and the movable member 4530 to continue to move in a distal direction relative to the carrier 4520. Said another way, the medicament container 4560 is configured to slidably move within the carrier 4520 when the carrier is moved from the first configuration to the second configuration. As the medicament container 4560 continues to move within the carrier 4520, the proximal end portion 4516 of the needle 4512 contacts and punctures the seal 4523 of the medicament container 4560. This allows the medicament contained in the medicament container 4560 to flow into the lumen (not shown) defined by the needle 4512, thereby defining a medicament delivery path.

As the medicament container 4560 contacts the distal end of the carrier 4520, the medicament container 4560 stops moving within the carrier 4520 while the movable member 4530 continues to move in a distal direction. This causes the piston portion 4534 of the movable member 4530 to sealingly slide and/or move within the medicament container 4560 containing a liquid medicament. As the piston portion 4534 of the movable member 4530 sealingly slides and/or moves within the medicament container 4560, the piston portion 4534 generates a pressure upon the medicament contained within the medicament container 4560, thereby allowing at least a portion of the medicament to flow out of the medicament container 4560 and into the lumen defined by the needle 4512. The medicament is delivered to a body of a user via the medicament delivery path defined by the medicament container 4560 and the needle 4512.

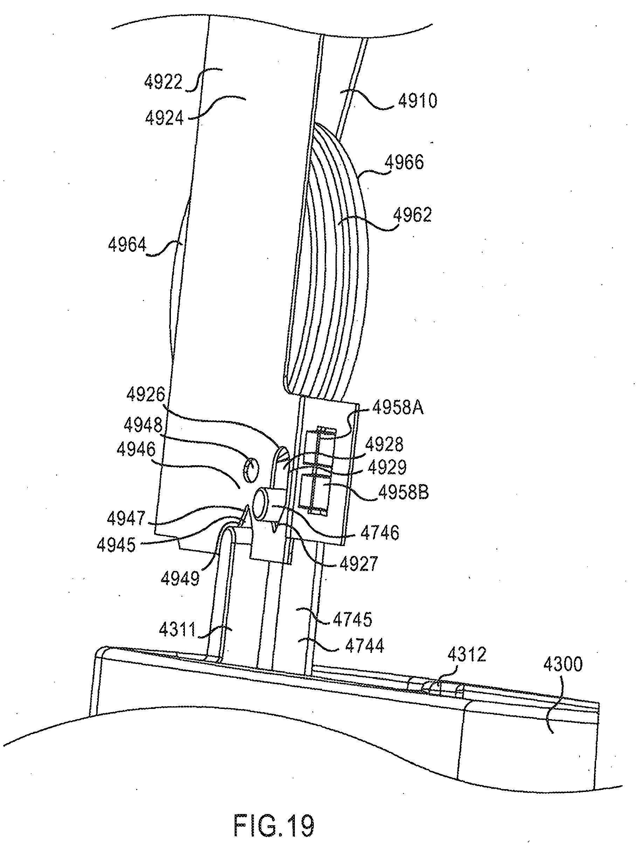

As described above, the actuator 4538 of the base 4300 actuates the electronic circuit 4900 to trigger a predetermined output or sequence of outputs when the base 4520 is moved from its first position to its second position (see, e.g., FIGS. 19-23). When the actuator 4538 is moved in a proximal direction relative to the opening 4945, as shown by the arrow DD in FIG. 23, the electronic circuit system 4900 is actuated to output one or more predetermined electronic outputs. For example, in some embodiments, the electronic circuit system 4900 can output an electronic signal associated with recorded speech to the audible output device 4956. Such an electronic signal can be, for example, associated with an audible countdown timer, instructing the user on the duration of the injection procedure. Said another way, if it takes, for example, ten seconds to complete an injection, an audible countdown timer can count from ten to zero ensuring that the user maintains the medical injector 4000 in place for the full ten seconds. In other embodiments, the electronic signal can be, for example, associated with a recorded message notifying the user that the injection is complete, instructing the user on post-injection disposal and safety procedures, instructing the user on post-injection medical treatment or the like. Such a status message can state, for example, "The injection is now complete. Please seek further medical attention from a doctor." The electronic circuit system 4900 can also simultaneously output an electronic signal to one and/or both LEDs 4958A, 4958B, thereby causing one and/or both LEDs 4958A, 4958B to stop flashing, change color or the like, to provide a visual indication that the injection is complete. In other embodiments, the electronic circuit system 4900 can send a wireless signal notifying a remote device that the injection is complete. In this manner, a patient's compliance can be monitored.

In some embodiments, the second actuation portion 4946 and the actuator 4538 can be configured such that the base 4500 and/or the actuator 4538 must move a predetermined distance before the actuator 4538 engages the boundary 4949 of the opening 4945. For example, in some embodiments, the actuator 4538 must move approximately 0.5 cm (0.200 inches) before the actuator 4538 engages the boundary 4949 of the opening 4945. In this manner, the base 4700 can be moved slightly without irreversibly moving the second switch 4973 of the electronic circuit system 4900 to the second state. Accordingly, this arrangement will permit the user to inadvertently and/or accidentally move the base 4500 without actuating the electronic circuit system 4900.

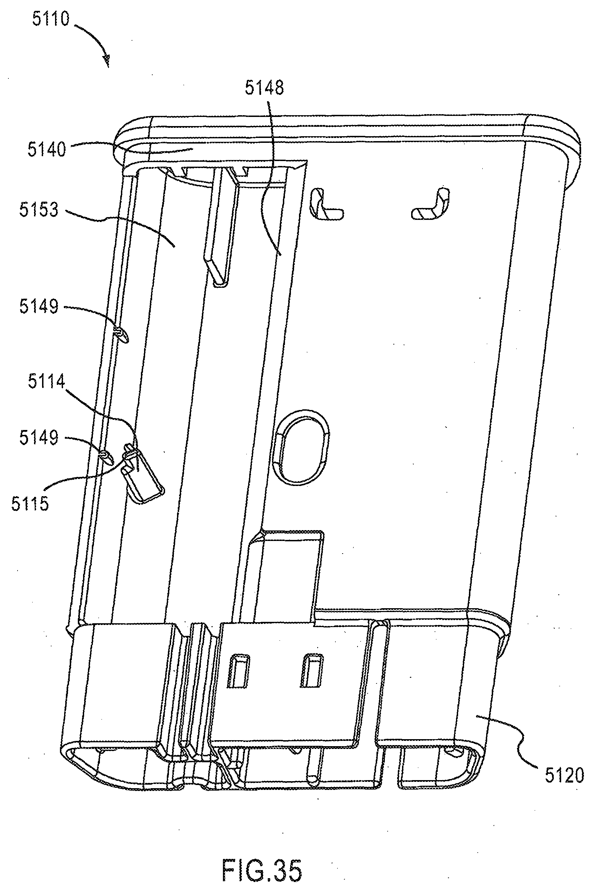

Although the electronic circuit system 4900 is shown and described above as having two irreversible switches (e.g., switch 4972 and switch 4973), in other embodiments, an electronic circuit system can have any number of switches. Moreover, such switches can be either reversible or irreversible. For example, FIGS. 35-40 show portions of a medicament delivery device 5000 having an electronic circuit system 5900 having three irreversible switches.

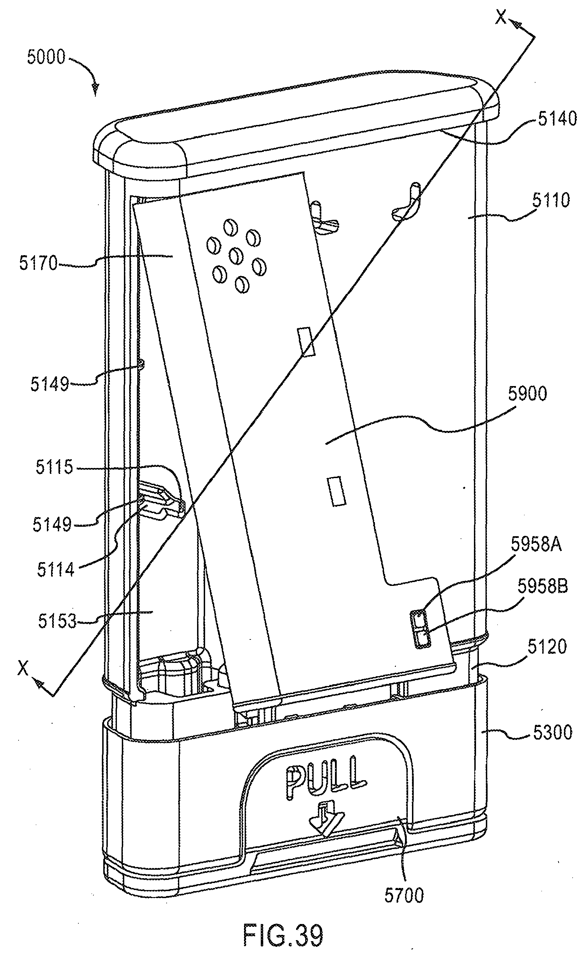

The medicament delivery device 5000 is similar to the medical injector 4000 described above. As shown in FIG. 39, the medicament delivery device 5000 includes a housing 5110, a delivery mechanism (not shown), an electronic circuit system 5900, a cover (not shown), a safety lock 5700 and a base 5300. The structure and operation of the delivery mechanism, the cover, the safety lock 5700 and the base 5300 are similar to the structure and operation of the delivery mechanism 4500, the cover 4200, the safety lock 4700 and the base 4300, respectively. Accordingly, only the electronic circuit system 5900 and the housing 5110 are described in detail below.

As shown in FIG. 35, the housing 5110 has a proximal end portion 5140 and a distal end portion 5120. The housing 5110 defines a gas cavity (not shown), a medicament cavity (not shown) and an electronic circuit system cavity 5153. The gas cavity and medicament cavity of the housing 5110 of the medicament delivery device 5000 are similar to the gas cavity 4154 and the medicament cavity 4157, shown and described above with reference to FIGS. 9 and 10.

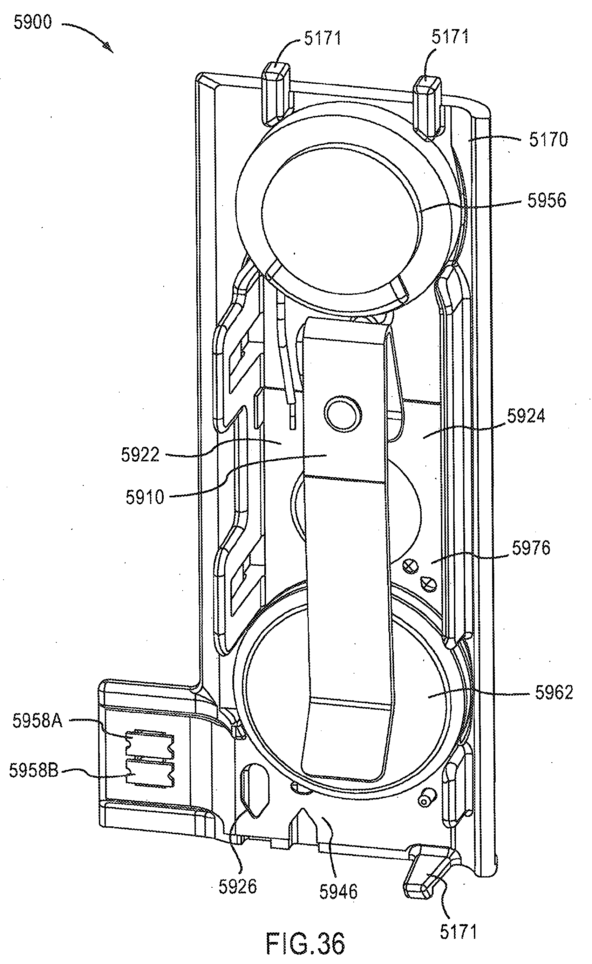

The electronic circuit system cavity 5153 is configured to receive the electronic circuit system 5900. As described above, the electronic circuit system cavity 5153 is fluidically, acoustically and/or physically isolated from the gas cavity and/or the medicament cavity by a sidewall 5148. The housing 5110 has protrusions 5149 configured to stabilize the electronic circuit system 5900 when the electronic circuit system 5900 is disposed within the electronic circuit system cavity 5153. The housing 5110 also defines connection apertures (not shown) configured to receive connection protrusions 5171 of the electronic circuit system 5900 (see e.g., FIG. 36). In this manner, the electronic circuit system 5900 can be coupled to the housing 5110 within the electronic circuit system cavity 5153 (see e.g., FIG. 39). In other embodiments, the electronic circuit system 5900 can be coupled within the electronic circuit system cavity 5153 by any other suitable means, such as an adhesive, a clip and/or the like.

The housing 5110 includes an actuation protrusion 5114 disposed within the electronic circuit system cavity 5153. As described in more detail herein, an angled end portion 5115 of the actuation protrusion 5114 of the housing 5110 is configured to engage a third actuation portion 5976 of a substrate 5924 of the electronic circuit system 5900 when the electronic circuit system 5900 is coupled to the housing 5110.

As shown in FIGS. 39, the electronic circuit system 5900 is configured to fit within the electronic circuit system cavity 5153 of the housing 5110. Accordingly, as described above, the electronic circuit system 5900 is physically, acoustically and/or fluidically isolated from the medicament cavity, the gas cavity and/or the medicament delivery path within the medicament delivery device 5000 (not shown). As described herein, the electronic circuit system 5900 is configured to output an electronic output associated with a use of the medicament delivery device 5000.

As shown in FIG. 36, the electronic circuit system 5900 is similar to the electronic circuit system 4900 described above. The electronic circuit system 5900 of the medicament delivery device 5000 includes an electronic circuit system housing 5170, a printed circuit board 5922, a battery assembly 5962, an audible output device 5956, two light emitting diodes (LEDs) 5958A, 5958B and a battery clip 5910. The electronic circuit system housing 5170, the battery assembly 5962, the audible output device 5956, the two light emitting diodes (LEDs) 5958A, 5958B and the battery clip 5910 are similar to the battery assembly 4962, the audible output device 4956, the two light emitting diodes (LEDs) 4958A, 4958B and the battery clip 4910 of the electronic circuit system 4900 described above. Thus, a detailed discussion of these components is omitted.

The electronic circuit system 5900 also includes a processor 5950 configured to process electronic inputs (e.g., from input switches) and produce electronic outputs. As described herein, such electronic outputs can include audio or visual outputs associated with a use of the medicament delivery device 5000. More particularly, the processor 5950 is configured to output an electronic signal to the audible output device 5956, which then converts the electronic signal into sound waves. Said another way, the processor 5950 is configured to output an electronic signal associated with an audible output to the audible output device 5956, which is configured to output the audible output. The electronic signal can be associated with, for example, recorded speech, a single tone, a sequence of tones, and/or the like. In this manner, the electronic circuit system 5900 can produce and/or output an audible output associated with a use of the medicament delivery device 5000.

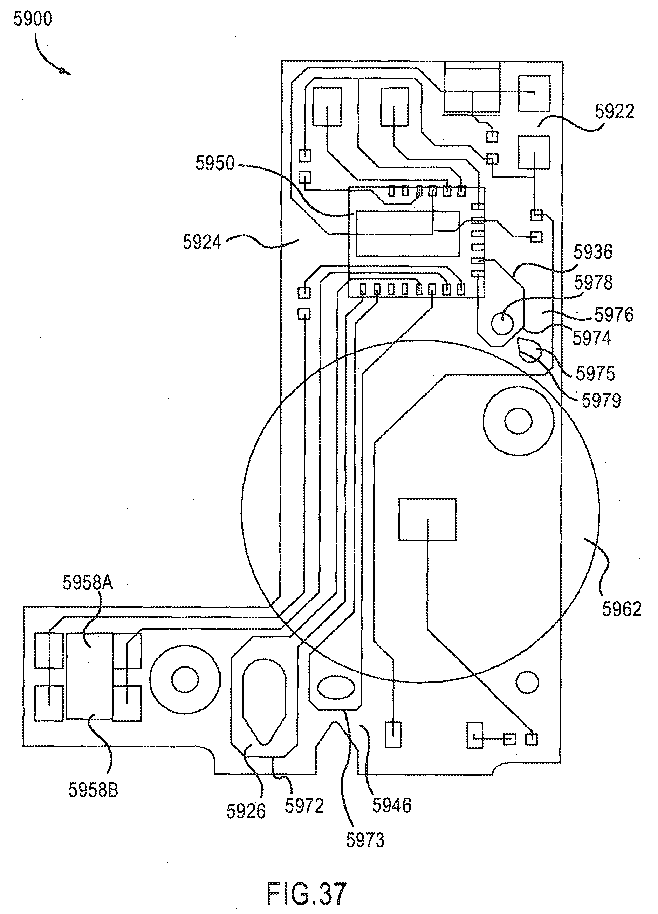

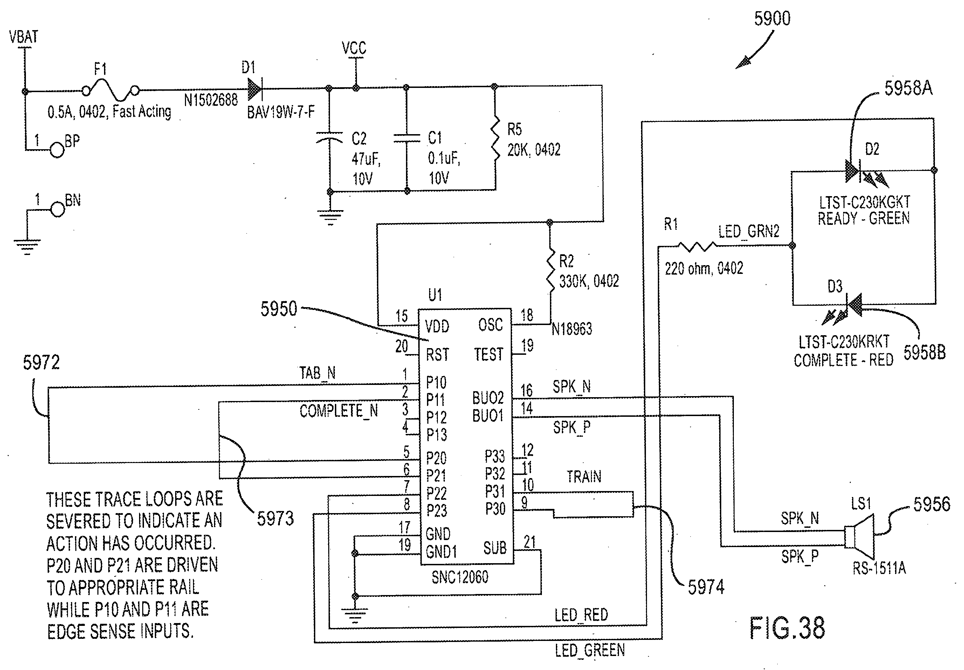

The electronic signals produced by the processor 5950 are conveyed to the audible output device 5956 via one or more electronic paths (not identified in the schematic shown in FIG. 37) defined by the printed circuit board 5922 (e.g., conductive traces or the like). As shown in FIG. 38, the electronic paths between the processor 5950 and the audible output device 5956 are devoid of an amplifier. Similarly stated, no amplifiers and/or drivers external to the processor 5956 are used to amplify or increase the electronic signals produced by the processor 5950. This arrangement can reduce the cost and/or complexity of the electronic circuit system 5900. Moreover, by being devoid of external amplification, the power of the electronic signals conveyed to the audible output device 5956 can be relatively low. In this manner, the life of the battery assembly 5962 can be extended, the battery assembly 5962 can include smaller batteries and/or the battery assembly 5962 can include fewer batteries. In some embodiments, for example, the electronic signal produced by the processor 5950 can have a power of less than 500 milliwatts (mW). In other embodiments, the electronic signal produced by the processor 5950 can have a power of less than 100 milliwatts (mW). In yet other embodiments, the electronic signal produced by the processor 5950 can have a power of approximately 80 milliwatts (mW).

The processor 5950 (and any of the processors described herein) can be a commercially-available processing device dedicated to performing one or more specific tasks. For example, in some embodiments, the processor 5950 can be a commercially-available microprocessor, such as the Sonix SNC 12060 voice synthesizer. Alternatively, the processor 5950 can be an application-specific integrated circuit (ASIC) or a combination of ASICs, which are designed to perform one or more specific functions. In yet other embodiments, the processor 5950 can be an analog or digital circuit, or a combination of multiple circuits. In some embodiments, the processor 5950 can be programmed through, for example, an internal controller (not shown) such that varied applications, including voice section combination, key trigger arrangement, and/or output control, for example, can be implemented.

The processor 5950 can include a memory device (not shown) configured to receive and store information, such as a series of instructions, processor-readable code, a digitized signal, or the like. The memory device can include one or more types of memory. For example, the memory device can include a read only memory (ROM) component and a random access memory (RAM) component. The memory device can also include other types of memory suitable for storing data in a form retrievable by the processor 5950, for example, electronically-programmable read only memory (EPROM), erasable electronically-programmable read only memory (EEPROM), or flash memory. In some embodiments, a memory device separate from the processor 5950 can be used to receive and store information.

FIG. 37 shows the printed circuit board 5922 of the electronic circuit system 5900. FIG. 38 is a schematic illustration of the electronic circuit system 5900. The printed circuit board 5922 of the electronic circuit system 5900 includes a substrate 5924, a first actuation portion 5926 (including a first switch 5972), a second actuation portion 5946 (including a second switch 5973), and a third actuation portion 5976 (including an electronic circuit system configuration switch 5974). The substrate 5924 of the printed circuit board 5922 includes the electrical components necessary for the electronic circuit system 5900 to operate as desired. For example, the electrical components can include resistors, capacitors, inductors, switches, microcontrollers, microprocessors and/or the like.

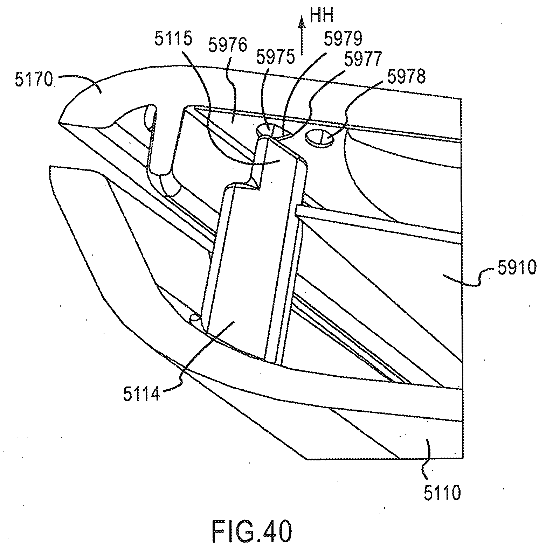

The first actuation portion 5926 and the second actuation portion 5946 are similar to the first actuation portion 4926 and the second actuation portion 4946 of the electronic circuit system 4900, described above (see e.g., FIG 36), and are therefore not described or labeled in detail. The third actuation portion 5976 includes a third electrical conductor 5936 (see e.g., FIG. 37) and defines an actuation aperture 5975 having a boundary 5979, and a tear propagation limit aperture 5978. As shown in FIGS. 36 and 40, the actuation aperture 5975 of the third actuation portion 5976 is configured to receive the angled end portion 5115 of the actuation protrusion 5114 of the housing 5110 when the electronic circuit system 5900 is disposed within the electronic circuit system cavity 5153. The boundary 5979 of the actuation aperture 5975 has a discontinuous shape, such as, for example, a teardrop shape, that includes a stress concentration riser 5977. The discontinuity and/or the stress concentration riser 5977 of the boundary 5979 can be of any suitable shape to cause the substrate 5924 to deform in a predetermined direction when the angled end portion 5115 of the actuation protrusion 5114 of the housing 5110 is inserted into the actuation aperture 5975 (see e.g., FIG. 40), as described below.

The third electrical conductor 5936 includes the electronic circuit system configuration switch 5974 (see e.g., FIG. 37) disposed between the actuation aperture 5975 and the tear propagation limit aperture 5978, which can be, for example, a frangible portion of the third electrical conductor 5436. As shown in FIGS. 39 and 40, when the electronic circuit system 5900 is attached to the housing 5110, a portion of the angled portion 5115 of the actuation protrusion 5114 is disposed within the actuation aperture 5975 of the third actuation portion 5976, as shown by the arrow HH in FIG, 40. Continued movement of the angled portion 5115 of the actuation protrusion 5114 within the third actuation portion 5976 of the substrate 5924 causes the third actuation portion 5976 of the substrate 5924 to tear, thereby separating the portion of the third electrical conductor 5936 including the electronic circuit system configuration switch 5974. Said another way, when the electronic circuit system 5900 is attached to the housing 5110, the actuation protrusion 5114 moves irreversibly the electronic circuit system configuration switch 5974 from a first state (e.g., a state of electrical continuity) to a second state (e.g., a state of electrical discontinuity).

The tear propagation limit aperture 5978 is configured to limit the propagation of the tear in the substrate 5924. Said another way, the tear propagation limit aperture 5978 is configured to ensure that the tear in the substrate 5924 does not extend beyond the tear propagation limit aperture 5978. The tear propagation limit aperture 5978 can be any shape configured to limit the propagation of a tear and/or disruption of the substrate 5924. For example, the tear propagation limit aperture 5978 can be oval shaped. In other embodiments, the boundary of the tear propagation limit aperture 5978 can be reinforced to ensure that the tear in the substrate 5924 does not extend beyond the tear propagation limit aperture 5978. The angled end portion 5115 of the actuation protrusion 5114 ensures that the tear in the substrate 5924 propagates in the desired direction. Said another way, the angled end portion 5115 of the actuation protrusion 5114 ensures that the tear in the substrate 5924 occurs between the actuation aperture 5975 and the tear propagation limit aperture 5978.

When the actuation protrusion 5114 of the housing 5110 moves irreversibly the electronic circuit system configuration switch 5974 of the electronic circuit system 5900 from the first state to the second state, the electronic circuit system 5900 can be moved between a first configuration and a second configuration. For example, in some embodiments, irreversibly moving the electronic circuit system configuration switch 5974 of the electronic circuit system 5900 to the second state places the electronic circuit system 5900 in the second configuration such that when power is applied to the electronic circuit system 5900, the electronic circuit system 5900 recognizes that the medicament delivery device 5000 is a certain type of medicament delivery device and/or is in a certain configuration. In some embodiments, the housing can be devoid of the actuation protrusion 5114, thus the electronic circuit system configuration switch 5974 is maintained in its first state when the electronic circuit system 5900 is attached to the housing 5110. In this manner, the electronic circuit system configuration switch 5974 can enable the electronic circuit system 5900 to be used in different types and/or configurations of medicament delivery devices. The dual functionality of the electronic circuit system 5900 enables production of the same electronic circuit system 5900 for multiple devices, thereby permitting mass production and decreasing the cost of production of the electronic circuit system 5900.

For example, in some embodiments the electronic circuit system 5900 can be used in either an actual medicament delivery device or a simulated medicament delivery device. A simulated medicament delivery device can, for example, correspond to an actual medicament delivery device and can be used, for example, to train a user in the operation of the corresponding actual medicament delivery device.

The simulated medicament delivery device can simulate the actual medicament delivery device in any number of ways. For example, in some embodiments, the simulated medicament delivery device can have a shape corresponding to a shape of the actual medicament delivery device, a size corresponding to a size of the actual medicament delivery device and/or a weight corresponding to a weight of the actual medicament delivery device. Moreover, in some embodiments, the simulated medicament delivery device can include components that correspond to the components of the actual medicament delivery device. In this manner, the simulated medicament delivery device can simulate the look, feel and sounds of the actual medicament delivery device. For example, in some embodiments, the simulated medicament delivery device can include external components (e.g., a housing, a needle guard, a sterile cover, a safety lock or the like) that correspond to external components of the actual medicament delivery device. In some embodiments, the simulated medicament delivery device can include internal components (e.g., an actuation mechanism, a compressed gas source, a medicament container or the like) that correspond to internal components of the actual medicament delivery device.

In some embodiments, however, the simulated medicament delivery device can be devoid of a medicament and/or those components that cause the medicament to be delivered (e.g., a needle, a nozzle or the like). In this manner, the simulated medicament delivery device can be used to train a user in the use of the actual medicament delivery device without exposing the user to a needle and/or a medicament. Moreover, the simulated medicament delivery device can have features to identify it as a training device to prevent a user from mistakenly believing that the simulated medicament delivery device can be used to deliver a medicament. For example, in some embodiments, the simulated medicament delivery device can be of a different color than a corresponding actual medicament delivery device. Similarly, in some embodiments, the simulated medicament delivery device can include a label clearly identifying it as a training device.

The actuation of the medicament delivery device configuration switch 5974 can configure the electronic circuit system 5900 to output a different electronic output when the medicament delivery device 5000 is a simulated medical injector than when the medicament delivery device 5000 is an actual medical injector. Said yet another way, the electronic circuit system 5900 can be configured to output a first series of electronic outputs when the electronic circuit system configuration switch 5974 is in the first state and a second series of electronic outputs when the electronic circuit system configuration switch 5974 is in the second state. In this manner, the electronic circuit system configuration switch 5974 can enable the same electronic circuit system 5900 to be used in both simulated medicament delivery devices and actual medicament delivery devices. When used on an actual medicament delivery device, for example, the housing can be devoid of the actuation protrusion 5114. The dual functionality of the electronic circuit system 5900 can decrease the cost of production of the electronic circuit system 5900 of the medicament delivery device 5000.

In other embodiments, moving the electronic circuit system configuration switch 5974 to the second state can place the electronic circuit system 5900 in any number of different functional configurations. For example, moving the electronic circuit system configuration switch 5974 from the first state to the second state can indicate the type of medicament in the medicament container, the dosage of the medicament and/or the language of the audible electronic outputs output by the electronic circuit system 5900.

In still other embodiments, any number of electronic circuit system configuration switches can be used. For example, multiple switches can be used to configure the electronic circuit system 5900 to output usage instructions in any number of languages. For example, if an electronic circuit system contained three configuration switches (e.g., switches A, B and C), switch A can correspond to English instructions, switch B to Spanish instructions and switch C to German instructions. Further, moving both switch A and B to the second state might correspond to French instructions. In this manner, a single electronic circuit system 5900 can be configured to output instructions in multiple languages.

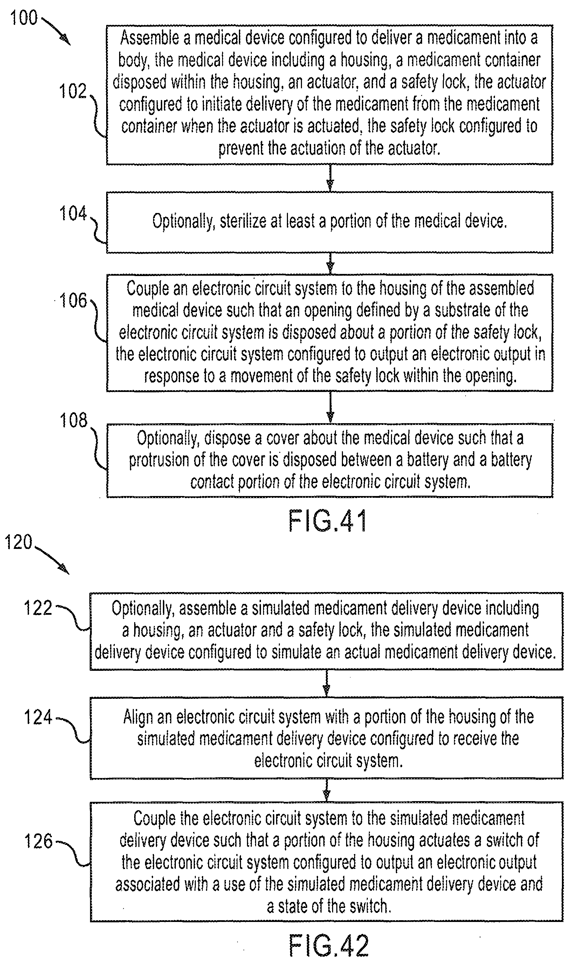

FIG. 41 is a flow chart of a method 100 according to an embodiment of the invention. The method includes assembling a medical device configured to deliver a medicament into a body of a patient, 102. The medical device includes a housing, a medicament container disposed within the housing, an actuator, and a safety lock. In some embodiments, the housing, medical container, the actuator, and the safety lock can be similar to the corresponding components in the medical injector 4000 and/or the medicament delivery device 5000, described above. The actuator of the medical device is configured to initiate delivery of the medicament from the medicament container when the actuator is actuated. The safety lock of the medical device is configured to prevent actuation of the actuator.

After the medical device is assembled, at least a portion of the medical device can optionally be sterilized, 104. Various sterilization techniques may be utilized. In some embodiments, a suitable sterilization technique includes the use of one or more of ethylene oxide, gamma radiation, e-beam radiation, ultraviolet radiation, steam, plasma, or hydrogen peroxide. In some embodiments, the needle is sterilized prior to installing the needle cover. In some embodiments, the needle is sterilized after the needle cover is installed. For example, in some embodiments, the needle cover is installed and then a gas sterilant is conveyed through at least a portion of the needle cover. The needle is sterilized using a gas sterilization technique that can penetrate one or more pores of a porous needle cover. In some embodiments, the needle can be sterilized using a gas sterilization technique that can penetrate one or more pores of a porous needle cover, but that will not react with a medicament in a medicament container disposed in the housing.

An electronic circuit system is then coupled to the housing of the assembled medical device, 106. The electronic circuit system is coupled to the housing such that an opening defined by a substrate of the electronic circuit system is disposed about a portion of the safety lock. The electronic circuit system is configured to output an electronic output in response to a movement of the safety lock within the opening. In some embodiments, for example, the electronic circuit system can be similar to the electronic circuit system 4900 of the medical injector 4000 and/or the electronic circuit system 5900 of the medicament delivery device 5000, as described above. In some embodiments, the electronic output can be, for example, a visual output, an audible output, and/or a haptic output, such as those described above. In other embodiments, the electronic output can be a wireless signal configured to be received by a remote device.

After the electronic circuit system is coupled to the housing, a cover can optionally be disposed about the medical device, 108. The cover can have a protrusion disposed between a battery and a battery contact portion of the electronic circuit system. In some embodiments, for example, the cover can be similar to the cover 4200 of the medical injector 4000 and/or the cover 5200 of the medical injector 5000.

FIG. 42 is a flow chart of a method 120 according to an embodiment of the invention. The method includes optionally assembling a simulated medicament delivery device, 122. The medicament delivery device can include a housing, an actuator and a safety lock. The simulated medicament delivery device is configured to simulate an actual medicament delivery device. An electronic circuit system is then aligned with a portion of the housing configured to receive the electronic circuit system, 124. Aligning the electronic circuit system with the housing ensures that portions of the housing align with corresponding portions of the electronic circuit system. If the corresponding portions do not align, a number of issues can arise. For example, the electronic circuit system may not function correctly and/or the electronic circuit system may be damaged as a result of improper alignment.

The electronic circuit system is then coupled to the simulated medicament delivery device such that a portion of the housing actuates a switch of the electronic circuit system, 126. The electronic circuit system is configured to output an electronic output associated with a use of the simulated medicament delivery device and a state of the switch. The switch can be similar to the electronic circuit system configuration switch 5974 of the medicament delivery device 5000. For example, the electronic circuit system can output a first electronic output associated with a use of the simulated medicament delivery device when the switch is in a first state and a second electronic output associated with a use of the simulated medicament delivery device when the switch is in a second state. In some embodiments, the electronic output can be, for example, a visual output, an audible output, and/or a haptic output, such as those described above. In other embodiments, the electronic output can be a wireless signal configured to be received by a remote device. As described above, any number of switches can be disposed on the electronic circuit system.

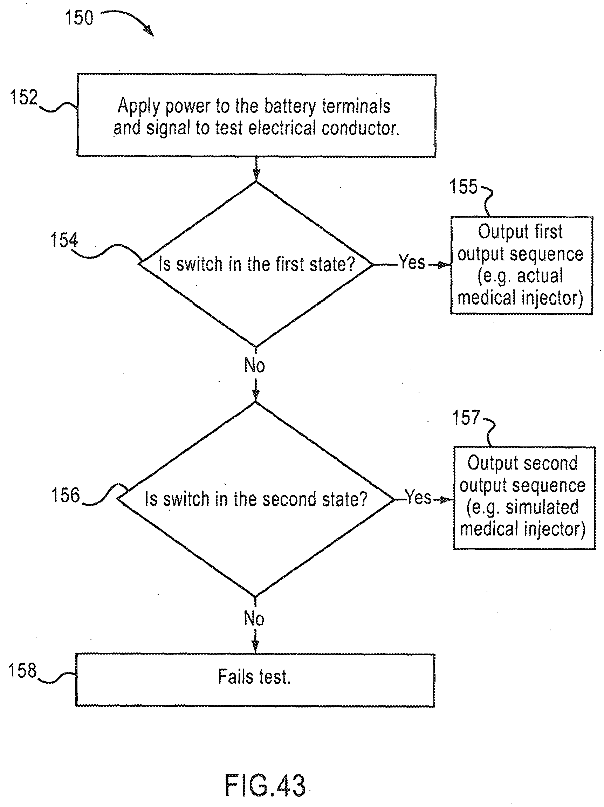

In some embodiments, an electronic self-test can be used to verify the integrity of an electronic circuit system and/or the switches of a medicament delivery device. FIG. 43 is a flow chart of a self-test method 150 that can be administered to ensure that a switch of the electronic circuit system is in the proper state (i.e., a state that corresponds to the configuration of the medicament delivery device). For example, in some embodiments the method 150 can ensure that the electronic circuit system configuration switch is in the correct state (i.e., a first state if the medicament delivery device is an actual medicament delivery device or a second state if the medicament delivery device is a simulated medicament delivery device). The method includes applying power to the battery terminals, 152 and thus the electronic circuit system. If the electronic circuit system configuration switch is in the first state, 154, the electronic circuit system will output a first output sequence, 155. For example, the first output sequence can consist of the LEDs blinking in a first predetermined sequence (e.g., green - red - green) followed by an audible output. The first output sequence can indicate that the medicament delivery device is an actual medicament delivery device and not a simulated medicament delivery device. If the electronic circuit system configuration switch is in the second state, 156, the electronic circuit system will output a second output sequence, different than the first, 157. For example, the second output sequence can consist of the LEDs blinking in a second predetermined sequence (e.g., red - green - green) followed by an audible output. The second output sequence can indicate that the medicament delivery device is a trainer. If neither the first output sequence or the second output sequence occurs, the medical injector has failed the test, 158, indicating that an error exists within the electronic circuit system.

In other embodiments, different electronic output sequences can be used to indicate and/or test different modes of the medical injector. For example, the LEDs could blink in a third sequence to indicate a Spanish medical injector. Additionally, any number of self tests can be used to determine the state of each switch of the electronic circuit system. Further, the integrity of any number of electronic components of the medicament delivery device can be tested by the self-test. For example, the integrity of the LEDs and/or audible output device can be tested using a similar self-test as the one described above.

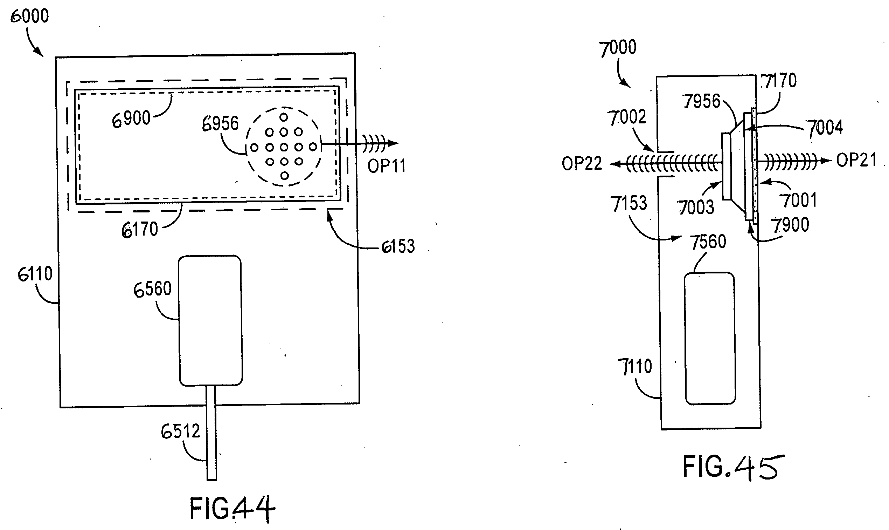

Although the medical injectors and/or medicament delivery devices shown are shown and described above as having a having a housing (e.g., housing 4110 or housing 5110) defining an electronic circuit system cavity that is fluidically, acoustically and/or physically isolated from a medicament cavity, in other embodiments, a medicament delivery device can define an electronic circuit system cavity and/or acoustic enclosure that is in fluid and/or acoustic communication with a medicament cavity. For example, FIG. 44 is a schematic illustration of a medicament delivery device 6000 having an acoustic enclosure, according to an embodiment of the invention. The medicament delivery device 6000 includes a housing 6110, a medicament container 6560, a medicament delivery member 6512, and an electronic circuit system 6900. The medicament container 6560, which can be, for example, a pre-filled cartridge, a vial, an ampule, or the like, is disposed within the housing 6110. At least a portion of the medicament delivery member 6512 is disposed within the housing 6110. In some configurations, the medicament delivery member 6512 can be in fluid communication with the medicament container 6560. In this manner, a medicament can be conveyed from the medicament container 6560 to a region outside the housing 6110 via the medicament delivery member 6512. The medicament delivery member 6512 can include, for example, a needle, a nozzle, a mouthpiece, or the like.

In some embodiments, the medicament delivery device 6000 can be any suitable medical injector for injecting a medicament into a body of a patient. For example, the medicament delivery device 6000 can be a syringe, pen injector, auto-injector, or the like. In other embodiments, the medicament delivery device 6000 can be an inhaler. In yet another embodiment, the medicament delivery device 6000 can be a transdermal delivery system. In some embodiments, the medicament delivery device 6000 can be a chronic-care medicament delivery device. Said another way, the medicament delivery device 6000 can be a reusable device containing multiple doses of medicament. For example, a medicament delivery device 6000 having multiple doses of medicament can be used to manage insulin delivery or the delivery of other medicaments (e.g., to treat Multiple Sclerosis, Anemia, Rheumatoid Arthritis, Osteoporosis or the like), which can, in some instances, require daily, weekly, and/or monthly dosages. In other embodiments, the medicament delivery device 6000 can be a single-use device. Said another way, the medicament delivery device 6000 can contain a single dose of medicament. In yet other embodiments, the medicament delivery device 6000 can be a simulated medicament delivery device or trainer similar to the simulated medicament delivery devices or trainers described in U.S. Patent Publication Number 2008/0059133, entitled "Medical Injector Simulation Device," filed February 27, 2007.

The electronic circuit system 6900 includes an audible output device 6956 and a cover 6170 coupled to the housing 6110. The audible output device 6956, which can be, for example, a microspeaker, is configured to produce an audible output OP11. Said another way, the audible output device 6956 is configured to produce a set of sound waves in response to an electronic signal from the electronic circuit system 6900. In some embodiments, the electronic circuit system 6900 and the audible output device 6956 can produce the audible output OP11 in association with the use of the medicament delivery device 6000.

The electronic circuit system 6900 can include any suitable electronic components operatively coupled to produce and/or output the audible output OP 11 and/or to perform the functions described herein. In some embodiments, the electronic circuit system 6900 can be similar to the electronic circuit systems described above (e.g., electronic circuit system 4900, electronic circuit system 5900, etc.).

The housing 6110 and the cover 6170 of the electronic circuit system 6900 collectively define a region 6153. Although the region 6153 is illustrated in FIG. 44 as a two-dimensional area, the region 6153 is associated with an enclosed volume or space within the housing 6110. Similarly stated, the region 6153 can be a cavity, a chamber, or an enclosure defined by the housing 6110 and the cover 6170. In some embodiments, the region 6153 can be associated with a volume or space within the housing 6110 having at least one opening (not shown in FIG. 44) to an area outside of the housing 6110.

At least a portion of the electronic circuit system 6900 is disposed within the region 6153 of the housing 6110. The electronic circuit system 6900 is coupled to the housing 6110 such that the audible output device 6956 is disposed within the region 6153 defined by the housing 6110 and the cover 6170. Moreover, the volume associated with the region 6153 is larger than the volume of the audible output device 6956. In this manner, the region 6153 can function as an acoustic enclosure for the audible output device 6956. As an acoustic enclosure, the region 6153 can be used to minimize or attenuate noise and/or to enhance the audible output OP11 of the audible output device 6956. In some embodiments, the region 6153 can reduce noise by isolating and/or absorbing sound and/or vibration associated with the audible output device 6956. In some embodiments, the region 6153 can enhance the audible output OP11 of an audible output device 6956 by acoustically amplifying the audible output at one or more acoustic resonant frequencies defined by the physical characteristics of the region 6153 (e.g., volume, shape, or the like). In some embodiments, for example, the region 6153 defines at least one resonant acoustic frequency within the acoustic frequency range of the audible output device 6956.

The audible output OP11 can be, for example, an audible representation of a recorded message or speech, a single tone or a sequence of tones, and/or the like. In some embodiments, the audible output OP11 can be associated with a pre-recorded speech, instruction, or prompt for using the medicament delivery device 6000. In other embodiments, the audible output OP 11 can be associated with post-use instructions or prompts, such as, for example, a recorded message notifying the user that the medicament delivery event is complete, instructing the user on post-medicament delivery disposal and safety procedures, instructing the user to seek post-medicament delivery medical treatment, and/or the like. In yet other embodiments, the audible output OP11 can be associated with the patient's compliance in using the medicament delivery device 6000. In some embodiments, the audible output OP11 can be associated with an actuation of the medicament delivery device 6000. Said another way, the audible output device 6956 can be configured to output the audible output OP 11 in response to the triggering or activating of a function, procedure, and/or mode associated with the medicament delivery device 6000.

Although the region 6153 is shown as being fully enclosed, in other embodiments the region 6153 can be partially enclosed. In some embodiments, for example, the cover 6170 and/or the housing 6110 define an opening (not shown) through which the audible output device 6956 can be disposed within the region 6153. In some embodiments, the shape of the audible output device 6956 can substantially match the shape of the partially enclosed region 6153. In other embodiments, the volume of the audible output device 6956 disposed within the partially enclosed region 6153 is smaller than the volume of the partially enclosed region 6153.

FIG. 45 is a schematic illustration of a medicament delivery device 7000 having a ported acoustic enclosure, according to an embodiment of the invention. The medicament delivery device 7000 includes a housing 7110, a medicament container 7560, and an electronic circuit system 7900. The medicament container 7560, which can be, for example, a pre-filled cartridge, a vial, an ampule, or the like, is disposed within the housing 7110.

The medicament delivery device 7000 can be a reusable device containing multiple doses of medicament. For example, a medicament delivery device 7000 having multiple doses of medicament can be used to manage insulin delivery or the delivery of other medicaments (e.g., to treat Multiple Sclerosis, Anemia, Rheumatoid Arthritis, Osteoporosis or the like), which can, in some instances, require daily, weekly, and/or monthly dosage. In other embodiments, the medicament delivery device 7000 can be a single-use device. Said another way, the medicament delivery device 7000 can contain a single dose of medicament. In yet other embodiments, the medicament delivery device 7000 can be a simulated medicament delivery device or trainer.

The electronic circuit system 7900 can include any suitable electronic components operatively coupled to produce and/or output the audible output and/or to perform the functions described herein. The electronic circuit system 7900 includes an audible output device 7956 and a cover 7170 coupled to the housing 7110. The audible output device 7956, which can be, for example, a microspeaker, includes a front portion 7004 and a back portion 7003. The front portion 7004 of the audible output device 7956 is configured to output a first audible output OP21 that includes a first set of sound waves. The back portion 7003 of the audible output device 7956 is configured to output a second audible output OP22 that includes a second set of sound waves. The first set sound waves associated with the first audible output OP21 can result from changes in air pressure that occur at the front portion 7004 of the audible output device 7956 from, for example, a controlled movement of a portion of the audible output device 7956 (e.g., a cone, membrane, diaphragm, or the like). The second set of sound waves associated with the second audible output OP22 can result from changes in air pressure that occur at the back portion 7003 of the audible output device 7956 from, for example, the movement of a portion of the audible output device 7956. In some embodiments, a single moving portion (e.g., a speaker cone) can produce both the first set of sound waves and the second set of sound waves. For example, a movement of the cone that produces an increase in air pressure at the front portion 7004 of the audible output device 7956 results in a corresponding decrease in air pressure at the back portion 7003 of the audible output device 7956. Similarly, a movement of the cone that produces a decrease in air pressure at the front portion 7004 of the audible output device 7956 results in a corresponding increase in air pressure at the back portion 7003 of the audible output device 7956. Accordingly, in some embodiments, the first set of sound waves produced at the front portion 7004 of the audible output device 7956 can be out-of-phase with the second set of sound waves produced at the back portion 7003 of the audible output device 7956. In this manner, the electronic circuit system 7900 and the audible output device 7956 can produce an audible output associated with the use of the medicament delivery device 7000.

The housing 7110 and the cover 7170 of the electronic circuit system 7900 collectively define a region 7153. Although the region 7153 is illustrated in FIG. 45 as a two-dimensional area, the region 7153 is associated with an enclosed volume or space within the housing 7110. Similarly stated, the region 7153 can be a cavity, a chamber, or an enclosure defined by the housing 7110 and the cover 7170. At least a portion of the electronic circuit system 7900 and/or the audible output device 7956 is disposed within the region 7153 of the housing 7110. In this manner, the region 7153 can function as an acoustic enclosure for the audible output device 7956.

The cover 7170 defines an opening 7001 through which the first set of sound waves associated with the audible output OP21 can travel. Similarly, the housing 7110 defines an opening 7002 through which the second set of sound waves associated with the audible output OP22 can travel. The opening 7002 can be referred to, for example, as a "port" of the acoustic enclosure associated with the region 7153. In some embodiments, the opening 7001 and the opening 7002 can be collectively configured such that the first set of sound waves associated with the audible output OP21 when exiting the housing 7110 through the opening 7001 is substantially in phase with the second set of sound waves associated with the audible output OP22 when exiting the housing 7110 through the opening 7002. Similarly stated, in some embodiments, the opening 7002 can be positioned and/or oriented relative to the opening 7001 to compensate, reduce and/or eliminate the phase difference that can exist between the first set of sound waves of the audible output OP21 and the second set of sound waves of the audible output OP22 within the housing 7110. Said another way, in some embodiments, the distance that the first set of sound waves of the audible output OP21 travels to exit through the opening 7001 (e.g., the distance between the front portion 7004 of the audible output device 7956 and the exit of the opening 7001) and the distance that the second set of sound waves of the audible output OP22 travels to exit through the opening 7002 (e.g., the distance between the back portion 7003 of the audible output device 7956 and the exit of the opening 7002) is such that the first set of sound waves associated with the audible output OP21 when exiting the housing 7110 is substantially in phase with the second set of sound waves associated with the audible output OP22 when exiting the housing 7110 through the opening 7002. In this manner, the phase compensation that results from the difference between the exit path of the first set of sound waves of the audible output OP21 and the exit path of the second set of sound waves of the audible output OP22 can increase (e.g., constructively interfere) the overall sound level of the audible output device 7956 outside the housing 7110.

Although the cover 7170 is shown as defining the opening 7001, in other embodiments, the housing 7110 can define both the opening 7001 and the opening 7002. Although the cover 7170 is described as defining a single opening 7001, in other embodiments, the cover 7170 can define multiple openings. Similarly, in some embodiments, the housing 7110 can define multiple "ports" or openings. In some embodiments, the opening 7002 is configured to be selectively covered by a moveable member (not shown) of the medicament delivery device 7000. For example, the opening 7002 can be selectively covered by at least one of a sleeve, a safety lock, or a needle guard.

The audible outputs OP21 and OP22 can be related to instructions, notifications, messages, actuations, and/or compliance associated with using the medicament delivery device 7000. The audible output OP21 and the audible output OP22 can be, for example, a recorded message or speech, a single tone or a sequence of tones, and/or the like. In this manner, the electronic circuit system 7900 can output information to the user through the audile outputs OP21 and OP22 in an unobtrusive manner and/or without impeding the delivery of the medicaments. The audible outputs OP21 and OP22 can be, for example, audible representations of a recorded message or speech, single tones or sequences of tones, and/or the like. In some embodiments, the audible outputs OP21 and OP22 can be associated with a pre-recorded speech, instruction, or prompt for using the medicament delivery device 7000. In other embodiments, the audible outputs OP21 and OP22 can be associated with post-use instructions or prompts, such as, for example, a recorded message notifying the user that the medicament delivery is complete, instructing the user on post-medicament delivery disposal and safety procedures, instructing the user to seek post-medicament delivery medical treatment, and/or the like. In yet other embodiments, the audible outputs OP21 and OP22 can be associated with the patient's compliance in using the medicament delivery device 7000. In some embodiments, the audible outputs OP21 and OP22 can be associated with an actuation of the medicament delivery device 7000. Said another way, the audible output device 7956 can be configured to output the audible outputs OP21 and OP22 in response to the triggering or activating of a function, procedure, and/or mode associated with the medicament delivery device 7000.

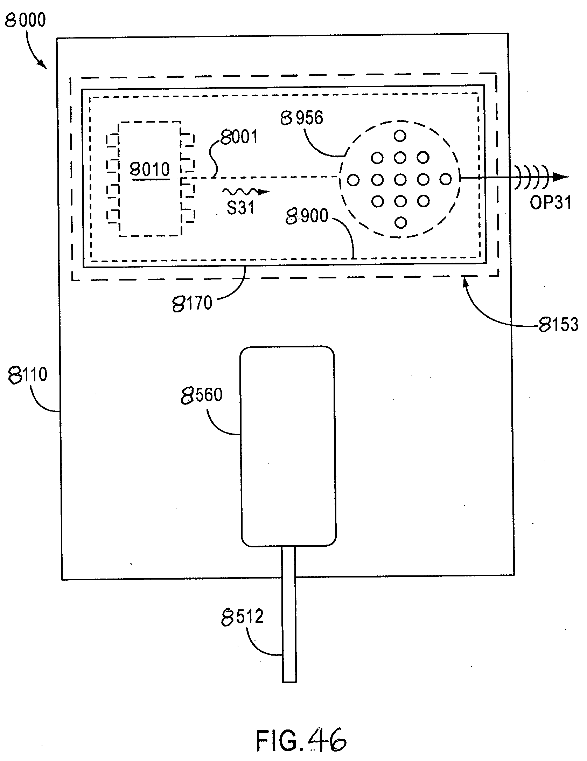

FIG. 46 is a schematic illustration of a medicament delivery device 8000 according to an embodiment of the invention. The medicament delivery device 8000 includes a housing 8110, a medicament container 8560, a medicament delivery member 8512, and an electronic circuit system 8900. The medicament container 8560 is disposed within the housing 8110. At least a portion of the medicament delivery member 8512 is disposed within the housing 8110. The medicament container 8560 and/or the medicament delivery member 8512 can be substantially similar to the medicament container 6560 and/or the medicament delivery member 6512, respectively, as shown and described above with reference to FIG. 44.

The electronic circuit system 8900, which can be can be similar to the electronic circuit system 1900 shown and described above with reference to FIG. 44, includes an audio processor 8010 and an audible output device 8956. The audio processor 8010 is configured to output an electronic output S31 to the audible output device 8956. The audio processor 8010 can be software-based (e.g., set of instructions executable at a processor, software code) and/or hardware-based (e.g., circuit system, processor, application-specific integrated circuit (ASIC), field programmable gate array (FPGA)). The electronic output S31 can be associated with, for example, a recorded message or speech, a single tone or a sequence of tones, and/or the like. In some embodiments, the electronic circuit system 8900 and/or the audio processor 8010 can include a separate memory (not shown) in which information associated with a recorded message or speech, tones, or the like can be stored. In this manner, the audio processor 8010 can receive the recorded message or tone information from the memory for processing and to produce the electronic output S31. In some embodiments, for example, the audio processor 8010 can include an embedded or built-in memory module in which information associated with the recorded message or tone is stored.

As shown in FIG. 46, the electronic output S31 is conveyed from the audio processor 8010 to the audible output device 8956 via an electronic path 8001 devoid of an amplifier. Similarly stated, no amplifiers and/or drivers external to the audio processor 8010 are used to boost or increase the electronic output S31. The electronic path 8001 can be defined by any suitable electronic components. For example, in some embodiments, the electronic circuit system 8900 can have the audio processor 8010, the audible output device 8956, and/or the electronic path 8001 disposed on one or more printed circuit boards (PCBs), Such an arrangement can reduce the cost and/or complexity of the electronic circuit system 8900. Moreover, by excluding external amplifiers and/or drivers, the power required to produce the audible output OP31 can be relatively low, as discussed in more detail herein.

The audible output device 8956, which can be, for example, a microspeaker, is configured to produce the audible output OP31 in response to the electronic output S31. In this manner, the electronic circuit system 8900 and the audible output device 8956 can produce an audible output associated with the use of the medicament delivery device 8000, as discussed above.

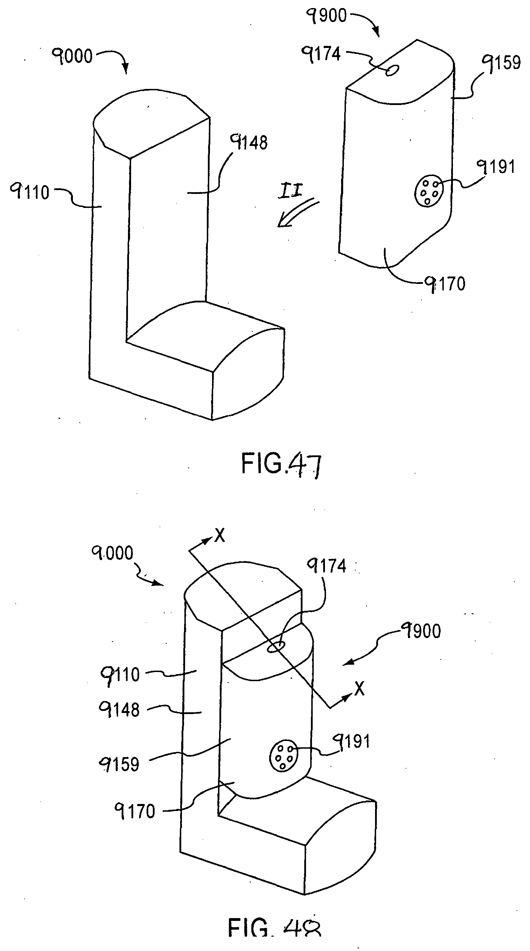

Although the housing 4110 is shown and described above as defining the electronic circuit system cavity 4153, in other embodiments, the housing 4110 need not define the electronic circuit system cavity 4153. For example, in some embodiments, the electronic circuit system assembly can include an electronic circuit housing that defines a cavity and/or an acoustic enclosure within which a speaker is disposed. For example, FIGS. 47-49 are schematic illustrations of medicament delivery device 9000 and an electronic circuit system assembly 9900 according to an embodiment. The medicament delivery device 9000 can be any suitable device for delivering a medicament into a body of a patient. For example, the medicament delivery device 9000 can be an inhaler, a medical injector (e.g., a syringe, pen injector, auto-injector, or the like), a transdermal medicament delivery system or the like. The medicament delivery device 9000 includes a housing 9110 containing a medicament container (not shown in FIGS. 47-49) and/or a medicament delivery mechanism (not shown in FIG. 47-49) for delivering the medicament into the body.

As shown in FIGS. 47 and 48, the electronic circuit system assembly 9900 is configured to be coupled to the housing 9110 of the medicament delivery device 9000. In some embodiments, for example, the electronic circuit system assembly 9900 can be configured to be coupled to a particular medicament delivery device as a kit (e.g., a retrofit kit). In other embodiments, the electronic circuit system assembly 9900 can be configured to be coupled to multiple different medicament delivery devices. Similarly stated, in some embodiments, the electronic circuit system assembly 9900 can be a part of a "universal" retrofit kit. As described above, the electronic circuit system 9900 is configured to produce and/or output an audible output associated with a use of the medicament delivery device 9000.

The electronic circuit system assembly 9900 includes at least a housing 9170, a printed circuit board 9922, and a speaker 9956. The printed circuit board 9922 includes electronic components (e.g., a processor, a battery assembly, or the like; not shown in FIGS. 47-49) operatively coupled to produce and/or output an audible output. The printed circuit board 9922, the speaker 9956 and the associated electronic components can be similar to those included in the electronic circuit system 4900 shown and described above.

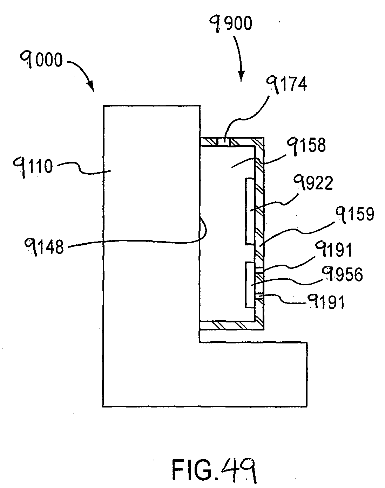

The housing 9170 of the electronic circuit system assembly 9900 includes a side wall 9159 that defines a cavity 9158, an end opening 9174 and multiple sound apertures 9191. As shown in FIG. 49 the printed circuit board 9922 and the speaker 9956 are coupled to the housing 9170 of the electronic circuit system assembly 9900 within the cavity 9158. In particular, the speaker 9956 is disposed against the side wall 9159 such that a first side (e.g., a front side) of the speaker 9956 is disposed adjacent the sound apertures 9191. In this manner, sound waves from the first side of speaker 9956 can travel from the speaker 9956 to a region outside of the housing 9170 of the electronic circuit system assembly 9900 via the sound apertures 9191.

The housing 9170 of the electronic circuit system assembly 9900 is coupled to the housing 9110 of the medicament delivery device 9000, as shown by the arrow II in FIG. 47. The housing 9170 can be coupled to the housing 9110 by any suitable mechanism (e.g., by mating protrusions and recesses, by heat staking, using an adhesive bond, or the like). As shown in FIG. 49, the housing 9110 of the medicament delivery device 9000 and the cavity 9158 of the housing 9170 of the electronic circuit system assembly 9900 collectively define an acoustic enclosure within which the speaker 9956 is disposed. Said another way, a surface 9148 of the housing 9110 and the side wall 9159 collectively define the boundary of a region, volume and/or space that is configured to minimize or attenuate noise and/or enhance the audible output of the speaker 9956, in a manner as described above. Moreover, the opening 9174 can function as a port to allow sound waves produced by a second side (e.g., a back side) of the speaker 9956 to exit the cavity 9158 of the housing 9170 to an area outside the housing 9170. As described above, the opening 9174 can be spaced a predetermined distance from the speaker 9956 to enhance the quality of the audible output produced by the speaker 9956.

Because the electronic circuit system 9900 includes the electronic components (e.g., a processor configured to produce an electronic signal, the speaker 9956 and the like) and defines the cavity 9158, the electronic components and the cavity 9158 can be complimentarily selected and/or configured to enhance the quality of the audible output produced by the speaker 9956. Similarly stated, this arrangement allows the sound performance of the electronic circuit system 9900 to be optimized substantially independent of the housing 9110 of the medicament delivery device 9900.

Although the acoustic enclosure is shown as being defined, in part, by a surface 9148 of the housing 9110 of the medicament delivery device 9000, in other embodiments, the housing 9170 of the electronic circuit system assembly 9900 can define a substantially enclosed cavity 9158 configured to function as an acoustic chamber. In some embodiments, for example, a housing 9170 can include a sidewall configured to be disposed against a portion of the medicament delivery device 9000 and define a boundary of an acoustic enclosure. In such embodiments, the side wall can be any suitable side wall for coupling the housing 9170 of the electronic circuit system assembly 9900 to the medicament delivery device 9000 and/or enhancing the audible output produced by the speaker 9956. For example, the side wall can be an elastic side wall. In other embodiments, the side wall can be a porous side wall.

Although the cavity 9158 of the housing 9170 of the electronic circuit system assembly 9900 is shown as being disposed adjacent the medicament delivery device 9000, in some embodiments, a portion of the medicament delivery device can be disposed within the cavity. For example, in some embodiments, the electronic circuit system assembly can be disposed about a portion of the medicament delivery device.

While various embodiments of the invention have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

For example, in some embodiments, the sidewall of the housing of a medicament delivery device can be rigid. In other embodiments, the sidewall can be a movable member such as, for example, a piston. In yet other embodiments, the sidewall can be a flexible member such as, for example, a diaphragm. In some embodiments, the sidewall can be transparent allowing light to pass from the a first region to a second region and vice versa. A transparent sidewall can be used in conjunction with an optical sensor. The sidewall can be integrally formed with the housing or can be separately formed.

In other embodiments, the medicament container can be substantially cylindrical with a substantially round and/or substantially elliptical cross-sectional shape. Thus, the medicament container can define a longitudinal axis, the longitudinal axes of the medicament container can be parallel, non-coaxial, and/or co-planar. The longitudinal axis of the medicament container can be co-axial with a longitudinal axis of the piston portion of a movable member 4530. In still other embodiments, a medicament delivery device can contain multiple medicament containers and thus, multiple doses of medicament.

Although medical devices having two LEDs and an audible output device have been shown, in other embodiments the medical device might have any number of LEDs and/or audible output devices. Additionally, other types of output devices, such as haptic output devices, can be used.

Although the audio output device 4956 is shown and described as having front portion 4957 configured to produce a first set of sound waves and a back portion 4955 opposite the front portion configured to produce a second set of sound waves, in other embodiments, an audio device can have a first portion configured to produce a first set of sound waves and a second portion configured to produce a second set of sound waves wherein the first portion is not opposite the second portion. For example, in some embodiments, an audio output device can have a first surface configured to produce a first set of sound waves and a second surface adjacent and/or in contact with the first surface, the second surface configured to produce a second set of sound waves.

Although the electronic circuit system cavity 4153 is shown as defining the acoustic enclosure, in other embodiments, a medicament delivery device can define an acoustic enclosure that is separate from a cavity within which a portion of the electronic circuit system is disposed. In some embodiments, for example a housing can define a first cavity within which a printed circuit board of an electronic circuit system is disposed and a second cavity distinct from the first cavity within which a portion of an audio output device is disposed. The first cavity can include, for example, potting material to enhance the reliability and/or performance of the printed circuit board. The second cavity can be devoid of internal structure and can function as an acoustic enclosure.

Although the electronic circuit system cavity 4153 is shown and described as being substantially devoid of structure, in other embodiments, an electronic circuit system cavity can include components therein to enhance the performance of the cavity as an acoustic enclosure. For example, in some embodiments, an electronic circuit system cavity can include a set of baffles. In this manner, the length through which sound waves produced by a back portion of an audio output device can be increased to a value greater than an overall length of the electronic circuit system cavity.

Although the electronic circuit system cavity 4153 is shown and described as having a single "port" (i.e., the battery isolation protrusion aperture 4121) disposed at the distal end thereof, in other embodiments, an electronic circuit system cavity 4153 can include a port disposed in any suitable location. For example, in some embodiments, an electronic circuit system cavity can include a port disposed at the proximal end thereof. Moreover, in some embodiments, an electronic circuit system cavity 4153 can include multiple openings at multiple different locations.

While the housing shown in the current embodiment is rigid, a portion of the housing can be made flexible such that the flexible portion of the housing operates as a passive counterpart to the active operation of the audible output device. In such embodiment, the flexible portion of the housing can dynamically adjust the acoustic enclosure size and/or shape to improve the sound pressure level produced by the audible output device. For example, in some embodiments, the sidewall of the housing that defines a portion of an acoustic enclosure can be a movable member such as, for example, a piston. In yet other embodiments, the sidewall can be a flexible member such as, for example, a diaphragm. The sidewall can be integrally formed with the housing or can be separately formed.

Although the battery assembly 4962 is shown and described as including two batteries stacked on top of one another, in other embodiments, a battery assembly can include two or more batteries that are not arranged in a stacked fashion. For example, in some embodiments, a battery assembly can include two or more batteries that are arranged end-to-end such that an edge of one battery is in contact with an edge of another battery. In other embodiments, a battery assembly can include two or more batteries that are spaced apart from each other. Similarly, although the battery clip 4910 is shown and described as having a single contact portion 4918 at the distal end thereof, in other embodiments (e.g., embodiments in which the battery assembly includes batteries in an unstacked relationship), a battery clip can include more than one contact portion.

Although the battery assembly 4962 is shown and described above as including three volt, "watch-style" batteries, in other embodiments, the electronic circuit system 4900 can be powered by any suitable power source. For example, in some embodiments, the battery assembly 4962 can include one or more rechargeable batteries. Such an arrangement is well-suited for multiple-use medicament delivery devices (e.g., chronic-care devices). In other embodiments, an electronic circuit system can be devoid of a battery assembly 4962. Said another way, in some embodiments, electrical power can be provided to an electronic circuit system by a source other than batteries (e.g., a solar power supply, a capacitance-based power supply, a bio-active power supply that produces electricity by breaking down organic materials, a small-scale mechanical generator, a small-scale fuel cell or the like).

Although the medicament delivery device 4000 is shown and described as being an actual medicament delivery device, in some embodiments, the housing 4110 and/or the electronic circuit system 4900 can be associated with a simulated medicament delivery device. Such simulated devices can be devoid of medicament and/or needles, and can be used, for example, to train users in the operation of a corresponding actual medicament delivery device.

Some embodiments include a processor and a related processor-readable medium having instructions or computer code thereon for performing various processor-implemented operations. Such processors can be implemented as hardware modules such as embedded microprocessors, microprocessors as part of a computer system, Application-Specific Integrated Circuits ("ASICs"), and Programmable Logic Devices ("PLDs"). Such processors can also be implemented as one or more software modules in programming languages as Java, C++, C, assembly, a hardware description language, or any other suitable programming language.

A processor according to some embodiments includes media and computer code (also can be referred to as code) specially designed and constructed for the specific purpose or purposes. Examples of processor-readable media include, but are not limited to: magnetic storage media such as hard disks, floppy disks, and magnetic tape; optical storage media such as Compact Disc/Digital Video Discs ("CD/DVDs"), Compact Disc-Read Only Memories ("CD-ROMs"), and holographic devices; magneto-optical storage media such as optical disks, and read-only memory ("ROM") and random-access memory ("RAM") devices. Examples of computer code include, but are not limited to, micro-code or micro-instructions, machine instructions, such as produced by a compiler, and files containing higher-level instructions that are executed by a computer using an interpreter. For example, an embodiment of the invention can be implemented using Java, C++, or other object-oriented programming language and development tools. Additional examples of computer code include, but are not limited to, control signals, encrypted code, and compressed code.

Although various embodiments have been described as having particular features and/or combinations of components, other embodiments are possible having a combination of any features and/or components from any of embodiments where appropriate. For example, electrical circuit system 4900 can include a electronic circuit system configuration switch similar to that of electrical circuit system 5900.

For example, although the medicament delivery device 4000 is shown and described as including an electronic circuit system cavity 4153 that can function as an acoustic enclosure and an electronic circuit system devoid of a signal amplifier, in other embodiments, a medicament delivery device can include an acoustic enclosure and an electronic circuit system having a signal amplifier. In such embodiments, for example, the electronic signal produced by a processor (which can have a power of less than 100 milliwatts) can be amplified to provide an input signal to a speaker having a power level of greater than the power level of the signal produced by the processor.

## Claims

1. An apparatus, comprising:
a medicament delivery device including a housing (1110, 4110), a medicament container (1560, 4560), an actuator (4311), and a medicament delivery member (1512, 4512), the housing defining a first region (1157, 4157) a second region (1153, 4153), at least one sidewall (1148) of the housing separating the first region, and the second region from each other,
the first region including the medicament container and the medicament delivery member, the medicament delivery member configured to be in fluid communication with an area outside the housing via a first opening of the housing, the second region being fluidically and/or physically isolated from the first region; and
an electronic circuit system (1900, 4900) configured to be disposed within the second region, the electronic circuit system configured to output an electronic output when the electronic circuit system is actuated,
a cover (4240) configured to be removeably coupled to the housing, a first portion of the cover configured to cover the first opening (4122) when the cover is coupled to the housing, a second portion (4236) of the cover extending into the second region of the housing via a second opening (4121) when the cover is coupled to the housing, the electronic circuit system configured to be actuated when the cover is removed from the housing.

2. The apparatus of claim 1, wherein the medicament delivery device includes any one of a pen injector, an auto-injector or an inhaler.

3. The apparatus of claim 1, wherein the electronic output of the electronic circuit system is recorded speech output associated with at least one of an instruction for using the medical injector, a post-use procedure, or a compliance tracking procedure.

4. The apparatus of claim 1, wherein the electronic output includes a signal configured to be received by a remote device.

5. The apparatus of claim 1, wherein the electronic output is a first electronic output, the medicament container and the delivery member collectively define a medicament delivery path, the electronic circuit system being physically isolated from the medicament delivery path, the electronic circuit system configured to output a second electronic output in response to a delivery of a medicament via the medicament delivery path.

6. The apparatus of claim 1, wherein the cover is a first cover, the_electronic circuit system includes an audible output device and a second cover (4170) such that the second region collectively defined, at least in part, by the housing of the medicament delivery device and the second cover is an acoustic enclosure, the audible output device configured to be disposed within the acoustic enclosure.

7. The apparatus of claim 5, wherein the acoustic enclosure defines at least one acoustic resonant frequency within the acoustic frequency range of the audible output device.

8. The apparatus of claim 5, wherein:
the audible output device is a speaker, the speaker includes a front portion and a back portion, the electronic output includes a first audible output produced by the front portion of the speaker, the first audible output including a first plurality of sound waves and a second audible output produced by the back portion of the speaker, the second audible output including a second plurality of sound waves;
the second cover defines a third opening (4191) through which the first plurality of sound waves is configured to travel; and
the second plurality of sound waves is configured to travel through the second opening,
the second opening and the third opening collectively configured such that the first plurality of sound waves is substantially in phase with the second plurality of sound waves when the first plurality of sound waves exits the second opening and the second plurality of sound waves exits the third opening.

9. The apparatus of claim 1, wherein the medicament delivery device is configured to deliver only a single dose of a medicament into a body.

10. The apparatus of claim 1, wherein:
the medicament delivery device is a medical injector; and
the electronic output of the electronic circuit system is an audible output associated with an instruction for using the medical injector.

11. The apparatus of claim 1, wherein at least a portion of the cover is configured to be disposed about at least a portion of the actuator when the cover is coupled to the housing.

12. The apparatus of claim 1, the actuator is configured to initiate delivery of a medicament from the medicament delivery device.

13. The apparatus of claim 1, wherein:
the electronic output is a first electronic output;
the actuator is configured to initiate delivery of a medicament from the medicament delivery device; and
the electronic circuit system includes a printed circuit board having a substrate and an electrical conductor disposed on the substrate, the substrate configured to receive a portion of the actuator, the actuator being configured to disrupt the electrical conductor, the electronic circuit system configured to output a second electronic output when the electrical conductor is disrupted.

14. The apparatus of claim 1, wherein the second portion of the cover includes an isolation member configured to electrically isolate the electronic circuit system from a power source when the cover is coupled to the medicament delivery device, the electronic circuit system being electrically coupled to the power source when the cover is removed from the medicament delivery device.

15. The apparatus of claim 1, wherein:
the electronic output is a first electronic output;
a first portion of the actuator is configured to initiate delivery of a medicament when the actuator is moved from a first position to a second position; and
a second portion of the actuator is configured to actuate the electronic circuit system to produce a second electronic output when the actuator is moved from the first position to the second position.

## Patentansprüche

1. Vorrichtung, umfassend:
eine Medikamentenfreisetzungsvorrichtung, die ein Gehäuse (1110, 4110), einen Medikamentenbehälter (1560, 4560), ein Betätigungselement (4311) und ein Medikamentenfreisetzungselement (1512, 4512) einschließt, wobei das Gehäuse einen ersten Bereich (1157, 4157), einen zweiten Bereich (1153, 4153), wenigstens eine Seitenwand (1148) des Gehäuses definiert, die den ersten Bereich und den zweiten Bereich voneinander trennt,
den ersten Bereich, der den Medikamentenbehälter und das Medikamentenfreisetzungselement einschließt, wobei das Medikamentenfreisetzungselement konfiguriert ist, in Fluidverbindung mit einem Bereich außerhalb des Gehäuses über eine erste Öffnung des Gehäuses zu sein, wobei der zweite Bereich fluidisch und/oder physikalisch vom ersten Bereich isoliert ist; und
eine elektronische Schaltung (1900, 4900), die konfiguriert ist, innerhalb des zweiten Bereichs angeordnet zu werden, wobei die elektronische Schaltung konfiguriert ist, eine elektronische Ausgangsleistung auszugeben, wenn die elektronische Schaltung betätigt wird,
eine Abdeckung (4240), die konfiguriert ist, entfernbar an das Gehäuse gekoppelt zu werden, wobei ein erster Abschnitt der Abdeckung konfiguriert ist, die erste Öffnung (4122) abzudecken, wenn die Abdeckung an das Gehäuse gekoppelt ist, sich ein zweiter Abschnitt (4236) der Abdeckung in den zweiten Bereich des Gehäuses via eine zweite Öffnung (4121) erstreckt, wenn die Abdeckung an das Gehäuse gekoppelt ist, wobei die elektronische Schaltung konfiguriert ist, betätigt zu werden, wenn die Abdeckung vom Gehäuse entfernt wird.

2. Vorrichtung nach Anspruch 1, wobei die Medikamentenfreisetzungsvorrichtung irgendeinen eines Pen-Injektors, eines automatischen Injektors oder eines Inhalators einschließt.

3. Vorrichtung nach Anspruch 1, wobei die elektronische Ausgangsleistung der elektronischen Schaltung aufgezeichnete Sprachausgangsleistung ist, die mit wenigstens einem von einem Befehl zur Verwendung des medizinischen Injektors, einem Vorgang nach der Verwendung oder einem Vorgang für Einhaltungsverfolgung assoziiert ist.

4. Vorrichtung nach Anspruch 1, wobei die elektronische Ausgangsleistung ein Signal einschließt, das konfiguriert ist, von einem fernen Gerät empfangen zu werden.

5. Vorrichtung nach Anspruch 1, wobei die elektronische Ausgangsleistung eine erste elektronische Ausgangsleistung ist, der Medikamentenbehälter und das Freisetzungselement kollektiv einen Medikamentenfreisetzungsweg definieren, die elektronische Schaltung physikalisch vom Medikamentenfreisetzungsweg isoliert ist, die elektronische Schaltung konfiguriert ist, eine zweite elektronische Ausgangsleistung, als Reaktion auf eine Freisetzung eines Medikamentes über den Medikamentenfreisetzungsweg, auszugeben.

6. Vorrichtung nach Anspruch 1, wobei die Abdeckung eine erste Abdeckung ist, die elektronische Schaltung ein Gerät mit hörbarer Ausgabe und eine zweite Abdeckung (4170) einschließt, derart, dass der zweite Bereich, wenigstens teilweise, durch das Gehäuse der Medikamentenfreisetzungsvorrichtung kollektiv definiert wird und die zweite Abdeckung ein akustisches Gehäuse ist, wobei das Gerät mit hörbarer Ausgabe konfiguriert ist, innerhalb des akustischen Gehäuses angeordnet zu werden.

7. Vorrichtung nach Anspruch 5, wobei das akustische Gehäuse wenigstens eine akustische Resonanzfrequenz innerhalb des akustischen Frequenzbereichs des Geräts mit hörbarer Ausgabe definiert.

8. Vorrichtung nach Anspruch 5, wobei:
das Gerät mit hörbarer Ausgabe ein Lautsprecher ist, wobei der Lautsprecher einen vorderen Teil und einen hinteren Teil einschließt, die elektronische Ausgangsleistung eine erste hörbare Ausgabe einschließt, die vom vorderen Teil des Lautsprechers erzeugt wird, die erste hörbare Ausgabe eine erste Vielzahl von Schallwellen und eine zweite hörbare Ausgabe einschließt, die vom hinteren Teil des Lautsprechers erzeugt wird, wobei die zweite hörbare Ausgabe eine zweite Vielzahl von Schallwellen einschließt;
die zweite Abdeckung eine dritte Öffnung (4191) definiert, durch welche die erste Vielzahl von Schallwellen zu wandern konfiguriert ist; und
die zweite Vielzahl von Schallwellen konfiguriert ist, durch die zweite Öffnung zu wandern,
wobei die zweite Öffnung und die dritte Öffnung kollektiv derart konfiguriert sind, dass die erste Vielzahl von Schallwellen im Wesentlichen in Phase mit der zweiten Vielzahl von Schallwellen ist, wenn die erste Vielzahl von Schallwellen aus der zweiten Öffnung austritt und die zweite Vielzahl von Schallwellen aus der dritten Öffnung austritt.

9. Vorrichtung nach Anspruch 1, wobei die Medikamentenfreisetzungsvorrichtung konfiguriert ist, nur eine Einzeldosis eines Medikaments in einen Körper freizusetzen.

10. Vorrichtung nach Anspruch 1, wobei:
Die Medikamentenfreisetzungsvorrichtung ein medizinischer Injektor ist; und
die elektronische Ausgangsleistung der elektronischen Schaltung eine hörbare Ausgabe ist, die mit einem Befehl zur Verwendung des medizinischen Injektors assoziiert ist.

11. Vorrichtung nach Anspruch 1, wobei wenigstens ein Teil der Abdeckung konfiguriert ist, um wenigstens einen Teil des Betätigungselements angeordnet zu sein, wenn die Abdeckung an das Gehäuse gekoppelt wird.

12. Vorrichtung nach Anspruch 1, wobei das Betätigungselement konfiguriert ist, die Freisetzung eines Medikamentes aus der Medikamentenfreisetzungsvorrichtung zu initiieren.

13. Vorrichtung nach Anspruch 1, wobei:
die elektronische Ausgangsleistung eine erste elektronische Ausgangsleistung ist;
das Betätigungselement konfiguriert ist, die Freisetzung eines Medikamentes aus der Medikamentenfreisetzungsvorrichtung zu initiieren; und
die elektronische Schaltung eine Leiterplatte einschließt, die ein Substrat und einen auf dem Substrat angeordneten elektrischen Leiter aufweist, wobei das Substrat konfiguriert ist, einen Teil des Betätigungselements aufzunehmen, das Betätigungselement konfiguriert ist, den elektrischen Leiter zu unterbrechen, die elektronische Schaltung konfiguriert ist, eine zweite elektronische Ausgangsleistung auszugeben, wenn der elektrische Leiter unterbrochen wird.

14. Vorrichtung nach Anspruch 1, wobei der zweite Teil der Abdeckung ein Isolierelement einschließt, das konfiguriert ist, die elektronische Schaltung von einer Stromquelle zu isolieren, wenn die Abdeckung an die Medikamentenfreisetzungsvorrichtung gekoppelt wird, wobei die elektronische Schaltung elektrisch an die Stromquelle gekoppelt wird, wenn die Abdeckung von der Medikamentenfreisetzungsvorrichtung entfernt wird.

15. Vorrichtung nach Anspruch 1, wobei:
die elektronische Ausgangsleistung eine erste elektronische Ausgangsleistung ist;
ein erster Teil des Betätigungselements konfiguriert ist, die Freisetzung eines Medikamentes zu initiieren, wenn das Betätigungselement aus einer ersten Position in eine zweite Position bewegt wird;
und
ein zweiter Teil des Betätigungselements konfiguriert ist, die elektronische Schaltung zu betätigen, um eine zweite elektronische Ausgangsleistung zu erzeugen, wenn das Betätigungselement aus der ersten Position in die zweite Position bewegt wird.

## Revendications

1. Appareil, comportant :
un dispositif de distribution de médicament comprenant un boîtier (1110, 4110), un contenant de médicament (1560, 4560), un actionneur (4311), un élément de distribution de médicament (1512, 4512), le boîtier définissant une première région (1157, 4157), une deuxième région (1153, 4153), au moins une paroi latérale (1148) du boîtier séparant la première région, et la deuxième région l'une par rapport à l'autre,
la première région comprenant le contenant de médicament et l'élément de distribution de médicament, l'élément de distribution de médicament étant configuré à des fins de communication fluidique avec une zone située à l'extérieur du boîtier par le biais d'une première ouverture du boîtier, la deuxième région étant isolée de manière fluidique et/ou de manière physique par rapport à la première région ; et
un système de circuit électronique (1900, 4900) configuré pour être disposé à l'intérieur de la deuxième région, le système de circuit électronique étant configuré pour émettre une sortie électronique quand le système de circuit électronique est actionné,
un capot (4240) configuré pour être accouplé de manière amovible au boîtier, une première partie du capot étant configurée pour recouvrir la première ouverture (4122) quand le capot est accouplé au boîtier, une deuxième partie (4236) du capot s'étendant jusque dans la deuxième région du boîtier par le biais d'une deuxième ouverture (4121) quand le boîtier est accouplé au boîtier, le système de circuit électronique étant configuré pour être actionné quand le capot est retiré du boîtier.

2. Appareil selon la revendication 1, dans lequel le dispositif de distribution de médicament comprend l'un quelconque parmi un stylo injecteur, un injecteur automatique ou un inhalateur.

3. Appareil selon la revendication 1, dans lequel la sortie électronique du système de circuit électronique est une sortie vocale enregistrée associée à au moins l'une parmi une instruction à des fins d'utilisation de l'injecteur médical, une procédure après l'utilisation, ou une procédure de suivi d'observance.

4. Appareil selon la revendication 1, dans lequel la sortie électronique comprend un signal configuré à des fins de réception par un dispositif distant.

5. Appareil selon la revendication 1, dans lequel la sortie électronique est une première sortie électronique, le contenant de médicament et l'élément de distribution définissent collectivement une trajectoire de distribution de médicament, le système de circuit électronique étant physiquement isolé par rapport à la trajectoire de distribution de médicament, le système de circuit électronique étant configuré pour émettre une deuxième sortie électronique en réponse à une distribution d'un médicament par le biais de la trajectoire de distribution de médicament.

6. Appareil selon la revendication 1, dans lequel le capot est un premier capot, le système de circuit électronique comprend un dispositif de sortie sonore et un deuxième capot (4170) de telle sorte que la deuxième région définie collectivement, au moins en partie, par le boîtier du dispositif de distribution de médicament et le deuxième capot est une enceinte acoustique, le dispositif de sortie sonore étant configuré pour être disposé à l'intérieur de l'enceinte acoustique.

7. Appareil selon la revendication 5, dans lequel l'enceinte acoustique définit au moins une fréquence de résonance acoustique dans les limites de la gamme de fréquences sonores du dispositif de sortie sonore.

8. Appareil selon la revendication 5, dans lequel :
le dispositif de sortie sonore est un haut-parleur, le haut-parleur comprend une partie avant et une partie arrière, la sortie électronique comprend une première sortie sonore produite par la partie avant du haut-parleur, la première sortie sonore comprenant une première pluralité d'ondes sonores et une deuxième sortie sonore produite par la partie arrière du haut-parleur, la deuxième sortie sonore comprenant une deuxième pluralité d'ondes sonores ;
le deuxième capot définit une troisième ouverture (4191) au travers de laquelle la première pluralité d'ondes sonores est configurée pour s'acheminer ; et
la deuxième pluralité d'ondes sonores est configurée pour s'acheminer au travers de la deuxième ouverture,
la deuxième ouverture et la troisième ouverture sont configurées collectivement de telle sorte que la première pluralité d'ondes sonores est sensiblement en phase avec la deuxième pluralité d'ondes sonores quand la première pluralité d'ondes sonores sort de la deuxième ouverture et quand la deuxième pluralité d'ondes sonores sort de la troisième ouverture.

9. Appareil selon la revendication 1, dans lequel le dispositif de distribution de médicament est configuré pour distribuer uniquement une seule dose d'un médicament dans un corps.

10. Appareil selon la revendication 1, dans lequel :
le dispositif de distribution de médicament est un injecteur médical ; et
la sortie électronique du système de circuit électronique est une sortie sonore associée à une instruction à des fins d'utilisation de l'injecteur médical.

11. Appareil selon la revendication 1, dans lequel au moins une partie du capot est configurée pour être disposée autour d'au moins une partie de l'actionneur quand le capot est accouplé au boîtier.

12. Appareil selon la revendication 1, dans lequel l'actionneur est configuré pour initialiser la distribution d'un médicament en provenance du dispositif de distribution de médicament.

13. Appareil selon la revendication 1, dans lequel :
la sortie électronique est une première sortie électronique ;
l'actionneur est configuré pour initialiser la distribution d'un médicament en provenance du dispositif de distribution de médicament ; et
le système de circuit électronique comprend une carte de circuit imprimé ayant un substrat et un conducteur électrique disposé sur le substrat, le substrat étant configuré pour recevoir une partie de l'actionneur, l'actionneur étant configuré pour interrompre le conducteur électrique, le système de circuit électronique étant configuré pour émettre une deuxième sortie électronique quand le conducteur électrique est interrompu.

14. Appareil selon la revendication 1, dans lequel la deuxième partie du capot comprend un élément d'isolement configuré pour isoler électriquement le système de circuit électronique par rapport à une source d'alimentation quand le capot est accouplé au dispositif de distribution de médicament, le système de circuit électronique étant accouplé électriquement à la source d'alimentation quand le capot est retiré du dispositif de distribution de médicament.

15. Appareil selon la revendication 1, dans lequel :
la sortie électronique est une première sortie électronique ;
une première partie de l'actionneur est configurée pour initialiser la distribution d'un médicament quand l'actionneur est déplacé d'une première position jusque sur une deuxième position ; et
une deuxième partie de l'actionneur est configurée pour actionner le système de circuit électronique pour produire une deuxième sortie électronique quand l'actionneur est déplacé de la première position jusque sur la deuxième position.
